(19) Europäisches Patentamt
European Patent Office
Office européen des brevets

(11) Veröffentlichungsnummer: **0 014 263**
**B1**

(12) # EUROPÄISCHE PATENTSCHRIFT

(45) Veröffentlichungstag der Patentschrift: **05.05.82**

(21) Anmeldenummer: **79105407.5**

(22) Anmeldetag: **31.12.79**

(51) Int. Cl.[3]: **C 08 G 65/32**, A 61 K 47/00, A 61 K 9/00, A 01 N 47/34, A 01 N 47/36, A 01 N 47/38, A 01 N 25/02

(54) **Verfahren zur Verbesserung der Löslichkeit biologisch aktiver Wirkstoffe in Wasser und niederen aliphatischen Alkoholen sowie neue Verbindungen mit verbesserter Löslichkeit.**

(30) Priorität: **12.01.79 DE 2901060**
**16.03.79 DE 2910356**

(43) Veröffentlichungstag der Anmeldung: **20.08.80 Patentblatt 80/17**

(45) Bekanntmachung des Hinweises auf die Patenterteilung: **05.05.82 Patentblatt 82/18**

(84) Benannte Vertragsstaaten: **BE CH DE FR GB IT NL**

(56) Entgegenhaltungen:
**DE-A-2 645 079**
**FR-A-2 194 446**
**FR-A-2 196 342**
**FR-A-2 264 522**
**US-A-2 857 261**

**DIE MAKROMOLEKULARE CHEMIE, Band 179, Nr. 5, Mai 1978, Seiten 1135-1144**
**A. CHAABONNI et al.:**
**«Synthèse de poly (oxyéthylène)s rendus biospécifiques par fixation de stéroides en extrémités de chaines. Utilisation d'un poly (oxyéthylène) substitué par des groupes estradiol pour extraire l'isomérase Δ5→4 oxo-3 stéroide, par partage d'affinité»**

(73) Patentinhaber: **BAYER AG, Zentralbereich Patente, Marken und Lizenzen, D-5090 Leverkusen 1, Bayerwerk (DE)**

(72) Erfinder: **Möhring, Edgar, Dr., Hufer Weg 49a, D-5060 Bergisch-Gladbach 2 (DE)**
Erfinder: **Müller, Hanns Peter, Dr., St. Pankratius-Strasse 2, D-5068 Odenthal (DE)**
Erfinder: **Roessler, Peter, Dr., Elster Strasse 15, D-5060 Bergisch-Gladbach 3 (DE)**
Erfinder: **Wagner, Kuno, Dr., Am Kiesberg 8, D-5090 Leverkusen 1 (DE)**
Erfinder: **Tietz, Helmut, Dr., Schwalbenweg 12, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Verbesserung der Löslichkeit biologisch aktiver Wirkstoffe in Wasser und niederen aliphatischen Alkoholen sowie neue Verbindungen mit verbesserter Löslichkeit

Die vorliegende Erfindung betrifft ein neues Verfahren zur Verbesserung der Löslichkeit biologisch aktiver Wirkstoffe mit mindestens einem Zerewitinoff-aktiven Wasserstoffatom in Wasser und niederen aliphatischen Alkoholen, neue nach diesem Verfahren hergestellte Verbindungen und ihre Verwendung.

Unter dem Begriff biologisch aktive Wirkstoffe werden organisch chemische Verbindungen verstanden, die auf dem Pflanzenschutzgebiet sowie in der Human- und Tiermedizin wirksam sind. Viele dieser Wirkstoffe sind in Wasser und in niederen aliphatischen Alkoholen schwer löslich. Sie lassen sich daher in flüssiger Form nur unter Zuhilfenahme von Lösungsvermittlern anwenden. Um die Verwendung solcher Stoffe bei den Anwendungsformen der biologisch aktiven Wirkstoffe so weit als möglich zu reduzieren, ist es wünschenswert, die Löslichkeit dieser Wirkstoffe in Wasser oder niederen aliphatischen Alkoholen zu verbessern.

Es war bereits bekannt, die Löslichkeit von Zinn- oder Blei-organischen Verbindungen durch Verknüpfung mit langkettigen hydrophilen Thioalkoholen zu erreichen. Eine allgemeine Anwendbarkeit des Verfahrens auf andere als die angegebenen metallorganischen Verbindungen ist nicht gegeben (FR-A Nr. 2194446).

Es waren ferner Sauerstoffüberträger nach Art des Hämoglobins bekannt, wobei an ein zentrales Metallion ein lösliches Polymer als vierzähniger Ligand gebunden ist (DE-OS Nr. 2645079). Es geht daraus jedoch nichts über die Verbesserung der Löslichkeit schwer löslicher biologisch aktiver Wirkstoffe hervor.

Es wurde nun gefunden, dass die Löslichkeit biologisch aktiver Wirkstoffe mit mindestens einem Zerewitinoff-aktiven Wasserstoffatom in Wasser und niederen aliphatischen Alkoholen dadurch verbessert wird, dass man

a) biologisch aktive Wirkstoffe mit mindestens einem Zerewitinoff-aktiven Wasserstoffatom im Molverhältnis von etwa 1:1 mit Verbindungen umsetzt, die erhalten werden, indem man einen OH-, NH- oder $NH_2$-monofunktionellen hydrophilen Polyäther mit einem Wasseraufnahmevermögen von mindestens 15%, bezogen auf das Gewicht des Polyäthers, mit einer Verbindung mit mindestens zwei gegenüber Zerewitinoff-aktiven Wasserstoffatomen reaktiven Gruppen im Verhältnis der Anzahl der OH-, NH-, $NH_2$-äquivalente des Polyäthers zur Anzahl der Äquivalenten der gegenüber Zerewitinoff-aktiven Wasserstoffatomen reaktiven Gruppen der organischen Verbindungen mit mindestens zwei gegenüber Zerewitinoff-aktiven Wasserstoffatomen reaktiven Gruppen wie (m-1):m (wobei m die Zahl der gegenüber Zerewitinoff-aktiven Wasserstoffatomen reaktiven Gruppen ist) umsetzt,
oder dass man

b) biologisch aktive Wirkstoffe mit mindestens einem Zerewitinoff-aktiven Wasserstoffatome im Molverhältnis 1:1 mit Verbindungen umsetzt, die m gegenüber Zerewitinoff-aktiven Wasserstoffatomen reaktive Gruppen besitzen, wobei m mindestens zwei ist und die erhaltenen Verbindungen anschliessend mit einem OH-, NH- oder $NH_2$-monofunktionellen hydrophilen Polyäther mit einem Wasseraufnahmevermögen von mindestens 15%, bezogen auf das Gewicht des Polyäthers im Äquivalentverhältnis m:m-1 umsetzt
oder dass man

c) biologisch aktive Wirkstoffe mit mindestens einem Zerewitinoff-aktiven Wasserstoffatom, und
Verbindungen mit m gegenüber Zerewitinoff-aktiven Wasserstoffatomen reaktiven Gruppen (wobei m mindestens zwei ist), und
OH-, NH- oder $NH_2$-monofunktionelle hydrophile Polyäther mit einem Wasseraufnahmevermögen von mindestens 15% bezogen auf das Gewicht des Polyäthers,
im Äquivalentverhältnis 1:m:(m-1) umsetzt.

Es wurden ferner neue Verbindungen gfunden, die dadurch gekennzeichnet sind, dass sie hergestellt werden, indem man

a) biologisch aktive Wirkstoffe mit mindestens einem Zerewitinoff-aktiven Wasserstoffatom im Molverhältnis 1:1 mit Verbindungen umsetzt, die erhalten werden, indem man einen OH-, NH- oder $NH_2$-monofunktionellen hydrophilen Polyäther mit einem Wasseraufnahmevermögen von mindestens 15% bezogen auf das Gewicht des Polyäthers mit einer organischen Verbindung mit mindestens zwei gegenüber Zerewitinoff-aktiven Wasserstoffatomen reaktiven Gruppen im Verhältnis der Anzahl der OH-, NH-, $NH_2$-Äquivalente des Polyäthers zu Anzahl der Äquivalente der gegenüber Zerewitinoff-aktiven Wasserstoffatomen reaktiven Gruppen der organischen Verbindungen mit mindestens zwei gegenüber Zerewitinoff-aktiven Wasserstoffatomen reaktiven Gruppen, d.h. wie (m-1):m (wobei m die Zahl der gegenüber Zerewitinoff-aktiven Waserstoffatomen reaktiven Gruppen ist) umsetzt,
oder dass man

b) biologisch aktive Wirkstoffe mit mindestens einem Zerewitinoff-aktiven Wasserstoffatom im Molverhältnis 1:1 mit Verbindungen umsetzt, die m gegenüber Zerewitinoff-aktiven Wasserstoffatomen reaktive Gruppen besitzen (wobei m mindestens zwei ist), und die erhaltenen Verbindungen anschliessend mit einem OH-, NH- oder $NH_2$-monofunktinoellen hydrophilen Polyäther mit einem Wasseraufnahmevermögen von mindestens 15%, bezogen auf das Gewicht des Polyäthers im Äquivalentverhältnis m:(m-1), umsetzt,
oder dass man

c) biologisch aktive Wirkstoffe mit mindestens einem Zerewitinoff-aktiven Wasserstoffatom, und

Verbindungen mit m gegenüber Zerewitinoff-aktiven Wasserstoffatomen reaktiven Gruppen (wobei m mindestens zwei ist), und

OH-, NH- oder $NH_2$-monofunktionelle hydrophile Polyäther mit einem Wasseraufnahmevermögen von mindestens 15%, bezogen auf das Gewicht des Polyäthers,

im Äquivalentverhältnis 1:m:(m-1) umsetzt.

Unter dem Begriff Zerewitinoff-aktives Wasserstoffatom versteht man ein Wasserstoffatom, welches in einer organischen Verbindung an ein Zentrum gebunden ist, welches, verglichen mit einem C-Atom eines Kohlenwasserstoffs, eine stark elektronenabziehende Wirkung ausübt. Im engeren Sinne bedeutet Zerewitinoff-aktiv ein im Sinne der Reaktion: $CH_3MgJ + H\text{-}X \rightarrow CH_4 + JMgX$ aktives H-Atom. (s.a. Beyer Lehrbuch der organischen Chemie (1968) Seite 147.)

Die so erhaltenen neuen Verbindungen weisen überraschenderweise bei gleichbleibender und zum Teil verbesserter oder erweiterter biologischer Aktivität verglichen mit den Ausgangswirkstoffen eine wesentlich verbesserte Löslichkeit in Wasser und niederen aliphatischen Alkoholen auf.

Die Tatsache, dass die nach dem erfindungsgemässen Verfahren erhaltenen Wirkstoffe, trotz einer das Molekulargewicht verändernden Modifizierung eine, verglichen mit den Ausgangswirkstoffen gleichbleibende Wirkung aufweisen, kann darauf zurückzuführen sein, dass im Organismus des Tieres oder der Pflanze der zur Löslichmachung dienende Polyätherteil, welcher über das di- oder mehrfunktionelle, gegenüber Zerewitinoff-aktiven Wasserstoffatomen reaktive Koppelglied an den Wirkstoff gebunden ist, wieder abgespalten werden kann. Dabei geht offenbar die Bindung des Koppelgliedes an den Wirkstoff leichter auf als die Bindung des Koppelgliedes an den Polyäther, was sich im Falle der Verwendung von Lysinesterdiisocyanat durch die unterschiedliche Reaktivität der beiden funktionellen Gruppen erklären lässt.

Als OH-, NH- oder $NH_2$-monofunktionelle hydrophile Polyäther kommen prinzipiell alle Polyäther mit einer -OH-, -NH- oder -$NH_2$-Endgruppe und mit mindestens zwei bis höchstens vierhundert Äthylenoxideinheiten sowie Mischblockpolyäther mit zusätzlichen Propylenoxideinheiten in Frage. Bevorzugt verwendet man Polyäther der allgemeinen Formel (I)

$$R^1\text{-}O\text{-}[C_2H_4O]_p\text{-}[i\text{-}C_3H_7\text{-}O]_q\text{-}[C_2H_4O]_r\text{-}H$$

in welcher

$R^1$ für Reste von Startermolekülen, die ein im Sinne der Alkoxylierungsreaktion aktives Wasserstoffatom aufweisen steht und

p, q und r für ganze Zahlen zwischen 0 und 400 stehen, wobei mindestens einer der Indizes p, oder r für eine ganze Zahl grösser als 2 stehen muss.

Damit der Polyäther ein Wasseraufnahmevermögen von mindestens 15%, bezogen auf das Gewicht des Polyäthers erhält, muss das Verhältnis $(p+r):q \geqq 0{,}5$ sein.

Als Startermoleküle dienen z.B. einwertige Alkohole, Phenole oder sekundäre Amine, bevorzugt für Methanol, Äthanol, 1-Propanol, 2-Propanol, 1-Butanol, 2-Butanol, 2-Methyl-2-Propanol, 1-Pentanol, 3-Methyl-1-butanol, 2,2-Dimethylpropanol, 1-Hexanol, 4-Methyl-2-pentanol, 2-Äthyl-1-butanol, 1-Octanol, 2-Äthyl-1-hexanol, 1-Nonanol, 1-Decanol, 1-Dodecanol, 1-Tetradecanol, 1-Hexadecanol, 1-Octadecanol, 1,2-Diäthoxy-2-propanol, Cyclohexanol, 2-Methylcyclohexanol, 3-Methylcyclohexanol, 4-Methylcyclohexanol, Cyclohexanmethanol, 3,3,5-Trimethylcyclohexanol, 4-tert.-Butylcyclohexanol, 2-Methylphenol, 3-Methylphenol, 4-Methylphenol, Benzylalkohol, 2-Äthylphenol, 1-Phenyläthanol, 2-Phenyläthanol, 2,3-Dimethylphenol, 3,4-Dimethylphenol, 2,6-Dimethylphenol, 2,4-Dimethylphenol, 3,5-Dimethylphenol, 3-Phenyl-1-propanol, 2-Isopropylphenol, 3-Äthyl-5-Methylphenol, 2,3,5-Trimethylphenol, 2-(1-Methyl-propyl)-phenol, 2-(1,1-Dimethyläthyl)-phenol, 4-(1,1-Dimethyläthyl)-phenol, 3-Methyl-5-(1-methyläthyl)-phenol, 5-Methyl-2-(1-methyläthyl)-phenol, 4-Methoxyphenol, 4-Methoxy-benzylalkohol, Dimethylamin, Diäthylamin, Di-n-propylamin, Di-iso-propylamin, Di-n-butylamin, Di-iso-butylamin, Bis-(2-äthylhexyl)amin, Methyloctadecylamin, N-Methylcyclohexylamin, N-Äthylcyclohexylamin, 2-Methyl cyclohexylamin, 3-Methylcyclohexylamin, 4-Methylcyclohexylamin, N-Methylanilin, N-Äthylanilin, N-Propylanilin, N-Butylanilin, N-Isobutylanilin, Diphenylamin, N,2-Dimethylanilin, N-Äthyl-2-methylanilin, N-Äthyl-3-methylanilin, N-Butyl-3-methylanilin, N,4-Dimethylanilin, N-Äthyl-4-Methylanilin, Methylbenzylamin, Phenylbenzylamin.

Besonders bevorzugt verwendet man Polyäther der allgemeinen Formel (Ia)

$$R^1\text{-}O\text{-}(CH_2CH_2O)_n\text{-}CH_2CH_2OH \qquad \text{(Ia)}$$

in denen

$R^1$ für Reste von Monoalkoholen mit 1–6 C-Atomen steht und

n eine ganze Zahl zwischen 1 und 160 ist.

Ganz besonders bevorzugt sind Polyäther, in denen $R^1$ für n-Butyl steht und n eine ganze Zahl zwischen 1 und 90 ist. Die beim erfindungsgemässen Verfahren einzusetzenden Polyäther bzw. Mischblockpolyäther müssen ein Wasseraufnahmevermögen von mindestens 15% bezogen auf das Gewicht des Polyäthers bzw. Mischblockpolyäthers besitzen.

Als Verbindungen mit mindestens zwei gegenüber Zerewitinoff-aktiven Wasserstoffatomen reaktiven Gruppen verwendet man bevorzugt Verbindungen der allgemeinen Formel (II)

$$X\text{-}R^2\text{-}Y \qquad \text{(II)}$$

in welcher

$R^2$ für substituierte oder unsubstituierte zweiwertige aliphatische, cycloaliphatische, araliphatische oder aromatische Kohlenwasserstoffreste steht.

Bevorzugt steht dabei $R^2$ für zweiwertige aliphatische Kohlenwasserstoffreste mit 2-40 C-Atomen, insbesondere mit 2-18 C-Atomen, die gegebenenfalls durch Halogen, Cyan, Nitro, gegebenenfalls substituiertes Alkyl- oder Arylmercapto, Alkoxycarbonyl, Alkoxysulfonyl, Alkoxyphosphoryl, Halogencarbonyl, Amidoyl, Alkoxy, Aryloxy, Aryloxycarbonyl, Aryloxysulfonyl, Aryloxyphosphoryl substituiert sind, wobei Aryl für gegebenenfalls durch $C_{1-6}$-Alkyl, Halogen, Cyano, Nitro, gegebenenfalls substituiertes Alkoxy, Alkylmercapto, Aryloxy, Arylmercapto, Halogenalkyl substituiertes Phenyl oder Naphthyl steht.

Ferner steht $R^2$ bevorzugt für cycloaliphatische Kohlenwasserstoffreste mit 4-15 C-Atomen oder für aromatische Kohlenwasserstoffreste mit 6-15 C-Atomen, die jeweils gegebenenfalls ein- oder mehrfach substituiert sind durch Alkyl mit 1 bis 20 C-Atomen, welches gegebenenfalls ein- oder mehrfach durch Halogen, Cyano, Nitro, gegebenenfalls substituiertes Alkylmercapto, Aryl (wobei Aryl bedeutet Phenyl oder Naphthyl, welches gegebenenfalls ein- oder mehrfach durch Alkyl mit 1-6 C-Atomen, Halogen, Cyano, gegebenenfalls substituiertes Alkoxy, Alkylmercapto, Arylmercapto, Halogenalkyl substituiert ist), Alkoxycarbonyl, Alkoxysulfonyl, Alkoxyphosphoryl, Halogencarbonyl, Amidoyl, Alkoxy, Aryloxy, Arylmercapto, Aryloxycarbonyl, Aryloxysulfonyl, Aryloxyphosphoryl, wobei Aryl jeweils die oben angegebene Bedeutung besitzt, substituiert ist, durch Cycloalkyl mit 5-20 C-Atomen, welches gegebenenfalls ein- oder mehrfach durch Alkyl mit 1-6 C-Atomen, Halogen, Cyano, Nitro, gegebenenfalls substituiertes Alkylmercapto, Alkoxycarbonyl, Alkoxysulfonyl, Alkoxyphosphoryl, Halogencarbonyl, Amidoyl, Alkoxy, Aryl, Aryloxy, Arylmercapto, Aryloxycarbonyl, Aryloxysulfonyl, Aryloxyphosphoryl, wobei Aryl jeweils die oben angegebene Bedeutung besitzt, substituiert ist, durch Phenyl oder Naphthyl, welches gegebenenfalls ein- oder mehrfach durch Alkyl, Halogen, CN, gegebenenfalls substituiertes Alkoxy, Alkylmercapto, Alkoxycarbonyl, Alkoxysulfonyl, Alkoxyphosphoryl, Halogencarbonyl, Amidoyl, Aryloxy, Arylmercapto, Aryloxycarbonyl, Aryloxysulfonyl, Aryloxyphosphoryl, wobei Aryl die oben angegebene Bedeutung besitzt, substituiert ist, durch Halogen, Cyano, Nitro, Alkoxycarbonyl, Alkoxysulfonyl, Alkoxyphosphoryl, Halogencarbonyl, Amidoyl, gegebenenfalls substituiertes Alkoxy, Alkylmercapto, Aryloxy, Arylmercapto, Aryloxycarbonyl, Aryloxysulfonyl, Aryloxyphosphoryl, wobei Aryl jeweils die oben angegebene Bedeutung besitzt. Ferner steht $R^2$ bevorzugt für araliphatische Kohlenwasserstoffreste mit 7-15 C-Atomen, wobei einer der Reste X oder Y an den aliphatischen Teil und der andere Rest X oder Y an den aromatischen Teil gebunden ist, oder beide Reste X und Y an den aliphatischen Teil gebunden sind. Sowohl der aliphatische als auch der aromatische Teil können durch die bei den aromatischen Kohlenwasserstoffen weiter oben angegebenen Substituenten substituiert sein.

X oder Y stehen für gleiche oder verschiedene, gegenüber Zerewitinoff-aktiven Wasserstoffatomen reaktive funktionelle Gruppen wie beispielsweise Halogencarbonyl, Alkoxycarbonyl, Carboxyl, Carbonsäureanhydrid, Sulfonsäure, Sulfonsäurehalogenid, Sulfonsäurealkylester, Phosphorsäure, Phosphorsäurehalogenid, Phosphorsäurealkylester, Isothiocyanat, Isocyanat, oder Isocyaniddihalogenid.

Besonders bevorzugt steht $R^2$ für zweiwertige aliphatische Kohlenwasserstoffreste mit 2-18 C-Atomen, die gegebenenfalls durch Halogen, Cyano, Nitro, Alkoxycarbonyl, Alkoxysulfonyl, Alkoxyphosphoryl, Alkoxy, Aryloxy, Aryloxycarbonyl, Aryloxysulfonyl, Aryloxyphosphoryl, wobei Aryl die oben angegebene Bedeutung besitzt, substituiert sind.

Ferner steht $R^2$ besonders bevorzugt für zweiwertige cycloaliphatische Kohlenwasserstoffreste mit 5-10 C-Atomen, aromatische Kohlenwasserstoffreste mit 6-13 C-Atomen, die jeweils gegebenenfalls ein- oder mehrfach substituiert sind durch Alkyl mit 1-4 C-Atomen, welches gegebenenfalls wie oben angegeben substituiert ist und/oder durch Halogen, Cyano, Nitro, Alkoxycarbonyl, Alkoxysulfonyl, Alkoxyphosphoryl, Alkoxy, Aryloxy, Aryloxycarbonyl, Aryloxysulfonyl, Aryloxyphosphoryl, wobei Aryl die oben angegebene Bedeutung besitzt.

Ferner steht $R^2$ besonders bevorzugt für zweiwertige araliphatische Reste mit 8-13 C-Atomen, die jeweils gegebenenfalls ein- oder mehrfach substituiert sind durch Halogen, Cyano, Nitro, Alkoxycarbonyl, Alkoxysulfonyl, Alkoxyphosphoryl, gegebenenfalls substituiertes Alkoxy, Alkylmercapto, Aryloxy, Arylmercapto, Aryloxycarbonyl, Aryloxysulfonyl, Aryloxyphosphoryl, wobei Aryl die oben angegebene Bedeutung besitzt. Zusätzlich zu den angegebenen Substituenten kann der aromatische Teil des araliphatischen Restes durch $C_{1-4}$-Alkyl oder Halogenalkyl substituiert sein.

X oder Y stehen besonders bevorzugt für gleiche oder verschiedene Reste wie Halogencarbonyl, Alkoxycarbonyl, Carboxyl, Carbonsäureanhydrid oder Isocyanat.

Ganz besonders bevorzugt sind Verbindungen der allgemeinen Formel (II), in welcher $R^2$ für einen zweiwertigen aliphatischen Kohlenwasserstoffrest mit 2-8 C-Atomen steht, der gegebenenfalls ein- oder mehrfach substituiert ist durch $C_{1-4}$-Alkoxycarbonyl, und X und Y für Isocyanat steht.

In diesem Zusammenhang seien genannt, z.B. Bernsteinsäure, Adipinsäure, Korksäure, Azelainsäure, Sebacinsäure, Phthalsäure, Isophthalsäure, Trimellitsäure, Tetrahydrophthalsäureanhydrid, Endomethylentetrahydrophthalsäureanhydrid, Glutarsäureanhydrid, Maleinsäure, Maleinsäureanhydrid, Fumarsäure, Ölsäure sowie Äthylendiisocyanat, 1,12-Dodecandiisocyanat, Cyclobutan-1,3-diisocyanat, Cyclohexan-1,4-diisocyanat, 1-Isocyanato-3,3,5-trimethyl-5-isocyanatomethylcyclohexan (DE-Auslegeschrift 1202785, US-Patentschrift 3401190), 2,4- und 2,6-Hexahydrotoluylendiisocyanat, Hexahydro-1,3- und/oder -1,4-phenylendiisocyanat, Perhydro-2,4'- und/oder -4,4'-diphenylmethandiisocyanat, 1,3- und 1,4-Phenylendiisocyanat, 2,4- und 2,6-Toluylendiisocyanat, Diphenylmethan-2,4- und -4,4'-diisocyanat, Naphthylen-1,5-diisocyanat. Besonders bevorzugt sind Hexamethylendiisocyanat, Tetramethylendiisocyanat, Isophorondiisocyanat, Toluylen-diisocyanat und 1,6-Diisocyanato-hexansäuremethylester.

Als biologisch aktive Wirkstoffe mit mindestens einem Zerewitinoff-aktiven Wasserstoffatom werden Verbindungen eingesetzt, die auf dem Gebiet des Pflanzenschutzes sowie der Human- und Tiermedizin wirksam sind. Als Wirkstoffe auf dem Gebiet des Pflanzenschutzes seien unter anderem genannt: Insektizide, Akarizide, Fungizide, Bakterizide, Mikrobizide, Herbizide, Pflanzenwachstumsregulatoren, Rodentizide, Nematizide.

Als Wirkstoffe auf dem Gebiet der Humanmedizin und Tiermedizin seien unter anderem genannt: Arzneimittel zur Schmerzbekämpfung, Hypnotica, Psychopharmaka, Analeptika, Appetitzügler, Antiphlogistica, Antihistaminica, Hämologica, Diuretica, Antidiuretica, Arzneimittel mit Wirkung auf Herz und Gefässe, Gefässwände und Blutlipide, Magen-Darm-Trakt und auf den Respirationstrakt, Antimykotica, Antihelmintika, Antibakterielle Mittel, Mittel gegen Protozoenerkrankungen. Voraussetzung ist, dass diese Wirkstoffe mindestens ein Zerewitinoff-aktives Wasserstoffatom besitzen.

Besonders erwähnt seien in diesem Zusammenhang Wirkstoffe, die eine oder mehrere $>$N-H Gruppen im Molekül aufweisen: Dazu gehören unter anderem Derivate von stickstoffhaltigen Heterocyclen wie Pyrol, Indol, Carbazol, Pyrazol, Imidazol, Benzimidazol, Hydantoin, Oxazol, Isoxazol, Triazol, Oxadiazol, Tetrazol, Pyridin, Chinolin, Isochinolin, Pyridazin, Pyrimidin, Uracil, Barbitursäure, Pyrin, Chinazolin, Pyrazin, Pteridin, Phenazin, Oxazin (1,2 und 1,3 und 1,4) Triazin (v, as, s), Thiazin (1,2 und 1,3 und 1,4), Thiadiazin oder Harnstoffderivate, Carbamate, Hydroxylaminderivate, Aminoalkohole, Aminophenole, Aminocarbonsäuren, Carbonsäurehydrazide, Amidine, Thioharnstoffderivate, Guanidinderivate, Cyansäurederivate, oder Naturstoffe (natürlich, halbsynthetisch oder synthetisch hergestellt) wie beispielsweise Aminosäuren, Peptide, Kohlenhydrate, Nucleinsäuren mit freier NH-Valenz.

Ferner seien in diesem Zusammenhang erwähnt biologisch aktive Alkohole, Carbonsäuren, Sulfonsäuren, Sulfensäuren, Sulfinsäuren, Mercaptane, Kohlensäure oder Phosphor- bzw. Phosphonsäuren bzw. deren Derivate.

Als Verbindungen mit einer Stickstoffheterocyclengruppierung im Molekül seien insbesondere genannt: Triazolderivate, Thiazolderivate, Imidazolderivate, Pyrimidinderivate, Uracilderivate und Triazinderivate. Besonders hervorzuheben sind dabei die nachfolgend aufgeführten Verbindungen: 2-Amino-4-methyl-5-carboxanilido-thiazol, 3-Amino-1,2,4-triazol, 2-Methoxy-4-äthylamino-6-isopropylamino-s-triazin, 5-Brom-3-sec.-butyl-6-methyluracil, 4-o-Chlorphenylhydrazinyl-3-methylisoxazol-5-on, 2,4-Dichloro-6-(o-chloranilin)-s-triazin, 5-Amino-1-bis(dimethylamido)-phosphoryl-3-phenyl-1,2,4-triazol, 2,4-Dimethyl-5-carboxanilidothiazol, O,O-Dimethyl-S-(4,6-diamino-1,3,5-triazin-2-yl)methylphosphordithioat, 4-(2-Chlorphenylhydrazonyl)-3-methylisoxazol-5-on, 3,3'-Äthylen-bis (tetrahydro-4,6-dimethyl-2H-, 1,3,5-thiadiazin-2-thion), 2-(2'-Furyl)-benzimidazol, S-(4,6-Diamino-1,3,5-triazinyl-methyl)-dimethylphosphordithioat, 3-Methyl-5-carboxymethyl-tetrahydro-1,3,5-thiazin-2-thion, 2,4-Bis-isopropylamino-6-methylmercapto-s-triazin, 2-Methylamino-4-isopropylamino-6-methylmercapto-s-triazin, 2-Isopropylamino-4-äthylamino-6-methylmercapto-s-triazin, 2-Äthylamino-4-tert.-butylamino-6-methylmercapto-s-triazin, 2-Isopropylamino-4-methoxypropylamino-6-methyl mercapto-s-triazin, 1-2-(3'-Pyridyl)pyrrolidin, Dibenzo-1,4-thiazin, 2-Methoxy-4,6-bis(isopropylamino)-s-triazin, 2-Chlor-4,6-bis(isopropylamino)-s-triazin, 5-Amino-4-chlor-2-phenyl-3(2H)-pyridazinon, 2-Chlor-4,6-bis-äthylamino-s-triazin, 1-(p-Sulfamylphenyl)-2,5-dimethyl-4-nitroso-pyrazol, 3-Cyclohexyl-5,6-trimethylenuracil, 4-Amino-6-tert.-butyl-3-(methylthio)-1,2,4-triazin-5-(4H)-on, 3-Methyl-4-amino-6-phenyl-1,2,4-triazin-5-(4H)-on, 1,3-Di-p-chlorphenyl-(1H,2H,3H,4H,5H,6H)-1,3,5-triazin-2,4-dion, 7-Chlor-2,3-dihydro-2,2-dihydroxy-5-phenyl-(1H)-benzo(f)-1,4-diazepin-3-carbonsäure, 3,5-Dioxo-2,3,4,5-tetrahydro-1,2,4-triazin, 2-Nitroimidazol, 2-Amino-5-nitrothiazol, D-4-Amino-3-isoxazolidinon, 1,3-Dimethyl-(1H,2H,3H,4H,5H,6H)-1,3,5-triazin-2,4-dion, 1,3-Diisopropyl-(1H,2H,3H,4H,5H,6H)-1,3,5-triazin-2,4-dion, 5,5-Dibrom-hexahydropyrimidin-2,4,6-trion, 5-Fluorpyrimidin-2,4-diol, 4-Amino-5-fluorpyrimidin-2-(1H)-on, 2-Thioxo-hexahydropyrimidin-4,6-dion, 1,3-Di-p-fluorphenyl-(1H,2H,3H,4H,5H,6H)-1,3,5-triazin-2,4-dion, 2-Amino-5-nitropyrimidin, 5-Amino-(1H)-vic.triazolo(d)-pyrimidin-7-ol, 1,3-Bis-4-Trifluormethylphenyl-(1H,2H,3H,4H,5H,6H)-1,3,5-triazin-2,4-dion, 1-Methyl-2-mercapto-imidazol, 2-Amino-4-methylthiazol, 4-Aminoperhydro-1,2-oxazin-3-on, 3,5-Dijod-4(1H)-pyridon, 1H-Pyrazolo-(3,4,d)-pyrimidin-4-ol, 1H-Pyrazolo-(3,4,d)pyrimidin-4-thiol, 6-Mercaptopurin, 6-Aminopurin, 6-Aminopyrin-8-(9H)-on, 4-Methyl-3-thiouracil, 1,3-Bis-4-Chlor-3-trifluormethylphenyl-(1H,2H,3H,4H,5H,6H)-1,3,5-triazin-2,4-dion, 6-Carbamoyl-3-hydarzino-pyridazin, 2-(5-Nitro-2-furyl)-5-amino-1,3,4-thiadiazol, Trimethylolmelamin, N-Guanidinoformimidoyl-morpholin, 4-Amino-2,2,5,5-tetrakis(trifluormethyl)-3-imidazolin, 6-Chlor-7-sulfamoyl-(2H)-1,2,4-benzothiadiazin-1,1-dioxid, 1,3-Bis-3-Chlor-4-trifluormethylphe-

nyl-(1H,2H,3H,4H,5H,6H)-1,3,5-triazin-2,4-dion, 1,3-Dimethylpurin-2,6(1H,3H)-dion, 4-Amino-1-ribofuranosyl-1,3,5-triazin-2-(1H)-on, 2-Amino-5-p-Chlorphenyl-2-oxazolin, 2-Sulfanilamidothiazol, 3,3-Diethylpyridin-2,4(1H,3H)-dion, 3-Äthyl-1-allyl-6-aminouracil, 3(5)-Ribofuranosyl-4-hydroxy-pyrazol-5(3)-carboxamid, 5-Äthyl-5-isopropyl-hexahydropyrimidin-2,4,6-trion, 3-Chlor-4-(3-Chlor-2-nitrophenyl)-pyrrol, 5,7-Dichlor-2-methylchinolin-8-ol, 2-Amino-5-(4-trifluormethylphenyl)-2-oxazolin, 2-Sulfanilamidopyrimidin, 1,3-Bis-(3,4-Dichlorphenyl)-(1H,2H,3H,4H,5H,6H)-1,3,5-triazin-2,4-dion, 1-(3-Aminophenyl)-2(1H)-pyridon, 4-Benzyl-2-thiouracil, 4-Amino-6-methyl-2-phenyl-3(2H)-pyridazinon, 2-(p-Aminobenzolsulfonamido)-4-methylpyrimidin, 2-(p-Aminobenzolsulfonamido)-5-methylpyrimidin, 3-(p-aminobenzolsulfonamido)-6-methoxy-pyridazin, 2-(p-Aminobenzolsulfonamido)-5-methoxypyrimidin, 4-(p-Aminobenzolsulfonamido)-6-methoxypyrimidin, 2-(p-Aminobenzolsulfonamido)-3-methoxypyrazin, 2-(p-Aminobenzolsulfonamido)-4,5-dimethyloxazol, 5-(p-Aminobenzolsulfonamido)-3,4-dimethylisoxazol, 2-Sulfonanilamid-4,6-dimethoxy-1,3,5-triazin, 1,3-Bis-(p-Chlor-4-phenoxyphenyl)-(1H,2H,3H,4H,5H,6H)-1,3,5-triazin-2,4-dion, 4,6-Diamino-1-(p-Chlorphenyl)-1,2-dihydro-2,2-dimethyl-1,3,5-triazin, 5-Allyl-5-isobutyl-2-thiobarbitursäure, 4,6-Dimethyl-2-sulfanilamido-pyrimidin, 2,4-Dimethyl-6-sulfanilamido-pyrimidin, 5,5-Diallylbarbitursäure, 4-Hydroxy-1H-pyrazolo(3,4,d)pyrimidin, 6-(1-Methyl-4-nitroimidazolyl-5-thio)-purin, 5,5-Diäthylbarbitursäure, 4-Methyl-4-äthyl-piperidin-2,6-dion, 3-Benzyl-6-trifluormethyl-7-sulfamoyl-3,4-dihydro-3,4-dihydro-2H-1,2,4-benzothiadiazin-1,1-dioxid, l1-l1-[3-(4-Fluorbenzoyl)-propyl]-4-piperidyl}-benzimidazolon, 2-(2-Methylaminoäthyl)-piperidin, 7-Brom-2-oxo-5-(2-pyridyl)-2,3-dihydro-1H-1,4-benzodiazepin, 3-Hydroxylmethyl-6-oxo-5-semicarbazono-2,3,5,6-tetrahydro-indol, 7-Chlor-2-methylamino-5-phenyl-3H-1,4-benzodiazepin-4-oxid, 7-Chlor-4-[(4-Diäthylamino-1-methyl-butyl)-amino]-chinolin, 5-Chlor-1,3-benzoxazolon, 5-(2-Chlorphenyl-7-nitro-2-oxo-2,3-dihydro-1H-1,4-benzodiazepin, 2-(2,6-Dichloranilino)-$\Delta^2$-imidazolin, 5-(3-Methylaminopropyl)-10,11-dihydro-5H-dibenzo(b,f)azepin, 5-Amino-1,3-bis-(2-äthylhexyl)-5-methylhexahydropyrimidin, 5-Methylisoxazol-3-carbonsäure-($N^2$-benzylhydrazid), Pyridin-4-carbonsäurehydrazid, 2-(1-Naphthylmethyl)- $\Delta^2$-imidazolin, 8-Amino-2-methyl-4-phenyl-1,2,3,4-tetrahydroisochinolin, 2-(4-tert.-Butyl-3-hydroxy-2,6-dimethylbenzyl)-$\Delta^2$-imidazolin, 2-(2,2--Dicyclohexyläthyl)-piperidin, 4-(2-Hydroxy-3-isopropylaminopropyloxy)-indol, 6-Chlor-2-methyl-7-sulfamoyl-3-(2,2,2-trifluoräthylmercaptomethyl)-3,4-dihydro-2H-1,2,4-benzothiadiazin-1,1-dioxid, 4-Aminobenzolsulfonsäure-(4,5-dimethyl-1,3-oxazol-2-yl-guanid), 3-(4-Aminobenzolsulfonamido)-5-methylisoxazol, 4-(4-Aminobenzolsulfonamido)-2,6-dimethylpiperidin, 2-(4-1,-3-Thiazolyl)-benzimidazol, 2-(5-Tetralylamino)-$\Delta^2$-imidazolin, 2-(4-tert.-Butyl-2,6-dimethylbenzyl)-$\Delta^2$-imidazolin, 2,4-Dimethoxy-6-sulfanilamido-pyrimidin, 5,6-Dihydro-2-(2,6-xylidino)-4H-1,3-thiazin, Triacetyl-6-azauridin, 1-(4-Chlor-3-sulfamoylbenzamido)-2,6-dimethylpiperidin, 1-Phenyl-5-sulfanilamidopyrazol, 4-(m-Hydroxyphenyl)-1-methyl-4-propionylpiperidin, 3-[3-(3-Hydroxy-2-piperidyl)-acetonyl]-4-(3H)-chinazolon, 4-(4-Diäthylamino-1-methylbutylamino)-7-chlor-chinolin, 2-(2,2-Dicyclohexylvinyl)-piperidin, 1-Methyl-2-(β-hydroxy-β-phenyläthyl)-6-phenacylpiperidin, Diäthylbarbitursäure, Äthylcyclohexenylbarbitursäure, Methyl-cyclohexenylmethylbarbitursäure, 2,6-Dimethyl-3,5-diacetyl-4-o-nitrophenyl-1,4-dihydropyridin, N-(4′-Chlor-3′-sulfamoyl-benzolsulfonyl)-N-methyl-2-aminomethyl-2-methyltetrahydrofuran, 2-(4-Sulfamoylphenyl)-tetrahydro-2H-1,2-thiazin-1,1-dioxid, 5-Acetamido-2-sulfamoyl-1,3,4-thiadiazol.

Als Wirkstoffe aus der Reihe der Harnstoffderivate seien genannt:

1-(1-Naphthyl)-2-thioharnstoff, 3-(p-Bromphenyl)-1-methyl-1-methoxyharnstoff, 3-(p-Chlorphenyl)-1,1-dimethylharnstoff, 3-[p(p-Chlorphenoxy-phenyl)]-1,1-dimethylharnstoff, 1,3-Bis-(1-hydroxy-2,2,2-trichloräthyl)harnstoff, 1,1-Dimethyl-3-(3-(N-tert.-butylcarbamyloxy)-phenyl)-harnstoff, 3-(3,4-Dichlorphenyl)-1,1-dimethylharnstoff, 3-Phenyl-1,1-dimethylharnstoff, 1,1-Dimethyl-3-(m-trifluormethylphenyl)-harnstoff, 3-(3,4-Dichlorphenyl)-1-methoxy-1-methylharnstoff, 3-(4-Chlorphenyl)-1-methoxy-1-methylharnstoff, 3-(3,4-Dichlorphenyl)-1-n-butyl-1-methylharnstoff, 1-(2-Methylcyclohexyl)-3-phenylharnstoff, 3-(2-Benzothiazolyl)-1-methylharnstoff, 3-(4-Brom-3-chlorphenyl)-1-methoxy-1-methylharnstoff, 3-Cyclooctyl-1,1-dimethylharnstoff, 3-(3-Methylphenyl)-1,1-dimethylthioharnstoff, 3-[5-(3a,4,5,6,7,7a-hexahydro-4-methanindanyl)]-1,1-dimethylharnstoff, 1,3-Dimethyl-3-(2-benzthiazolyl)-harnstoff, 3-[3-Chlor-4-(2′-chlor-4′-nitrophenoxy)phenyl]-1-(2,2′-dichlorbenzoyl)-harnstoff, 3-[4-(2-Chlor-4-trifluormethylphenoxy)phenyl]-1-(2,2′-difluorbenzoyl)-harnstoff, 3-[4-(Chlor-4-trifluormethylphenoxy)phenyl]-1-o-chlorbenzoylharnstoff, 3-(4-Chlorphenyl)-1-(2,2′-difluorbenzoyl)-harnstoff, 3-(4-Nitrophenyl)-1-(2,2′-difluorbenzoyl)-harnstoff, 3-(4-Trifluormethoxyphenyl)-1-o-chlorbenzoylharnstoff, 3-(4-Trifluormethoxyphenyl)-1-(2,2-dichlorbenzoyl)-harnstoff, 1,3-Bis-(3,4-Dichlorphenyl)-harnstoff, Phenylthioharnstoff, 1,3-Bis-(4-Dichlorphenyl)-harnstoff, 1-p-Chlorphenyl-3-o-chlorphenylharnstoff, 1-p-Chlorphenyl-3-o,m-dichlorphenylharnstoff, 1-p-Chlorphenyl-3-o,p-dichlorphenylharnstoff, 1-p-Chlorphenyl-3-(2,5-dichlorphenyl)-harnstoff, 1-Hydroxymethyl-3-methyl-2-thioharnstoff, 2-Äthylisothioharnstoff, Allylthioharnstoff, N,N-Dimethylbiquanid, 5-Nitro-2-furaldehydsemicar-

bazon, 1-Äthyl-3-(5-nitro-2-thiazolyl)-harnstoff, 2-Äthyl-cis-crotonoylharnstoff, α-Äthyl-α-brombutyryl-harnstoff, α-Chlorphenylacetyl-harnstoff, p-Äthoxyphenyl-harnstoff, Vanillinthiosemicarbazon, N-Acetyl-N'-diäthylbromacetyl-harnstoff, 2-Isopropyl-4-pentenoyl-harnstoff, p-Acetamidobenzaldehydthiosemicarbazon, 1-(p-Chlorphenylsulfonyl)-3-propyl-harnstoff, 2-Phenylbutyrylharnstoff, p-(3-Äthylureido)-benzaldehydthiosemicarbazon, 1-Butyl-3-sulfanilylharnstoff, Phenyläthylmalonylharnstoff, N,N'-(m-Amidinophenyl)-harnstoff, p-Cinnamoyloxyphenyl-harnstoff, 1-(Hexahydro-1H-azepin-1-yl)-3-{p[5-(methyl-3-isoxazolcarboxamido)-äthyl]-phenylsulfonyl]-harnstoff, N-Acetyl-N'-(2,2-Diäthyl-2-brom)-acetyl-harnstoff, α-Brom-isovaleroyl-harnstoff, 2-β-Aminoäthyl-isothioharnstoff.

Als Wirkstoffe aus der Reihe der Carbamate seien genannt:

4-Benzothienyl-N-methylcarbamat, 1-Naphthyl-N-methylcarbamat, 2-Chlorphenyl-N-methylcarbamat, Isopropyl-N-(m-chlorphenyl)-carbamat, 3,4-Dimethyl-N-methylcarbamat, 4-Dimethylamino-3,5-xylyl-N-methylcarbamat, 2,6-Di-tert.-butyl-p-tolyl-N-methylcarbamat, 3-(1-Methylbutyl)-phenyl-N-methylcarbamat, 3-Isopropyl-phenyl-N-methylcarbamat, 4-(Methylthio)-3,5-xylyl-N-methylcarbamat, N-3,4-Dichlorphenyl-O-methylcarbamat, N-Phenyl-O-isopropylcarbamat, 2-Isopropoxyphenyl-N-methylcarbamat, m-Tolyl-N-methylcarbamat, 3,4-Xylyl-6-Chlor-4-methylcarbamat, O-methyl-N-4-aminobenzolsulfonylcarbamat, 1-Methyl-prop-2-inyl-N-(3-chlorphenyl)-carbamat, 2,3-Dihydro-2,2-dimethyl-7-benzofuranyl-N-methylcarbamat, 2-Isopropylphenyl-N-methylcarbamat, 3,5-Dimethyl-4-methylmercaptophenyl-N-methylcarbamat, 2-Chlorphenyl-N-o,o'-difluorbenzoylcarbamat, 4-Chlorphenyl-N-o,o'-difluorbenzoylcarbamat, Methyl-N-o,o'-difluorbenzoylcarbamat, Isopropyl-N-o,o'-dilfuorbenzoylcarbamat, 2,6-Dichlorphenyl-N-o,o'-difluorbenzoylcarbamat, 2,6-Dichlorphenyl-N-o,o'-dichlorbenzoylcarbamat, 2-Nitrophenyl-N-o,o'-difluorbenzoylcarbamat, 4-Nitrophenyl-N-o,o'-difluorbenzoylcarbamat, 2-Nitro-4-chlorphenyl-N-o,o'-difluorbenzoylcarbamat, 2-Chlor-4-nitrophenyl-N-o,o'-difluorbenzoylcarbamat, N-o,o'-Difluorbenzoyl-S-p-chlorphenyl-carbaminicacidthioester, 1-Methyl-5-nitroazol-2-yl-methylcarbamat, 1-Äthyl-1-methylpropylcarbamat, 3-Methyl-2,4-pentandioldicarbamat, 1-Äthinylcyclohexylcarbamat, 2-Methyl-2.n-propyl-1,3-propandioldicarbamat, α-Äthinylbenzylcarbamat, 3-(p-Chlorphenoxy)-2-hydroxypropylcarbamat, α-Methylphenyläthylcarbamat, 2-Hydroxyäthyl-N-benzylcarbamat, 1-Cyclohexyl-propylcarbamat, α-Isopropyl-benzylcarbamat, (2-Methyl-2-nonyl-1,3-dioxolan-4-yl-)-methylcarbamat, 2-Methyl-2-propylpropan-1,3-dioldicarbamat.

Als Wirkstoffe aus der Reihe der Carbon-, Sulfon- und Phosphorsäurederivate seien genannt:

N-[2-Mercaptoäthyl-benzolsulfonamido-S(O,O'-diisopropylphosphordithioat)], 4-Tert.-butyl-2-chlorphenyl-O,N-dimethylphosphorimidat, 3-Amino-2,5-dichlorbenzoesäure, N-(3-Chlor-4-methylphenyl)-2-methylpentanamid, O,O-Dimethyl-(2,2,2-trichlor-hydroxyäthyl)phosphonat, 5,2'-Dichlor-4'-nitro-salicylanilid-äthanolamin, O,O-Dimethyl-S-(N-methylcarbamoylmethyl)-thiophosphat, O-Äthyl-O-(3-methyl-4-methylthiophenyl)-isopropylamido-phosphat, 3,4-Dichlorphenylpropionanilid, 5-Methyl-1,3,4-thiadiazol-2-sulfonamid, L-2-Amino-3-(N-nitroso-hydroxyamino)-propionsäure, Pyrazol-1-carboxamid, Pyrazol-1-thiocarboxamid, 2-Acetylamino-1,3,4-thiadiazol-5-sulfonamid, Pyrazincarboxamid, 5-Acetylimino-4-methyl-$\Delta^2$-1,3,4-thiadiazolin-2-sulfonamid, Pyridin-3-carboxamid, 4-Amino-3-jodbenzolsulfonamid, Pyridin-4-carbonsäurehydrazid, p-Aminobenzolsulfonamid, 2-Methyl-4-aminopyrimidin-5-carbonsäurehydrazid, α-Aminotoluol-p-sulfonamid, 2-Äthyl-isonicotinsäurethioamid, α,α-Dichlor-N-[β-(hydroxymethyl)-p-nitrophenacyl]-acetamid, D(−)-threo-N-(β-Hydroxy-α-hydroxymethyl-p-nitrophenyläthyl)-dichloracetamid, D(−)-threo-N-(β-Hydroxy-α-hydroxymethyl-p-nitrophenyläthyl)-azidoacetamid, N-Sulfanilyl-3-methyl-2-butenamid, N-(2-Chlor-4-nitrophenyl)-5-chlorsalicylamid, p-Aminosalicylsäurephenylester, 4-Chlor-$N^1$-methyl-$N^1$-(2-methyltetrahydrofurfuryl)-m-benzoldisulfonamid, 2-Diäthylaminoäthyl-p-aminobenzoat, 2-(Diäthylaminoacetylamino), -3-methylbenzoesäuremethylester, 2-Äthyl-6-methyl-α-diäthylamino-acetanilid, β-Dimethylaminoäthyl-p-butylaminobenzoat, D-7-(2-Amino-2-phenylacetamido)-3-methyl-8-oxo-5-thia-1-azabicyclo-(4.2.O)-oct-2-en-2-carbonsäure, β-Diäthylaminoäthyl-3-amino-4-propoxybenzoat, 7-Chlor-4-dimethylamino-1,4,4a,5,5a,6,11,12a-oktahydro-3,6,10,12,12a-pentahydroxy-1,11-dioxo-2-naphthacencarboxamid, 2,2-Diallyl-N,N'-bis-(4-Amino-2-methyl-6-chinolyl)-malonamid, 4-Methyl-3-(2-propylamino-propionylamino)-thiophen-2-carbonsäuremethylester, 4-Chlor-5-sulfamoyl-salicylsäure-2,6-dimethylanilid.

Als Wirkstoffe aus der Reihe der Naturstoffe seien insbesondere genannt:

2-Äthyl-isonicotinthioamid, 5-Hydroxytryptamin, (−)-3-(3,4-Dihydroxyphenyl)-2-methylalanin, 7-(2,3-Dihydroxypropyl)-theophyllin, α-1-(Methylaminoäthyl)-benzylalkohol, DL-α-Methyltryptamin, 4-(2,2'-Dichloräthylamino)-prolinäthylester, p-[Bis-(2-Chloräthyl)-amino]-L-phenylalanin, D-4-Amino-4-carboxybutylpenicillin, 2',4-Epoxy-3-(4'-hydroxyphenyl)-7-hydroxycumarin, 4-Hydroxybenzylpenicillin, 6-[D(−)-α-Aminophenylacetamido)]-penicillansäure, 7-(5-Amino-5-carboxyvaleramido)-cephalosporansäure, 6-(3-Amino-2-phenylpropionamido)-penicillansäure, 6-(α-Phenoxybutyramido)-penicillansäure, 7-(2-(α-Methylphenäthylamino)-äthyl)-theophyllin, N-(2-Hydroxy-1-methyläthyl)-D-(+)-lysergamid, 4-Chlor-17β-hydroxyandrost-4-en-3-on, $N^2$-{p-[1-(2-Amino-4-hydroxy-6-pteridinyl)-äthyl-methylamino]-benzoyl}-glutamin, 1,4-Pregnadien-17α-21-diol-3,11,20-trion, 7-Chlor-4-dimethylamino-1,4,4a,5,5a,6,11,12a-oktahydro-3,6,10,12,12a-pentahydroxy-6-methyl-1,11-dioxo-2-naphthacencarboxamid, 9-Acetyl-7,8,9,10-tetrahydro-6,7,9,11-tetrahydroxy-4-methoxy-5,12-naphthacenchinon-7-(3-amino-5-methyl-2,3-didesoxy-L-lyxopyranosid, 2α,11-Dimethoxy-3-(3,4,5-trimethoxybenzoyloxy)-yohimbin-1-carbonsäuremethylester, O-[2,6-Diamino-2,3,4,6-tetradesoxy-D-glycero-hex-4-enpyranosyl(1→4)]-O-[3-desoxy-4-C-methyl-3-methylamino-β-L-arabinopyranosyl-(1→6)]-2-desoxy-D-streptamin, 1-β-D-Arabinofuranosylcytosin-5′-(1-adamantancarboxylat), 6-Aminopenicillansäure, D-α-Aminobenzoylaminopenicillansäure, L-3-(3,4-Dihydroxyphenyl)-2-methylalanin, 6-[(R)-2-(2-Oxo-imidazolidin-1-carboxamido)-2-phenylacetamido]-penicillansäure, 7-(D-α-Amino-phenylacetamido)-3-methyl-3-cephem-4-carbonsäure, 7-[2-(1-Methyl-2-phenyläthylamino)-äthyl]-theophyllin.

Besonders zu erwähnen ist der herbizide Wirkstoff 4-Amino-6-tert.-butyl-3-methylthio-1,2,4-triazin-5-(4H)-on, welcher nach Modifizierung gemäss vorliegender Erfindung zusätzlich insektizide Wirkung aufweist.

Bei der Durchführung der erfindungsgemässen Verfahrensvariante a) geht man vorteilhaft in der nachstehend beschriebenen Weise vor.

Verwendet man beispielsweise einen auf n-Butanol gestarteten Äthylenoxidpolyäther mit einer endständigen OH-Gruppe und Hexamethylendiisocyanat sowie 4-Amino-6-tert.-butyl-3-(methylthio)-1,2,4-triazin-5-(4H)-on als Wirkstoff, so ist das erfindungsgemässe Verfahren wie folgt durchzuführen:

Man entwässert die einem Äquivalent OH-Gruppen entsprechende Menge des Äthylenoxidpolyäthers während 10 Minuten bis 2 Stunden, vorzugsweise 20 Minuten bis 60 Minuten im Vakuum bei 1333 bis 26666 Pa bei einer Temperatur von 60 bis 160°C, vorzugsweise 80 bis 120°C und führt anschliessend mit einem Inertgas den Ausgleich mit dem Aussendruck durch. Man gibt 0,01 bis 1 Gew.-%, bezogen auf die verbliebene Menge an Äthylenoxidpolyäther eines organischen Carbonsäurehalogenids, vorzugsweise Benzoylchlorid, zu und rührt bei 60–160°C, vorzugsweise 70–120°C, 1 bis 30 Minuten, vorzugsweise 5 bis 10 Minuten, nach. Man bestimmt die verbliebene OH-Äquivalentmenge titrimetrisch an einer Probe. Anschliessend gibt man eine der verbliebenen OH-Menge äquivalente Menge an Hexamethylendiisocyanat hinzu, so dass ein Äquivalentverhältnis von OH:NCO = 1:2 eingehalten wird. Man rührt nach bei einer Temperatur von 60–160°C, bevorzugt 80–100°C, wobei darauf geachtet wird, dass keine Feuchtigkeit in die Apparatur gelangt, bis der berechnete Rest-Isocyanatgehalt erreicht ist. Man kühlt ab und erhält ein Polyätherisocyanat der idealisierten Formel (IV):

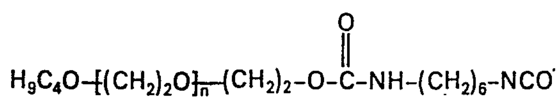
(IV)

in der
n die oben angegebene Bedeutung hat.

Dieses Polyätherisocyanat wird in einer zweiten, sich an die erste unmittelbar anschliessenden Stufe des Verfahrens bei einer Temperatur von 25–150°C, bevorzugt 80–140°C und unter Feuchtigkeitsausschluss mit dem Wirkstoff umgesetzt, wobei der Wirkstoff in einem inerten Lösungsmittel gelöst sein kann. Die Reaktion ist vollständig abgelaufen, wenn in der Reaktionsmischung IR-spektroskopisch kein Isocyanat mehr nachgewiesen werden kann. Der modifizierte Wirkstoff hat die allgemeine idealisierte Formel V:

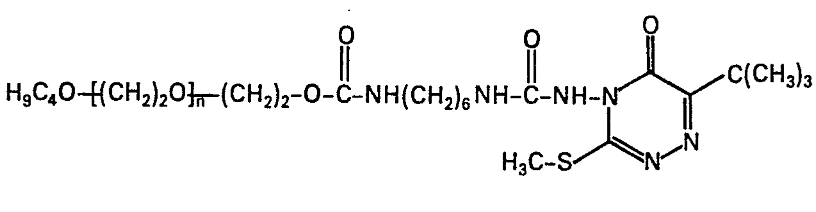
(V)

in welcher
n die oben angegebene Bedeutung hat.

In analoger Weise können selbstverständlich auch Umsetzungen anderer Polyäther mit Hydroxylendgruppe mit anderen Di- oder Polyisocyanaten oder Di- bzw. Polyisothiocyanaten und anderen mindestens ein Zerewitinoff-aktives Wasserstoffatom pro Molekül aufweisenden Wirkstoffen durchgeführt werden.

Eine weitere Möglichkeit zur Herstellung der erfindungsgemässen Substanzen besteht in der Verwendung eines Polyäthers mit einer Hydroxylendgruppe und einer Dicarbonsäure bzw. eines Dicarbonsäurederivates, bevorzugt eines Dicarbonsäureanhydrids oder eines Dicarbonsäuredimethylesters, als Ausgangskomponenten und eines, mindestens ein Zerewitinoff-aktives Wasserstoffatom aufweisenden Wirkstoffes, beispielsweise eines Triazin-2,4-dions.

Die für die in der zweiten Stufe ablaufende Umsetzung notwendigen Polyätherester können in an sich bekannter und üblicher Weise hergestellt werden. Dazu bieten sich insbesondere die beiden nachstehenden Verfahren an.

Im ersten Fall geht man von einer mineralsäurefreien Dicarbonsäure aus, die gegebenenfalls

durch Umkristallisieren zu reinigen ist. Als zweite Ausgangskomponente wird der gewünschte Polyätheralkohol zugegeben, wobei das Äqauivalentverhältnis -OH : -COOH = 1:2 ist. Die Reaktionskomponenten werden nach Zugabe von 0,05 bis 0,5 Gew.-%, vorzugsweise 0,05 bis 0,2 Gew.-%, eines Katalysators, z.B. einer Zinnverbindung, wie Di-n-butylzinnoxid, Di-n-butylzinndiester u.a., oder Titanester insbesondere Tetraisopropyltitanat, in einer geeigneten Apparatur unter Durchleiten eines Inertgases, z.B. Stickstoff, erhitzt. Bei etwa 180°C kommt es zur ersten Wasserabspaltung. Das Wasser wird destillativ aus dem Reaktionsgemisch entfernt. Innerhalb von mehreren Stunden wird die Reaktionstemperatur bis auf 240°C erhöht. Das Reaktionsmedium bleibt bis kurz vor Ende der vollständigen Veresterung inhomogen. Nach etwa 24 Stunden ist die Reaktion abgeschlossen.

Bei dem zweiten Verfahren geht man von Dicarbonsäuredimethylester als Ausgangskomponente aus und estert unter Durchleiten eines Inertgasstromes, z.B. Stickstoff, mit dem gewünschten Polyätheralkohol um. Als Umesterungskatalysatoren können wieder Titanester, Dialkylzinnester oder Di-n-butylzinnoxid in Konzentrationen von 0,005 bis 0,5 Gew.-% eingesetzt werden. Nach dem Erreichen von etwa 120°C kommt es zur ersten Methanolabspaltung. Innerhalb von mehreren Stunden wird die Temperatur auf 220 bis 230°C gesteigert. Je nach gewähltem Ansatz ist die Umesterung nach 2 bis 24 Stunden abgeschlossen.

Die weitere Reaktion der Polyäthercarbonsäure, entstanden nach der ersten Variante bzw. des Polyäthercarbonsäureester, entstanden nach der zweiten Variante mit dem einzusetzenden Wirkstoff, bevorzugt einer NH-funktionellen Verbindung, erfolgt nach an sich bekannten Methoden.

Im Falle der Polyäthercarbonsäure als Ausgangsverbindung setzt man gegebenenfalls in Gegenwart eines geeigneten organischen Lösungsmittels mit einem niederen aliphatischen Alkohol, bevorzugt Methanol, zum Polyäthercarbonsäureester um.

In beiden Fällen wird dann der Polyäthercarbonsäureester in einem geeigneten organischen Lösungsmittel bei einer Temperatur, welche ausreicht, entstehendes Methanol abdestillieren zu lassen, bevorzugt zwischen 80 und 140°C, mit der Wirkstoffkomponente, im Falle einer NH-funktionellen Verbindung zum entsprechenden Amid, umgesetzt.

Dabei ist es häufig von besonderem Vorteil, bei der Reaktion katalytische Mengen eines Alkalimetalls, insbesondere Natrium, zuzugeben, da hierdurch die Reaktionszeiten erheblich verkürzt werden können.

Die Reaktion ist beendet, wenn kein Methanol mehr abdestilliert werden kann, üblicherweise nach 1 bis 24 Stunden. Man lässt dann abkühlen, entfernt das Lösungsmittel und kristallisiert den entstandenen, erfindungsgemäss modifizierten Wirkstoff gegebenenfalls um.

Im Falle der Verwendung eines auf Butanol gestarteten Polyäthers und Tetramethylendicarbonsäuredimethylesters und des 1,3-Di-p-chlorphenyl-(1H,2H,3H,4H,5H,6H)-triazin-2,4-dion als Wirkstoff lässt sich die Verfahrensweise formelhaft in nachstehender Weise darstellen:

Stufe 1:

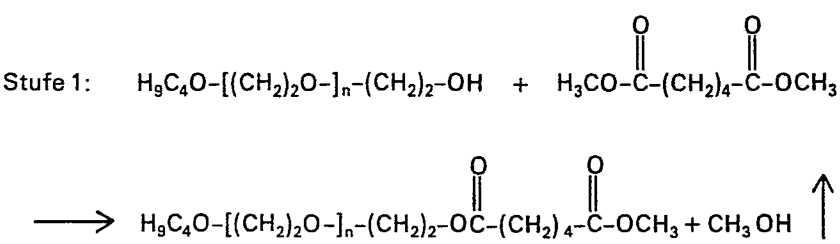

Stufe 2:

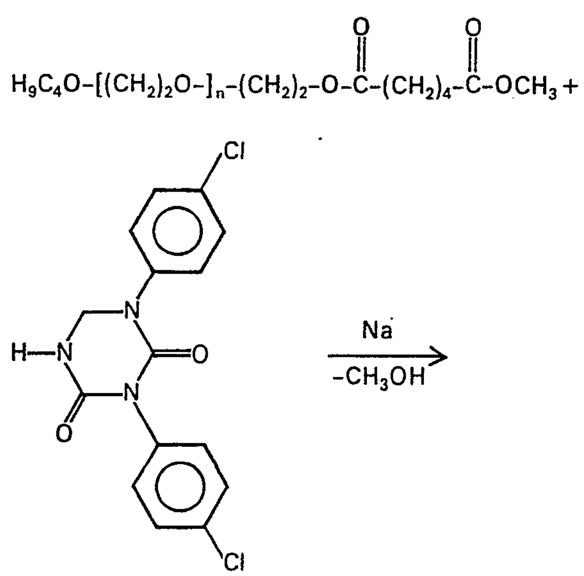

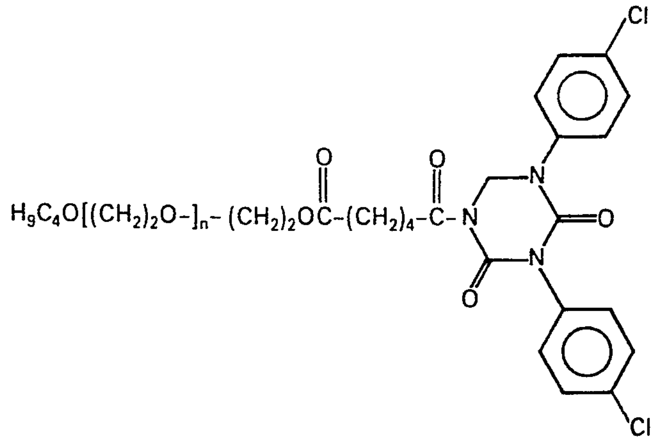

wobei
n die oben angegebene Bedeutung hat.

Eine weitere Möglichkeit zur Herstellung der erfindungsgemässen Substanzen besteht in der Verwendung eines Polyäthers mit einer Hydroxylendgruppe, eines Di-Kohlensäureesterchlorids und eines, mindestens ein Zerewitinoff-aktives Wasserstoffatom aufweisenden Wirkstoffes, beispielsweise eines Triazindions. Die für die in der zweiten Stufe ablaufende Umsetzung notwendigen Polyätherkohlensäureesterchloride können in an sich bekannter und üblicher Weise (vgl. Houben-Weyl-Methoden der organischen Chemie VIII, S. 101–105) hergestellt werden. Besonders bietet sich nachstehendes Verfahren an.

Man geht aus von einer bifunktionellen Verbindung mit zwei endständigen OH-Gruppen, beispielsweise Butandiol-1,4. Diese Verbindung wird in einem geeigneten organischen Lösungsmittel gelöst und bei Raumtemperatur mit Phosgen umgesetzt. Dazu wird zwischen ½ bis 10 Stunden Phosgen durch die Lösung geleitet, während gleichzeitig die Mischung durch externe Kühlung auf Raumtemperatur (max. 40°C) gehalten wird. Anschliessend wird auf 80–150°C, bevorzugt 100–120°C erhitzt, wodurch die Umsetzung vervollständigt und überschüssiges Phosgen entfernt wird. Wird beispielsweise Butandiol-1,4 verwendet, so liegt zu diesem Zeitpunkt Butan-1,4-bis-chlorkohlensäureester im jeweiligen Lösungsmittel in roher Form vor. Der Bischlorkohlensäureester wird dann mit dem monofunktionellen Polyäther zum Monochlorkohlensäure-monokohlensäurepolyätherester umgesetzt.

Dabei geht man zweckmässigerweise so vor, dass man den Bis-chlorkohlensäureester in einem geeigneten Lösungsmittel vorlegt, zusammen mit ausreichenden Mengen einer organischen und/oder anorganischen Base zum Binden der bei der Reaktion entstehenden Salzsäure und den hydroxylfunktionellen Polyäther sehr langsam zudosiert, wobei auf ein Äquivalent Hydroxylgruppen zwei Äquivalente Chlorkohlensäureesterendgruppen kommen. Man lässt das Gemisch dann ½ bis 12 Stunden, bevorzugt 1 bis 4 Stunden ausreagieren bei einer Temperatur von 10 bis 100°C, bevorzugt 30 bis 70°C. Das Basenhydrochlorid wird in an sich bekannter Weise abgetrennt. Der resultierende Mono-chlorkohlensäureester-mono-kohlensäurepolyätherester ist für die nachfolgende Umsetzung mit dem zu modifizierenden Wirkstoff rein genug.

Bei dieser Umsetzung mit dem Wirkstoff, insbesondere einer NH-funktionellen Verbindung, geht man vorzugsweise in nachstehend beschriebener Art vor. In einer Vorstufe wird der Mono-chlorkohlensäure-monokohlensäurepolyätherester mit äquivalenten Mengen Methanol zum Kohlensäuremethylester-kohlensäurepolyätherester bzw. im Falle des Butandiol-1,4 zum Butan-1,4-kohlensäuremethylester-kohlensäurepolyätherester umgesetzt. Derartige Veresterungen mit Methanol als Alkoholkomponente sind bekannt.

In der dieser Vorreaktion unmittelbar folgenden Reaktion wird der Kohlensäuremethylesterkohlensäurepolyätherester in einem geeigneten organischen Lösungsmittel bei einer Temperatur, welche ausreicht, entstehendes Methanol abdestillieren zu lassen, bevorzugt zwischen 80 und 140°C, mit der Wirkstoffkomponente, im Falle einer NH-funktionellen Verbindung zum entsprechenden Amid, umgesetzt. Dabei ist es häufig von besonderem Vorteil, bei der Reaktion katalytische Mengen eines Alkalimetalls, insbesondere Natrium, zuzugeben, da hierdurch die Reaktionszeiten erheblich verkürzt werden können. Die Reaktion ist beendet, wenn kein Methanol mehr abdestilliert werden kann, üblicherweise nach 1 bis 24 Std. Man lässt dann abkühlen, entfernt das Lösungsmittel und kristallisiert gegebenenfalls den entstandenen, nach dem erfindungsgemässen Verfahren modifizierten Wirkstoff um.

Im Falle der Verwendung eines auf Butanol gestarteten Polyäthers und Butan-1,4-bischlorkohlensäureester als Ausgangskomponenten und des 1,3-Dimethyl-(1H,2H,3H,4H,5H,6H)-triazin-2,4-dion als Wirkstoff lässt sich die oben angegebene Verfahrensweise formelhaft in nachstehender Weise darstellen.

Stufe 1:

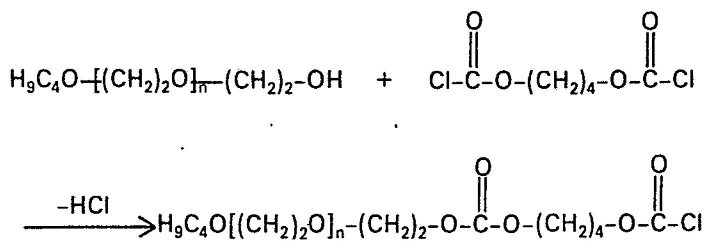


Stufe 2:

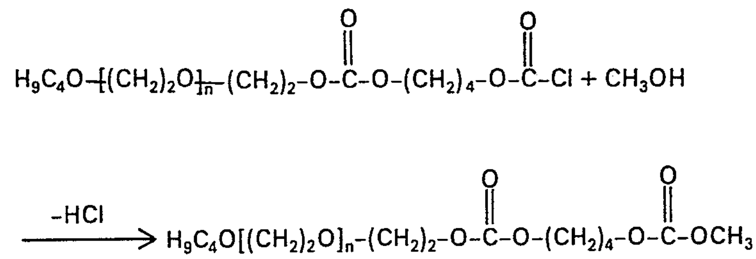


Stufe 3:

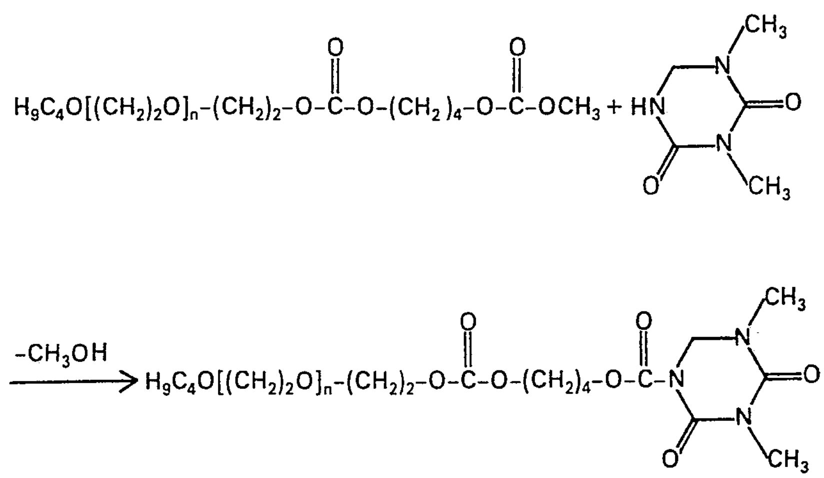


wobei
n die oben angegebene Bedeutung hat.

Als Verdünnungs- bzw. Lösungsmittel können bei dem erfindungsgemässen Verfahren alle inerten organischen Lösungsmittel in Frage kommen. Hierzu gehören vorzugsweise Benzol, Toluol, Xylol, Methylenchlorid, Chloroform, Chlorbenzol, Diäthyläther, Dibutyläther, Dioxan sowie Aceton, Methyläthylketon, Essigsäureäthylester und Acetonitril.

Bei der Durchführung der erfindungsgemässen Verfahrensvariante b) geht man vorteilhaft in der nachstehend beschriebenen Weise vor.

Verwendet man beispielsweise einen auf n-Butanol gestarteten Äthylenoxidpolyäther mit einer endständigen OH-Gruppe, Hexamethylendiisocyanat sowie 4-Amino-6-tert.-butyl-3-(methylthio)-1,2,4-triazin-5-(4H)-on, so ist das erfindungsgemässe Verfahren wie folgt durchzuführen.

Man entwässert die einem Äquivalent OH-Gruppen entsprechende Menge des Äthylenoxidpolyäthers während 10 Minuten bis 2 Stunden, vorzugsweise 20 Minuten bis 60 Minuten, im Vakuum bei 1333 bis 26666 Pa bei einer Temperatur von 60 bis 160°C, vorzugsweise 80 bis 120°C und führt anschliessend mit einem Inertgas den Ausgleich mit dem Aussendruck durch. Man gibt 0,01 bis 1 Gew.-%, bezogen auf die verbliebene Menge an Äthylenoxidpolyäther, eines organischen Carbonsäurehalogenids, vorzugsweise Benzoylchlorid, zu und rührt bei 60 bis 160°C, vorzugsweise 70 bis 120°C, 1 bis 30 Minuten, vorzugsweise 5 bis 10 Minuten, nach. Man bestimmt die verbliebene OH-Äquivalentmenge titrimetrisch an einer Probe. In einem zweiten Reaktionsgefäss wird dann eine solche Menge an Hexamethylendiisocyanat vorgelegt, dass bezogen auf verbliebene OH-Äquivalente des Polyäthers ein Äquivalentverhältnis von OH:NCO = 1:2 eingehalten wird. Hierzu wird unter starkem Rühren eine solche Menge an Wirkstoff, evtl. gelöst in einem geeigneten Lösungsmittel, zudosiert, dass ein Äquivalentverhältnis NCO-Gruppen:Zerewitinoff-aktive H-Atome = 2:1 eingehalten wird. Man rührt 10 Minuten bis 48 Stunden bei 40 bis 160°C, bevorzugt 60 bis 120°C, nach und gibt dann die gesamte Menge an entwässertem Polyäther hinzu. Die Reaktion ist vollständig abgelaufen, wenn in der Reaktionsmischung IR-spektroskopisch kein Isocyanat mehr nachgewiesen werden kann. Der modifizierte Wirkstoff hat die idealisierte Formel V.

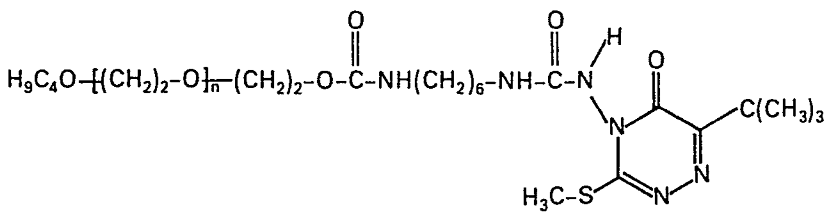

(V)

in welcher
n die oben angegebene Bedeutung hat.

Es sei in diesem Zusammenhang darauf hingewiesen, dass nur bei symmetrisch aufgebauten Verbindungen X-$R^2$-Y die sowohl nach Verfahrensvariante a) wie nach Verfahrensvariante b) erhältlichen Produkte dieselbe idealisierte Formel besitzen. Wird hingegen als Verbindung X-$R^2$-Y eine Verbindung gewählt, die nicht symmetrisch aufgebaut ist, und deren Endgruppen X und Y zwar strukturell gleich (z.B. beide NCO) aber von, bedingt durch die chemische Nachbarschaft, unterschiedlicher Reaktivität sind, so erhält man stets bevorzugt das Produkt, bei dem die reaktionsfähigere Endgruppe X oder Y mit der zuerst eingesetzten Verbindung, welche ein Zerewitinoff-aktives Wasserstoffatom aufweist, reagiert hat.

So erhält man beispielsweise beim Einsatz eines auf n-Butanol gestarteten Äthylenoxidpolyäthers, von Isophorondiisocyanat und von 3-Methyl-4-amino-6-phenyl-1,2,4-triazin-5-(4H)-on nach der Verfahrensvariante a) ein Produkt der idealisierten Formel VI

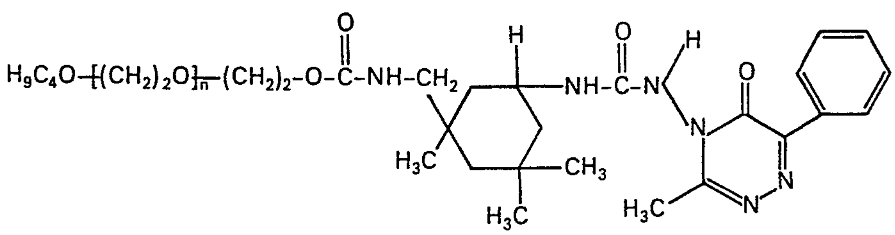

(VI)

in welcher
n die oben angegebene Bedeutung hat,

nach der Verfahrensvariante b) hingegen ein Produkt der idealisierten Formel VII

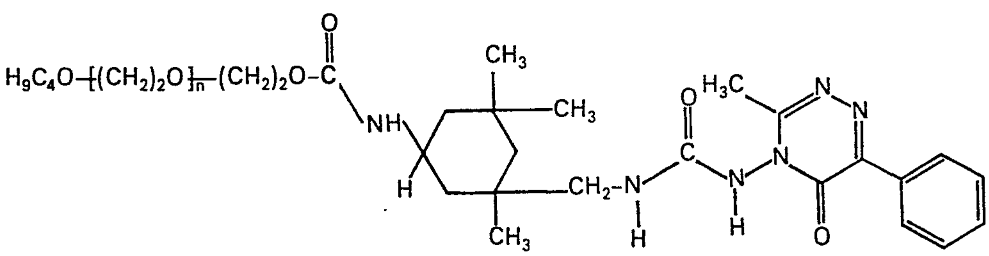

(VII)

in welcher
n die oben angegebene Bedeutung hat.

Bei der Durchführung der erfindungsgemässen Verfahrensvariante c), geht man vorteilhaft in der nachstehend beschriebenen Weise vor.

Verwendet man beispielsweise einen auf n-Butanol gestarteten Äthylenoxid-Propylenoxid-Mischblockpolyäther mit endständiger Hydroxyäthylgruppe, 1,6-Diisocyanatohexansäuremethylester und 2,6-Dimethyl-3,5-diacetyl-4-o-nitrophenyl-1,4-dihydropyridin, so ist das erfindungsgemässe Verfahren wie folgt durchzuführen.

Man entwässert die einem Äquivalent OH-Gruppen entsprechende Menge des Polyäthers während 5 Minuten bis 1 Stunde, vorzugsweise 10 Minuten bis 30 Minuten, im Vakuum bei 1333 bis 26666 Pa, bei einer Temperatur von 60 bis 160°C, vorzugsweise 80 bis 120°C und führt anschliessend mit einem Inertgas den Ausgleich mit dem Aussendruck durch. Man gibt dann 0,01 bis 1 Gew.-%, bezogen auf die verbliebene Menge an Polyäther, eines organischen Carbonsäurehalogenids, vorzugsweise Benzoylchlorid, zu und rührt bei 60 bis 160°C, vorzugsweise 70 bis 120°C, 1 bis 30 Minuten, vorzugsweise 5 bis 10 Minuten, nach. Man bestimmt die verbliebene OH-Äquivalentmenge titrimetrisch an einer Probe. Anschliessend gibt man im Verhältnis OH-Äquivalente des Polyäthers:Zerewitinoff-aktive H-Äquivalente des Wirkstoffes = 1:1 in einem geeigneten Lösungsmittel, zu. Die Mischung wird homogenisiert und danach mit der benötigten Menge Diisocyanat im Äquivalentverhältnis: Summe Zerewitinoff-aktive H-Äquivalente:Summe aller NCO-Äquivalente = 1:1 tropfenweise versetzt. Man rührt bei 60 bis 120°C unter Feuchtigkeitsausschluss 10 Minuten bis 48 Stunden nach. Gegebenenfalls zieht man anschliessend das Lösungsmittel im Vakuum ab. Der modifizierte Wirkstoff hat die idealisierte Formel VIII

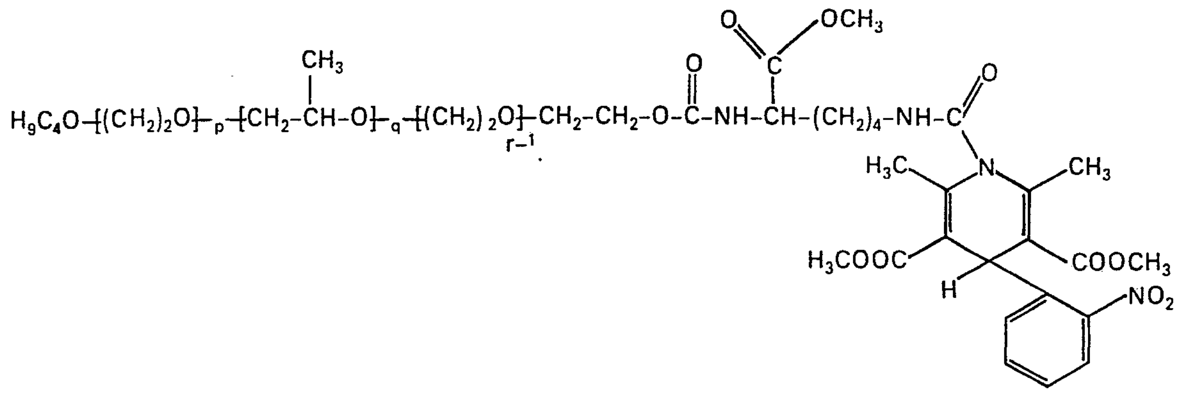

in der
p, q und r die oben angegebene Bedeutung haben.

Eine weitere Möglichkeit zur Herstellung der erfindungsgemässen Wirkstoffe besteht in der Verwendung eines Polyäthers mit endständiger N-(Isocyanatophenyl)-urethan-Gruppe, der, nach bekannten Verfahren aus einem Polyäther mit endständiger Hydroxylgruppe durch Umsetzung mit p-Nitrophenylisocyanat, anschliessender Reduktion der Nitrogruppe zur Aminogruppe und anschliessender Phosgenierung erhalten wird, und eines Wirkstoffes mit einem Zerewitinoff-aktiven Wasserstoffatom.

Bei der Durchführung dieses Verfahrens geht man zweckmässig in analoger Weise wie vorstehend beschrieben bei der Verwendung von Polyätherisocyanaten vor.

Verwendet man beispielsweise einen auf n-Butanol gestarteten Äthylenoxidpolyäther, welcher durch Umsetzung mit p-Nitrophenylisocyanat und weiter durch Reduktion und Phosgenierung zum Polyätherisocyanat abgewandelt wurde, und 2-Isopropyloxyphenyl-N-methylcarbamat, so hat der neue modifizierte Wirkstoff, welcher nach dem erfindungsgemässen Verfahren entsteht, die allgemeine idealisierte Formel

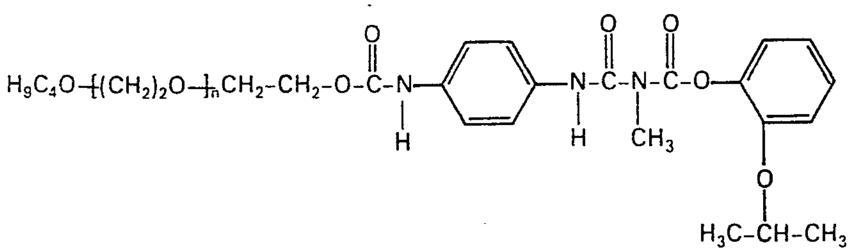

wobei
n die oben angegebene Bedeutung hat.

Wie bereits erwähnt, sind die nach dem erfindungsgemässen Verfahren modifizierten Wirkstoffe besser in Wasser oder niederen aliphatischen Alkoholen löslich als die unmodifizierten Wirkstoffe. Sie lassen sich daher einfacher und preiswerter einsetzen, da keine oder weniger Lösungsvermittler zur Herstellung der anwendungsfertigen Mittel erforderlich sind.

Die verbesserte Wasserlöslichkeit bewirkt ausserdem, dass auch bis dahin nicht-systemisch einsetzbare Wirkstoffe systemisch eingesetzt werden können, da sie nun durch den Saftstrom der Pflanze transportiert werden können.

Die folgenden Herstellungsbeispiele erläutern das erfindungsgemässe Verfahren anhand von Beispielen, bei denen als Ausgangskomponente auf Butanol gestartete Äthylenoxidpolyäther eingesetzt wurden, ohne jedoch den Erfindungsgegenstand auf diese Beispiele und Verfahrensweisen zu beschränken.

Herstellungsbeispiele

Beispiel 1a

600 g (0,3 mol) eines auf n-Butanol gestarteten monofunktionellen Äthylenoxidpolyäthers vom mittleren Molgewicht 2000 (mittlere Anzahl an Äthylenoxideinheiten n = 43) und der OH-Zahl 27,6 werden während 30 Min. bei 20 mbar und 120°C gerührt, wodurch evtl. vorhandene Wasserspuren entfernt werden. Man belüftet dann mit trockenem Stickstoff und gibt 3 ml Benzoylchlorid zu. Noch einmal wird auf 20 mbar evakuiert und anschliessend wieder mit Stickstoff belüftet. Man lässt auf 90°C abkühlen und gibt dann 63,7 g (0,3 mol) 1,6-Diisocyanatohexansäuremethylester zu. Nach 25 Min. ist der NCO-Gehalt auf den berechneten Wert von 1,9 Gew.-% gesunken. Man kühlt ab und erhält 662 g eines weisslichgelben Produktes der idealisierten Formel

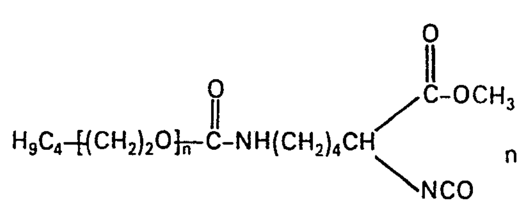

n = 43

welches mit gegenüber der verbliebenen NCO-Gruppe reaktiven Verbindungen ohne weitere Reinigung umgesetzt werden kann.

Beispiel 1b

600 g (0,3 mol ) eines auf n-Butanol gestarteten monofunktionellen Äthylenoxidpolyäthers vom mittleren Molgewicht 2000 (mittlere Anzahl an Äthylenoxideinheiten n = 43) und der OH-Zahl 27,6 werden analog zu Beispiel 1a) mit 50,5 g (0,3 mol) Hexamethylendiisocyanat umgesetzt. Nach 18 Min. ist der NCO-Gehalt auf den berechneten Wert von 1,9 Gew.-% abgesunken. Man erhält 648 g eines graugelben Produktes der idealisierten Formel

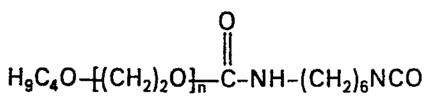

n = 43

Beispiel 1c

600 g (0,3 mol) eines auf n-Butanol gestarteten monofunktionellen Äthylenoxidpolyäthers vom mittleren Molgewicht 2000 (mittlere Anzahl an Äthylenoxideinheiten n = 43) und der OH-Zahl 27,6 werden zusammen mit 0,033 g (0,005 Gew.-%) Di-n-butylzinnoxid und 52,2 g (0,3 mol) Adipinsäuredimethylester auf 130°C erhitzt. Innerhalb von 2 Std. bei dieser Temperatur gehen ca. 4 g Methanol über. Man steigert die Temperatur innerhalb von 2 Std. auf 225°C. Nach Erreichen dieser Temperatur rührt man nach bis kein Methanol mehr übergeht (20 Min.) und lässt dann abkühlen. Man erhält 641 g eines dunkelgelben Produktes der idealisierten Formel

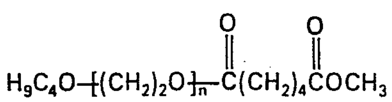

n = 43

Beispiel 2a

600 g (0,6 mol) eines auf n-Butanol gestarteten monofunktionellen Äthylenoxidpolyäthers vom mittleren Molgewicht 1002 (mittlere Anzahl an Äthylenoxideinheiten n = 21) und der OH-Zahl 56 werden während 30 Min. bei 20 mbar und 120°C gerührt, wodurch evtl. vorhandene Wasserspuren entfernt werden. Man belüftet dann mit trokkenem Stickstoff und gibt 3 ml Benzoylchlorid zu. Noch einmal wird auf 20 mbar evakuiert und anschliessend wieder mit Stickstoff belüftet. Man lässt auf 90°C abkühlen und gibt dann 127,3 g (0,6 mol) 1,6-Diisocyanatohexansäuremethylester zu. Nach 18 Min. ist der NCO-Gehalt auf den berechneten Wert von 3,5 Gew.-% gesunken. Man kühlt ab und erhält 721 g eines gelben Produktes der idealisierten Formel

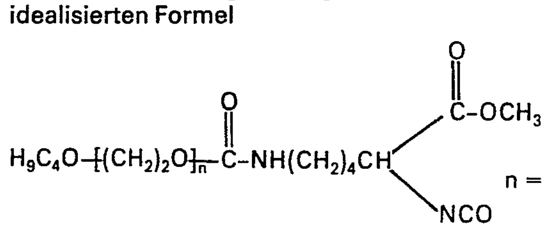

n = 21

welches mit gegenüber der verbliebenen NCO-Gruppe reaktiven Verbindungen ohne weitere Reinigung umgesetzt werden kann.

Beispiel 2b

600 g (0,6 mol) eines auf n-Butanol gestarteten monofunktionellen Äthylenoxidpolyäthers vom mittleren Molgewicht 1002 (mittlere Anzahl an Äthylenoxideinheiten n = 21) und der OH-Zahl 56 werden analog zu Beispiel 2a) mit 101 g (0,6 mol) Hexamethylendiisocyanat umgesetzt. Nach 16 Min. ist der NCO-Gehalt auf den berechneten Wert von 3,6 Gew.-% abgesunken. Man erhält 694 g eines graugelben seifenartigen Produktes der idealisierten Formel

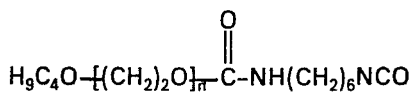

n = 21

Beispiel 3a

470 g (0,6 mol) eines auf n-Butanol gestarteten monofunktionellen Äthylenoxidpolyäthers vom mittleren Molgewicht 784 (mittlere Anzahl an Äthylenoxideinheiten n = 16) und der OH-Zahl 71,6 werden während 30 Min. bei 20 mbar und 120°C gerührt, wodurch evtl. vorhandene Wasserspuren entfernt werden. Man belüftet dann mit trockenem Stickstoff und gibt 3 ml Benzoylchlorid zu. Noch einmal wird auf 20 mbar evakuiert und anschliessend wieder mit Stickstoff belüftet. Man lässt auf 85°C abkühlen und gibt dann 127,3 g (0,6 mol) 1,6-Diisocyanatohexansäuremethylester zu. Nach 17 Min. ist der NCO-Gehalt auf den berechneten Wert von 4,2 Gew.-% gesunken. Man kühlt ab und erhält 593 g eines weissen Produktes der idealisierten Formel

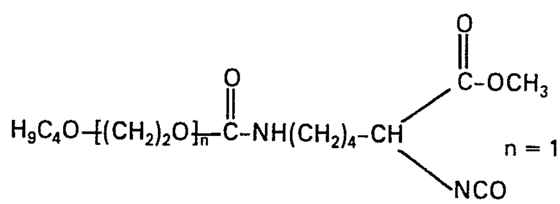

n = 16

welches mit gegenüber der verbliebenen NCO-Gruppe reaktiven Verbindungen ohne weitere Reinigung umgesetzt werden kann.

Beispiel 3b

470 g (0,6 mol) eines auf n-Butanol gestarteten monofunktionellen Äthylenoxidpolyäthers vom mittleren Molgewicht 784 (mittlere Anzahl an Äthylenoxideinheiten n = 16) und der OH-Zahl 71,6 werden analog zu Beispiel 3a mit 101 g (0,6 mol) Hexamethylendiisocyanat umgesetzt. Nach 12 Min. ist der NCO-Gehalt auf den berechneten Wert von 4,4 Gew.-% abgesunken. Man erhält 564 g eines fahlgelben seifenartigen Produktes der idealisierten Formel

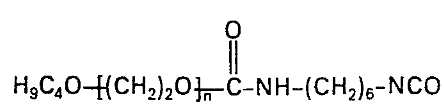

n = 16

Beispiel 3c

470 g (0,6 mol) eines auf n-Butanol gestarteten monofunktionellen Äthylenoxidpolyäthers vom

mittleren Molgewicht 784 (mittlere Anzahl an Äthylenoxideinheiten n = 16) und der OH-Zahl 71,6 werden analog zu Beispiel 1c mit 52,2 g (0,3 mol) Adipinsäuredimethylester in Gegenwart von 0,05 g Di-n-Butylzinnoxid umgesetzt. Gesamtreaktionszeit: 2½ Stunden.

Man erhält 521 g eines hochviskosen, rotgelben flüssigen Produktes der idealisierten Formel

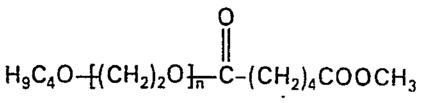

n = 16

Beispiel 4

312 g (0,6 mol) eines auf n-Butanol gestarteten monofunktionellen Äthylenoxidpolyäthers vom mittleren Molgewicht 519 (mittlere Anzahl an Äthylenoxideinheiten n = 10) und der OH-Zahl 108 werden während 30 Min. bei 20 mbar und 120°C gerührt, wodurch evtl. vorhandene Wasserspuren entfernt werden. Man belüftet dann mit trockenem Stickstoff und gibt 2 ml Benzoylchlorid zu. Noch einmal wird auf 20 mbar evakuiert und anschliessend wieder mit Stickstoff belüftet. Man lässt auf 80°C abkühlen und gibt dann 127,3 g (0,6 mol) 1,6-Diisocyanatohexansäuremethylester zu. Nach 9 Min. ist der NCO-Gehalt auf den berechneten Wert von 5,74 Gew.-% gefallen. Man kühlt ab und erhält 431 g eines gelblichen, flüssigen Produktes der idealisierten Formel

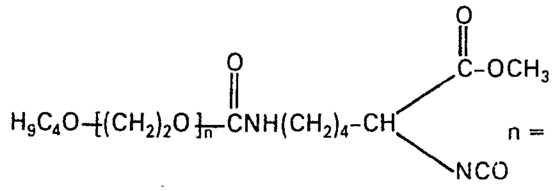

n = 10

welches mit gegenüber der verbliebenen NCO-Gruppe reaktiven Verbindungen ohne weitere Reinigung umgesetzt werden kann.

Beispiel 5

162,2 g (1,0 mol) 2-(2-Butoxyäthoxy)äthanol werden während 30 Min. bei 20 mbar und 80°C gerührt, wodurch evtl. vorhandene Feuchtigkeitsspuren entfernt werden. Man belüftet dann mit trockenem Stickstoff und gibt 1 ml Benzoylchlorid zu. Noch einmal wird auf 20 mbar evakuiert und anschliessend wieder mit Stickstoff belüftet. Man lässt auf 75°C abkühlen und gibt dann 212,2 g (1,0 mol) 1,6-Diisocyanatohexansäuremethylester zu. Nach 8 Min. ist der NCO-Gehalt auf den berechneten Wert von 11,2 Gew.-% gefallen. Man kühlt ab und erhält 371 g eines farblosen, flüssigen Produktes der idealisierten Formel

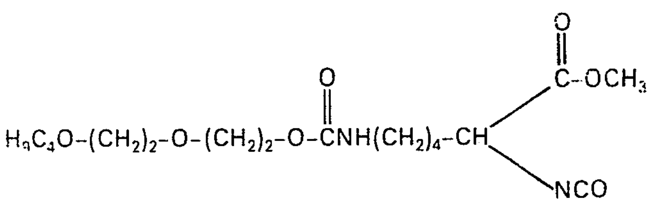

welches mit gegenüber der verbliebenen NCO-Gruppe reaktiven Verbindungen ohne weitere Reinigung umgesetzt werden kann.

welches mit gegenüber der verbliebenen NCO-Gruppe reaktiven Verbindungen ohne weitere Reinigung umgesetzt werden kann.

Beispiel 6

134,2 g (1,0 mol) 2-(2-Äthoxyäthoxy)äthanol werden während 30 Min. bei 20 mbar und 80°C gerührt, wodurch evtl. vorhandene Feuchtigkeitsspuren entfernt werden. Man belüftet dann mit trockenem Stickstoff und gibt 1 ml Benzoylchlorid zu. Noch einmal wird auf 20 mbar evakuiert und anschliessend wieder mit Stickstoff belüftet. Man lässt auf 70°C abkühlen und gibt dann 212,2 g (1,0 mol) 1,6-Diisocyanatohexansäuremethylester zu. Nach 12 Min. ist der NCO-Gehalt auf den berechneten Wert von 12,1 Gew.-% gesunken. Man kühlt ab und erhält 339 g eines farblosen, flüssigen Produktes der idealisierten Formel

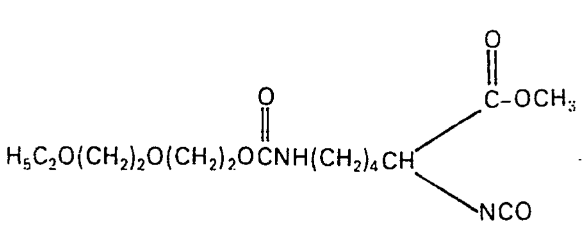

Beispiel 7

120,1 g (1,0 mol) 2-(2-Methoxyäthoxy)äthanol werden während 30 Min. bei 20 mbar und 80°C gerührt, wodurch evtl. vorhandene Feuchtigkeitsspuren entfernt werden. Man belüftet dann mit trockenem Stickstoff und gibt 1 ml Benzoylchlorid zu. Noch einmal wird auf 20 mbar evakuiert und anschliessend wieder mit Stickstoff belüftet. Man lässt auf 70°C abkühlen und gibt dann 212,2 g (1,0 mol) 1,6-Diisocyanatohexansäuremethylester zu. Nach 10 Min. ist der NCO-Gehalt auf den berechneten Wert von 12,6 Gew.-% gesunken. Man kühlt ab und erhält 227 g eines farblosen, flüssigen Produktes der idealisierten Formel

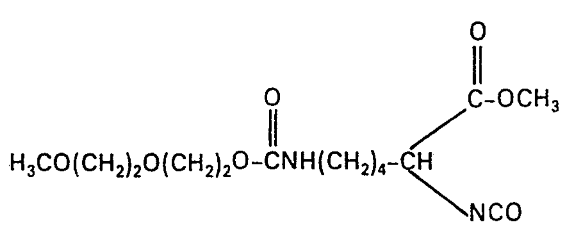

welches mit gegenüber der verbliebenen NCO-Gruppe reaktiven Verbindungen ohne weitere Reinigung umgesetzt werden kann.

Beispiel 8

148,2 g (1,0 mol) 1,3-Diäthoxy-2-propanol werden während 20 Min. bei 20 mbar und 90°C gerührt, wodurch evtl. vorhandene Feuchtigkeitsspuren entfernt werden. Man belüftet dann mit trockenem Stickstoff und gibt 1 ml Benzoylchlorid zu. Noch einmal wird auf 20 mbar evakuiert und anschliessend wieder mit Stickstoff belüftet. Man lässt auf 65°C abkühlen und gibt dann 212,2 g (1,0 mol) 1,6-Diisocyanatohexansäuremethylester zu. Nach 20 Min. ist der NCO-Gehalt auf den berechneten Wert von 11,7 Gew.-% gesunken. Man kühlt ab und erhält 351 g eines gelblichen, flüssigen Produktes der idealisierten Formel

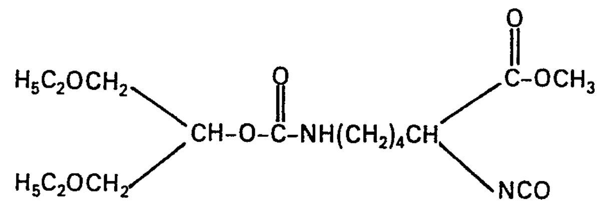

welches mit gegenüber der verbliebenen NCO-Gruppe reaktiven Verbindungen ohne weitere Reinigung umgesetzt werden kann.

Beispiel 9

22,1 g (0,01 mol) der nach Beispiel 1a erhaltenen Verbindung werden in 50 ml absolutem Toluol gelöst (Lösung 1) 3,4 g (0,01 mol) 1,3-Di-p-chlorphenyl-(1H, 2H, 3H, 4H, 5H, 6H)-triazin-2,4-dion werden in 50 ml absolutem Aceton gelöst (Lösung 2). Man legt Lösung 1 bei 40°C vor und tropft dann Lösung 2 sehr langsam zu, wobei schnell gerührt wird. Anschliessend wird bei 40°C und 200-300 mbar das Aceton abgezogen. Man belüftet mit trockenem Stickstoff. Anschliessend werden weitere 200 ml absolutes Toluol zugegeben und unter Feuchtigkeitsausschluss solange am Rückfluss gekocht, bis im IR-Spektrum die zur NCO-Gruppe gehörige Bande bei 2270 $cm^{-1}$ vernachlässigbar klein ist (1 Stunde). Anschliessend destilliert man das Toluol zuerst bei Normaldruck und danach im Wasserstrahlvakuum ab.

Als Rückstand erhält man 25,5 g eiens Feststoffes einer Erweichungstemperatur von 46-50°C. Dieser erfindungsgemäss modifizierte neue Wirkstoff ist sehr gut in Wasser und/oder niederen Alkoholen löslich und besitzt die idealisierte Formel

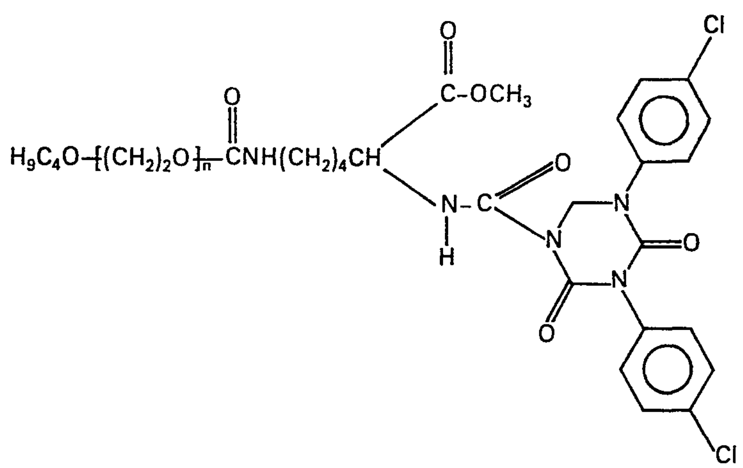

n = 43

Beispiel 10

12,0 g (0,01 mol) der nach Beispiel 2a erhaltenen Verbindung werden in 50 ml absolutem Toluol gelöst (Lösung 1). 3,4 g (0,01 mol) 1,3-Di-p-chlorphenyl-(1H, 2H, 3H, 4H, 5H, 6H)-S-triazin-2,4-dion werden in 50 ml absolutem Aceton gelöst (Lösung 2). Man legt Lösung 1 bei 40°C vor und tropft dann Lösung 2 sehr langsam zu, wobei schnell gerührt wird. Anschliessend wird bei 40°C und 200-300 mbar das Aceton abgezogen. Man belüftet mit trockenem Stickstoff. Anschliessend werden weitere 200 ml absolutes Toluol zugegeben und unter Feuchtigkeitsausschluss solange am Rückfluss gekocht, bis im IR-Spektrum die zur NCO-Gruppe gehörige Bande bei 2270 $cm^{-1}$ vernachlässigbar klein geworden ist (50 Min.). Anschliessend destilliert man das Toluol zuerst bei Normaldruck, danach im Wasserstrahlvakuum ab.

Als Rückstand erhält man 15,4 g eines bei Raumtemperatur wachsartigen Feststoffes. Dieser erfindungsgemäss modifizierte neue Wirkstoff ist gut in Wasser und/oder niederen Alkoholen löslich und besitzt die idealisierte Formel

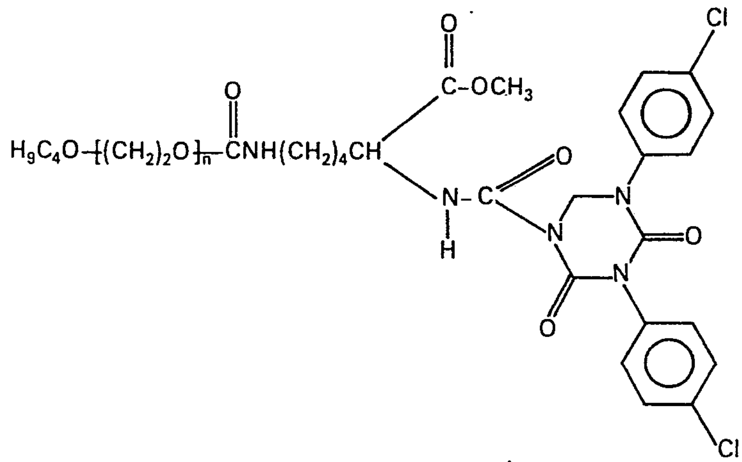

n = 21

Beispiel 11

9,9 g (0,01 mol) der nach Beispiel 3a erhaltenen Verbindung werden geschmolzen bei 50°C vorgelegt. 3,4 g (0,01 mol) 1,3-Di-p-chlorphenyl-(1H, 2H, 3H, 4H, 5H, 6H)-triazin-2,4-dion werden in 150 ml absolutem Aceton gelöst und anschliessend unter starkem Rühren zu der Vorlage zugetropft. Man zieht das Aceton bei 200–300 mbar ab und belüftet danach mit trockenem Stickstoff. Die Mischung wird solange bei 90°C gerührt, bis im IR-Spektrum die zur NCO-Gruppe gehörige Bande bei 2270 $cm^{-1}$ vernachlässigbar klein geworden ist (50 Min.). Man erhält 13,3 g einer goldgelb gefärbten Flüssigkeit. Dieser erfindungsgemäss modifizierte neue Wirkstoff ist gut in Wasser, und/oder niederen Alkoholen löslich und besitzt die idealisierte Formel

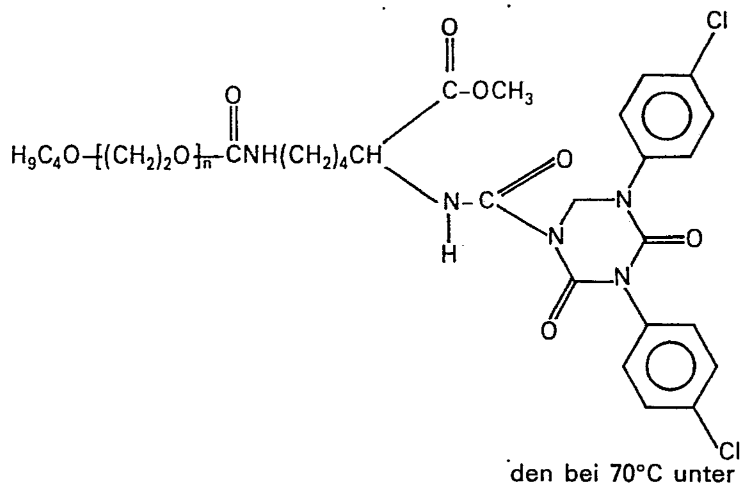

n = 16

Beispiel 12

7,3 g (0,01 mol) der nach Beispiel 4 erhaltenen Verbindung werden bei 40°C vorgelegt. Dazu tropft man unter starkem Rühren 3,4 g (0,01 mol) 1,3-Di-p-chlorphenyl-(1H, 2H, 3H, 4H, 5H, 6H)-triazin-2,4-dion gelöst in 150 ml absolutem Aceton. Das Aceton wird dann bei 200–300 mbar wieder abgezogen und der Kolben mit trockenem Stickstoff belüftet. Die Mischung wird 2½ Stunden bei 70°C unter Feuchtigkeitsausschluss gerührt. Nach dieser Zeit ist die zur NCO-Gruppe gehörige Bande im IR-Spektrum bei 2270 $cm^{-1}$ vernachlässigbar klein geworden.

Man erhält 10,7 g einer goldgelb gefärbten Flüssigkeit. Dieser erfindungsgemäss modifizierte neue Wirkstoff ist gut in Wasser und/oder niederen Alkoholen löslich und besitzt die idealisierte Formel

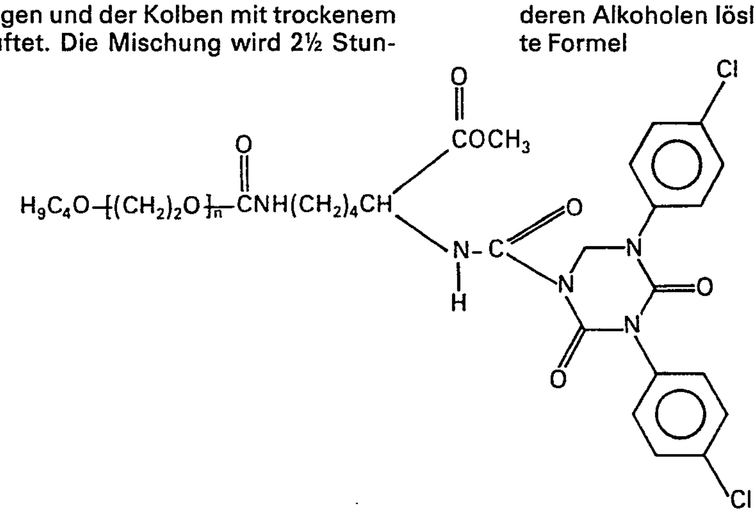

n = 10

Beispiel 13

7,5 g (0,02 mol) der nach Beispiel 5 erhaltenen Verbindung werden bei 50°C vorgelegt. Dazu tropft man unter starkem Rühren 6,7 g (0,02 mol) 1,3-Di-p-chlorphenyl-(1H, 2H, 3H, 4H, 5H, 6H)-triazin-2,4-dion gelöst in 150 ml absolutem Aceton. Man zieht dann das Aceton bei 200–300 mbar wieder ab und belüftet mit trockenem Stickstoff. Die Mischung wird 120 Min. bei 75°C unter Feuchtigkeitsausschluss gerührt; danach ist die zur NCO-Gruppe gehörige Bande im IR-Spektrum bei 2270 $cm^{-1}$ vernachlässigbar klein geworden.

Man erhält 14,2 g einer gelb gefärbten viskosen Flüssigkeit. Dieser erfindungsgemäss modifizierte neue Wirkstoff ist gut in Wasser und/oder niederen Alkoholen löslich und besitzt die idealisierte Formel

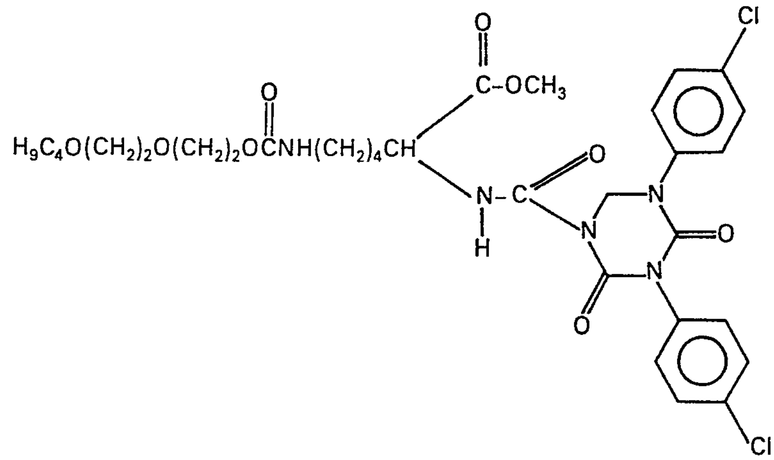

Beispiel 14

6,9 g (0,02 mol) der nach Beispiel 6 erhaltenen Verbindung werden analog zu Beispiel 13 mit 6,7 g (0,02 mol) 1,3-Di-p-chlorphenyl-(1H, 2H, 3H, 4H, 5H, 6H)-triazin-2,4-dion umgesetzt.

Nachrührzeit: 140 Min. bei 70°C.

Man erhält 13,6 g einer gelb gefärbten viskosen Flüssigkeit. Dieser erfindungsgemäss modifizierte neue Wirkstoff ist in Wasser mässig gut; jedoch in niederen Alkoholen löslich und besitzt die idealisierte Formel

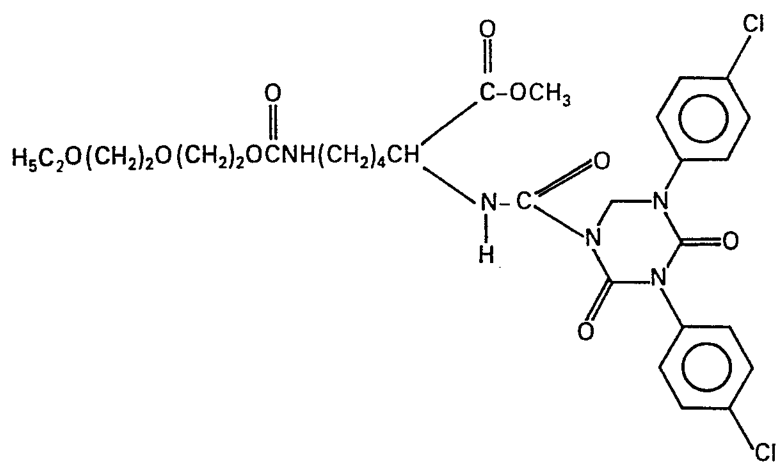

Beispiel 15

6,6 g (0,02 mol) der nach Beispiel 7 erhaltenen Verbindung werden analog zu Beispiel 13 mit 6,7 g (0,02 mol) 1,3-Di-p-chlorphenyl-(1H, 2H, 3H, 4H, 5H, 6H)-triazin-2,4-dion umgesetzt.

Nachrührzeit: 110 Min. bei 80°C.

Man erhält 13,3 g einer dunkelgelben viskosen Flüssigkeit. Dieser erfindungsgemäss modifizierte neue Wirkstoff ist in Wasser mässig gut; jedoch in niederen Alkoholen löslich und besitzt die idealisierte Formel

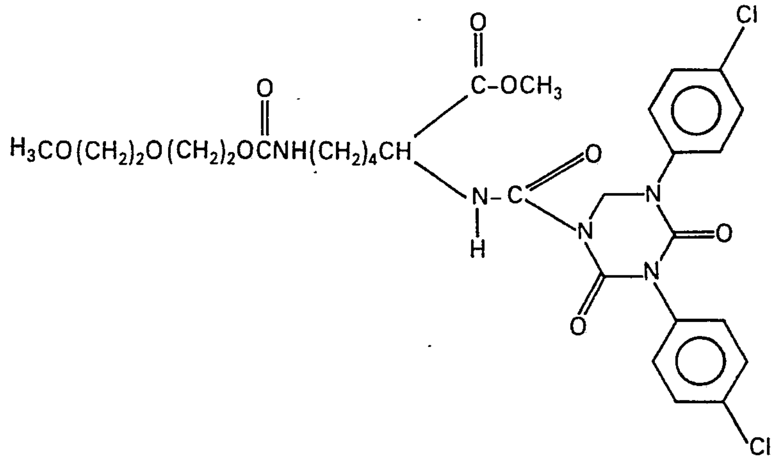

Beispiel 16

7,2 g (0,02 mol) der nach Beispiel 8 erhaltenen Verbindung werden analog zu Beispiel 13 mit 6,7 g (0,02 mol) 1,3-Di-p-chlorphenyl-(1H, 2H, 3H, 4H, 5H, 6H)-triazin-2,4-dion umgesetzt.

Nachrührzeit: 120 Min. bei 75°C.

Man erhält 13,9 g einer gelbroten viskosen Flüssigkeit. Dieser erfindungsgemäss modifizierte neue Wirkstoff ist in Wasser wie auch Methanol gut löslich und besitzt die idealisierte Formel

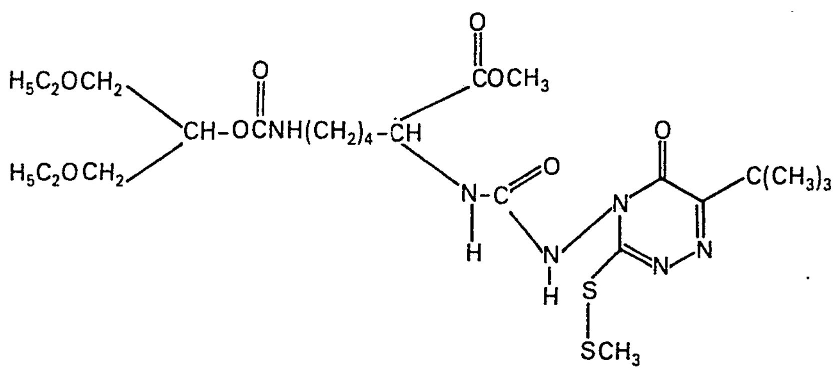

Beispiel 17

100 g (0,045 mol) der nach Beispiel 1a erhaltenen Verbindung werden analog zu Beispiel 9 mit 12,0 g (0,045 mol) 1,3-Diphenyl-(1H, 2H, 3H, 4H, 5H, 6H)-triazin-2,4-dion umgesetzt.

Nachrührzeit: 110 Min. bei 110°C.

Man erhält 112 g eines wachsartigen Feststoffes. Dieser erfindungsgemäss modifizierte neue Wirkstoff ist sehr gut in Wasser und/oder niederen Alkoholen löslich und besitzt die idealisierte Formel

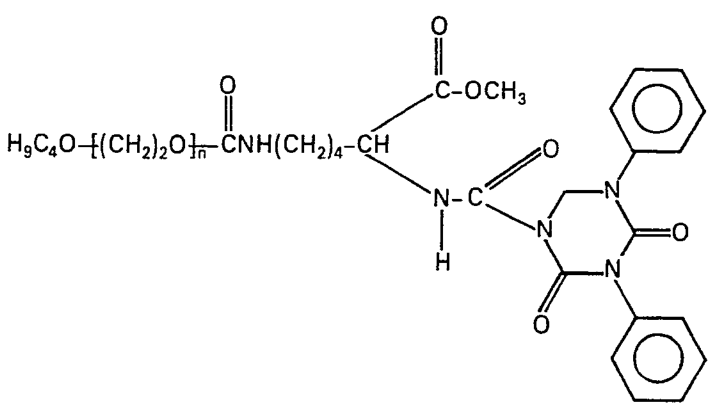

n = 43

Beispiel 18

12,0 g (0,01 mol) der nach Beispiel 2a erhaltenen Verbindung werden analog zu Beispiel 10 mit 2,7 g (0,01 mol) 1,3-Diphenyl-(1H, 2H, 3H, 4H, 5H, 6H)-triazin-2,4-dion umgesetzt.

Nachrührzeit: 110 Min. bei 110°C.

Man erhält 14,7 g eines wachsartigen Feststoffes. Dieser erfindungsgemäss modifizierte neue Wirkstoff ist sehr gut in Wasser und/oder niederen Alkoholen löslich und besitzt die idealisierte Formel

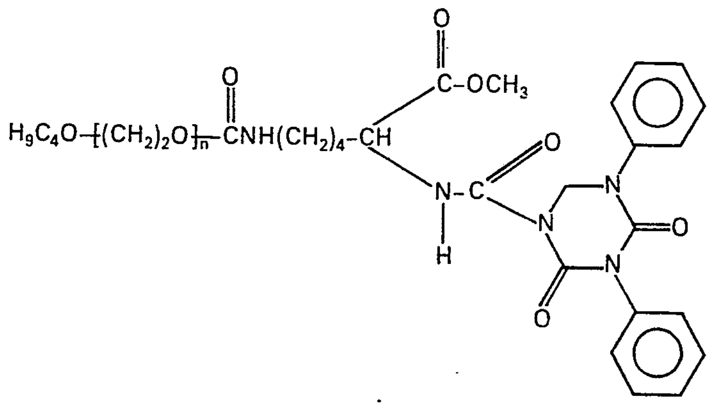

n = 21

Beispiel 19

9,9 g (0,01 mol) der nach Beispiel 3a erhaltenen Verbindung werden analog zu Beispiel 11 mit 2,7 g (0,01 mol) 1,3-Diphenyl-(1H, 2H, 3H, 4H, 5H, 6H)-triazin-2,4-dion umgesetzt.

Nachrührzeit: 100 Min. bei 90°C.

Man erhält 12,6 g eines wachsartigen Feststoffes. Dieser erfindungsgemäss modifizierte neue Wirkstoff ist sehr gut in Wasser und/oder niederen Alkoholen löslich und besitzt die idealisierte Formel

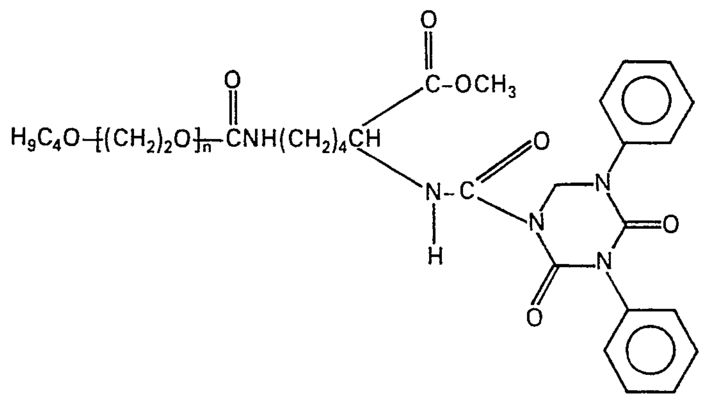

n = 16

Beispiel 20

7,3 g (0,01 mol) der nach Beispiel 4 erhaltenen Verbindung werden analog zu Beispiel 12 mit 2,7 g (0,01 mol) 1,3-Diphenyl-(1H, 2H, 3H, 4H, 5H, 6H)-triazin-2,4-dion umgesetzt.

Nachrührzeit: 120 Min. bei 70°C.

Man erhält 10,0 g einer goldgelben viskosen Flüssigkeit. Dieser erfindungsgemäss modifizierte neue Wirkstoff ist sehr gut in Wasser und/oder niederen Alkoholen löslich und besitzt die idealisierte Formel

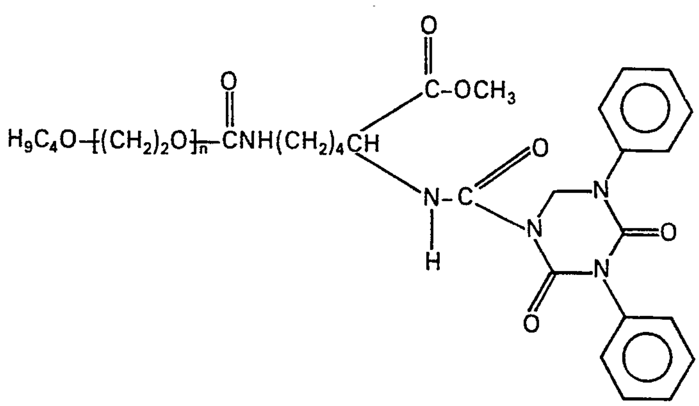

n = 10

Beispiel 21

110,6 g (0,05 mol) der nach Beispiel 1a erhaltenen Verbindung werden analog zu Beispiel 9 mit 7,2 g (0,05 mol) 1,3-Dimethyl-(1H, 2H, 3H, 4H, 5H, 6H)-triazin-2,6-dion umgesetzt.

Nachrührzeit: 140 Min. bei 110°C.

Man erhält 117 g eines weisslichgelben, wachsartigen Feststoffes. Dieser erfindungsgemäss modifizierte neue Wirkstoff ist sehr gut in Wasser und/oder niederen Alkoholen löslich und besitzt die idealisierte Formel

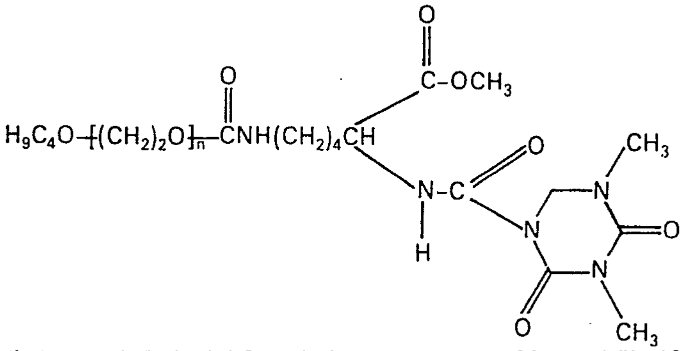

n = 43

Beispiel 22

12,0 g (0,01 mol) der nach Beispiel 2a erhaltenen Verbindung werden analog zu Beispiel 10 mit 1,43 g (0,01 mol) 1,3-Dimethyl-(1H, 2H, 3H, 4H, 5H, 6H)-triazin-2,4-dion umgesetzt.

Nachrührzeit: 110 Min. bei 110°C.

Man erhält 13,4 g einer gelben wachsartigen Substanz. Dieser erfindungsgemäss modifizierte neue Wirkstoff ist sehr gut in Wasser und/oder niederen Alkoholen löslich und besitzt die idealisierte Formel

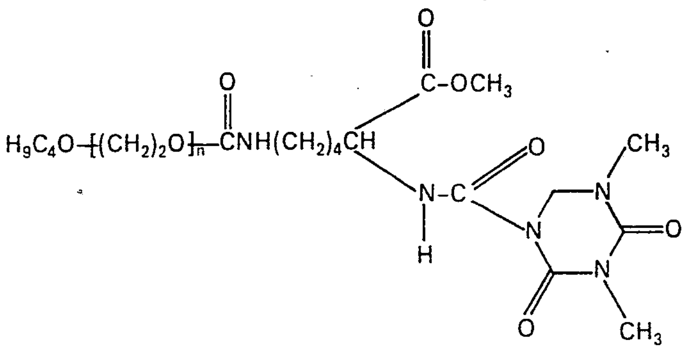

n = 21

Beispiel 23

9,9 g (0,01 mol) der nach Beispiel 3a erhaltenen Verbindung werden analog zu Beispiel 11 mit 1,43 g (0,01 mol) 1,3-Dimethyl-(1H, 2H, 3H, 4H, 5H, 6H)-triazin-2,4-dion umgesetzt.

Nachrührzeit: 60 Min. bei 90°C.

Man erhält 11,3 geiner gelben viskosen Flüssigkeit. Dieser erfindungsgemäss modifizierte neue Wirkstoff ist sehr gut in Wasser und/oder niederen Alkoholen löslich und besitzt die idealisierte Formel

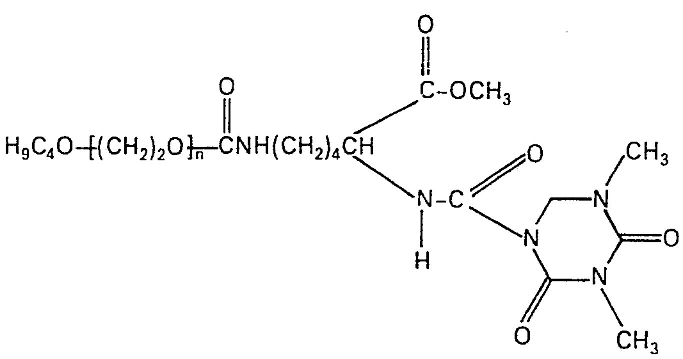

n = 16

Beispiel 24

7,3 g (0,01 mol) der nach Beispiel 4 erhaltenen Verbindung werden analog zu Beispiel 12 mit 1,43 g (0,01 mol) 1,3-Dimethyl-(1H, 2H, 3H, 4H, 5H, 6H)-triazin-2,4-dion umgesetzt.

Nachrührzeit: 70 Min. bei 70°C.

Man erhält 8,7 g einer goldgelben viskosen Flüssigkeit. Dieser erfindungsgemäss modifizierte neue Wirkstoff ist sehr gut in Wasser und/oder niederen Alkoholen löslich und besitzt die idealisierte Formel

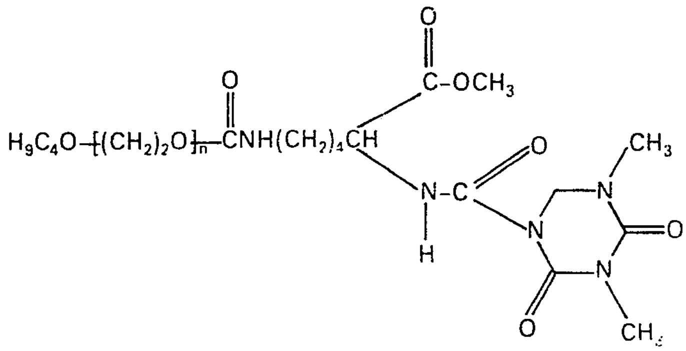

n = 10

Beispiel 25

100 g (0,045 mol) der nach Beispiel 1a erhaltenen Verbindung werden analog zu Beispiel 9 mit 9,6 g (0,045 mol) 4-Amino-6-tert.-butyl-3-(methylthio)-1,2,4-triazin-5-(4H)-on umgesetzt.

Nachrührzeit: 180 Min. bei 110°C.

Man erhält 109, g eines gelben Feststoffes. Dieser erfindungsgemäss modifizierte neue Wirkstoff ist sehr gut in Wasser und/oder niederen Alkoholen löslich und besitzt die idealisierte Formel

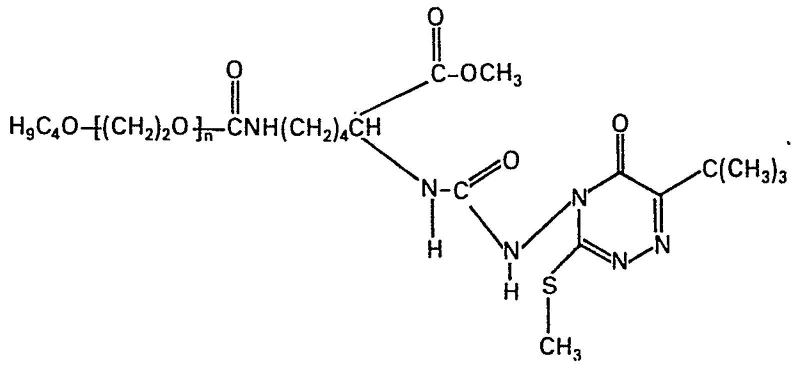

n = 43

Beispiel 26

60,6 g (0,05 mol) der nach Beispiel 2a erhaltenen Verbindung werden analog zu Beispiel 10 mit 10,7 g (0,05 mol) 4-Amino-6-tert.-butyl-3-(methylthio)-1,2,4-triazin-5-(4H)-on umgesetzt. Nachrührzeit: 105 Min. bei 110°C.

Man erhält 71,3 g eines gelben, zerfliesslichen Produktes. Dieser erfindungsgemäss modifizierte neue Wirkstoff ist sehr gut in Wasser und/oder niederen Alkoholen löslich und besitzt die idealisierte Formel

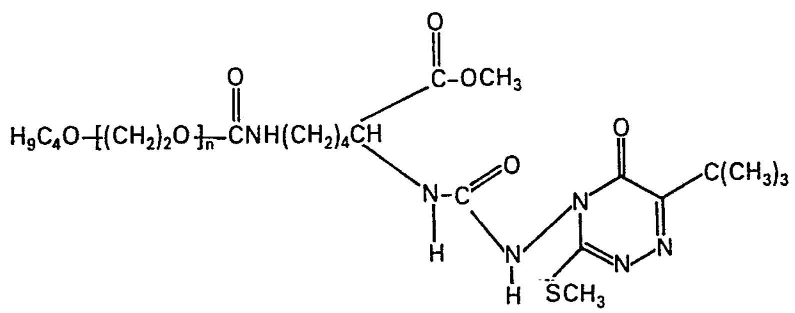

n = 21

Beispiel 27

49,6 g (0,05 mol) der nach Beispiel 3a erhaltenen Verbindung werden analog zu Beispiel 11 mit 10,7 g (0,05 mol) 4-Amino-6-tert.-butyl-3-(methylthio)-1,2,4-triazin-5-(4H)-on umgesetzt.

Nachrührzeit: 115 Min. bei 90°C.

Man erhält 60,3 g einer dunkelgelben hochviskosen Flüssigkeit. Dieser erfindungsgemäss modifizierte neue Wirkstoff ist gut in Wasser und/oder niederen Alkoholen löslich und besitzt die idealisierte Formel

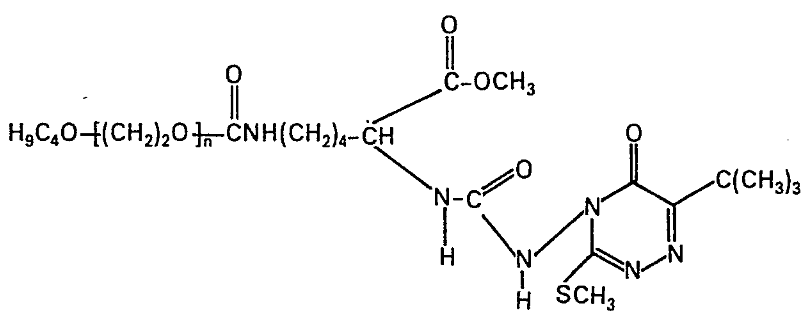

n = 16

Beispiel 28

36,6 g (0,05 mol) der nach Beispiel 4 erhaltenen Verbindung werden analog zu Beispiel 12 mit 10,7 g (0,05 mol) 4-Amino-6-tert.-butyl-3-(methylthio)-1,2,4-triazin-5-(4H)-on umgesetzt.

Nachrührzeit: 140 Min. bei 70°C.

Man erhält 47,3 g einer rotgelben viskosen Flüssigkeit. Dieser erfindungsgemäss modifizierte neue Wirkstoff ist gut in Wasser und/oder niederen Alkoholen löslich und besitzt die idealisierte Formel

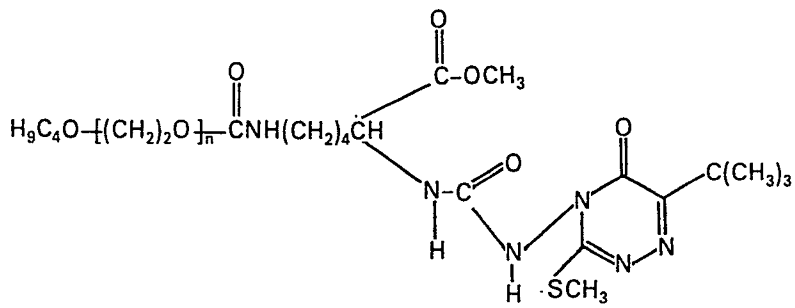

n = 10

Beispiel 29

18,7 g (0,05 mol) der nach Beispiel 5 erhaltenen Verbindung werden analog zu Beispiel 13 mit 10,7 g (0,05 mol) 4-Amino-6-tert.-butyl-3-(methylthio)-1,2,4-triazin-5-(4H)-on umgesetzt.

Nachrührzeit: 12 Stunden bei 75°C.

Man erhält 29,2 g einer dunkelgelben, hochviskosen Flüssigkeit. Dieser erfindungsgemäss modifizierte neue Wirkstoff ist gut in Wasser und/oder den niederen Alkoholen löslich und besitzt die idealisierte Formel

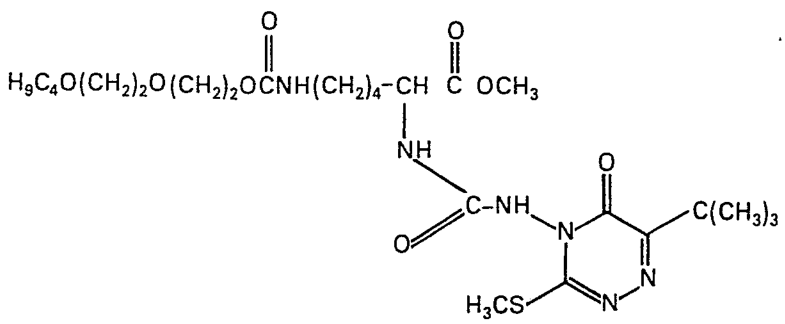

Beispiel 30

17,3 g (0,05 mol) der nach Beispiel 6 erhaltenen Verbindung werden analog zu Beispiel 13 mit 10,7 g (0,05 mol) 4-Amino-6-tert.-butyl-3-(methylthio)-1,2,4-triazin-5-(4H)-on umgesetzt.

Nachrührzeit: 16 Stunden bei 75°C.

Man erhält 27,4 g einer blassgelben, viskosen Flüssigkeit. Dieser erfindungsgemäss modifizierte neue Wirkstoff ist gut in Wasser und Methanol löslich und besitzt die idealisierte Formel

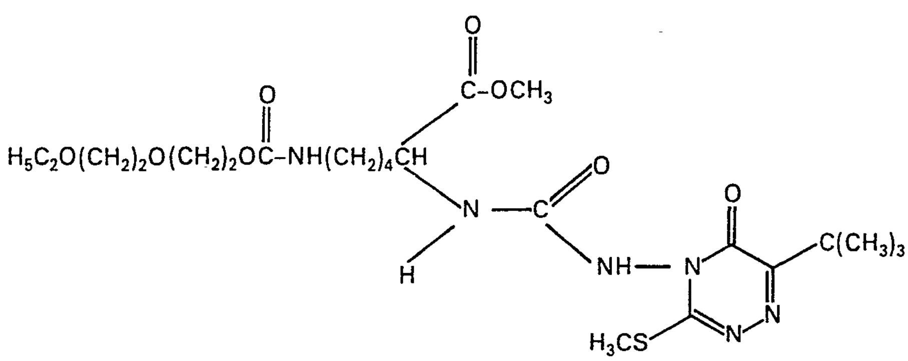

Beispiel 31

16,6 g (0,05 mol) der nach Beispiel 7 erhaltenen Verbindung werden analog zu Beispiel 13 mit 10,7 g (0,05 mol) 4-Amino-6-tert.-butyl-3-(methylthio)-1,2,4-triazin-5-(4H)-on umgesetzt.

Nachrührzeit: 21 Stunden bei 75°C.

Man erhält 26 g einer gelben, viskosen Flüssigkeit. Dieser erfindungsgemäss modifizierte neue Wirkstoff ist gut in Wasser und/oder Methanol löslich und besitzt die idealisierte Formel

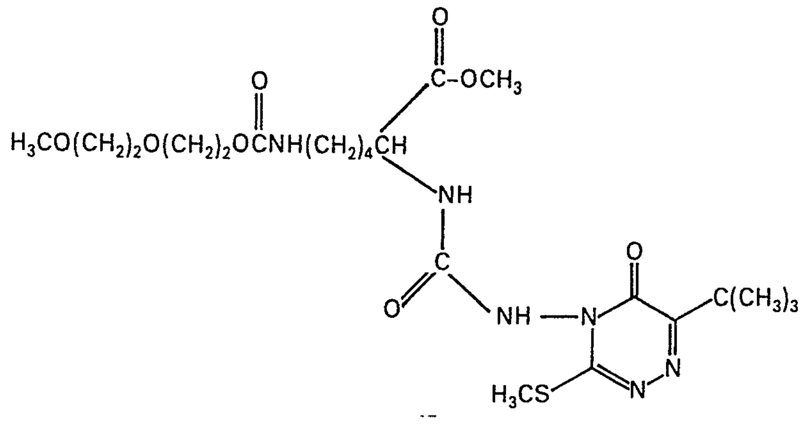

Beispiel 32

18,0 g (0,05 mol) der nach Beispiel 8 erhaltenen Verbindung werden analog zu Beispiel 13 mit 10,7 g (0,05 mol) 4-Amino-6-tert.-butyl-3-(methylthio)-1,2,4-triazin-5-(4H)-on umgesetzt.

Nachrührzeit: 3 Stunden bei 80°C.

Man erhält 27,4 g einer goldgelben, viskosen Flüssigkeit. Dieser erfindungsgemäss modifizierte neue Wirkstoff ist gut in Wasser und/oder Methanol bzw. Äthanol löslich und besitzt die idealisierte Formel

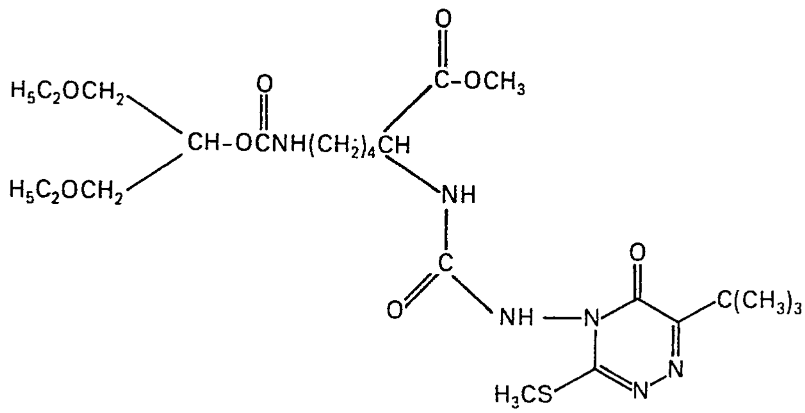

Beispiel 33

22,1 g (0,01 mol) der nach Beispiel 1a erhaltenen Verbindung werden analog zu Beispiel 9 mit 3,5 g (0,01 mol) N,N'-bis[3,4-dichlorphenyl]-harnstoff umgesetzt.

Nachrührzeit: 22 Stunden bei 110°C.

Man erhält 24,9 g eines blassgelben Feststoffes. Dieser erfindungsgemäss modifizierte neue Wirkstoff ist gut in Wasser und niederen Alkoholen löslich und besitzt die idealisierte Formel

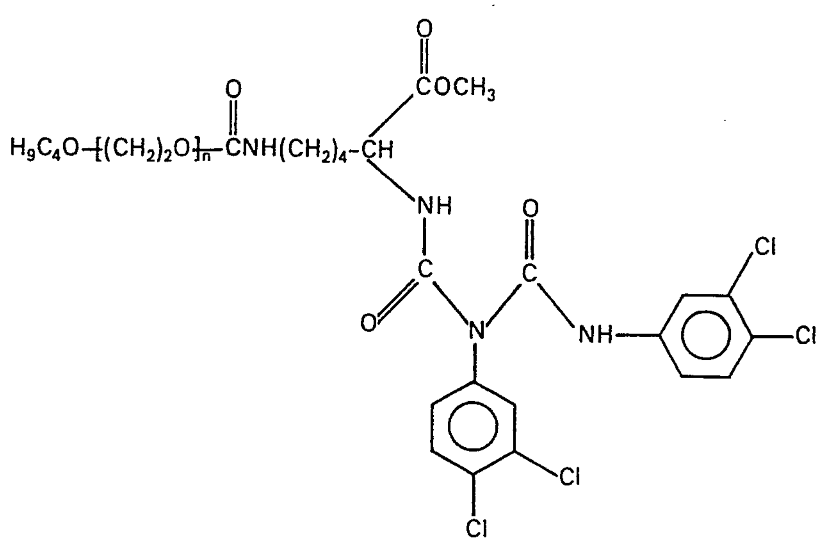

n = 43

Beispiel 34

12,1 g (0,01 mol) der nach Beispiel 2a erhaltenen Verbindung werden analog zu Beispiel 10 mit 3,5 g (0,01 mol) N,N'-Bis[3,4-dichlorphenyl]-harnstoff umgesetzt.

Nachrührzeit: 95 Min. bei 110°C.

Man erhält 14 g eines fahlgelben Feststoffes. Dieser erfindungsgemäss modifizierte neue Wirkstoff ist gut in Wasser und Methanol löslich und besitzt die idealisierte Formel

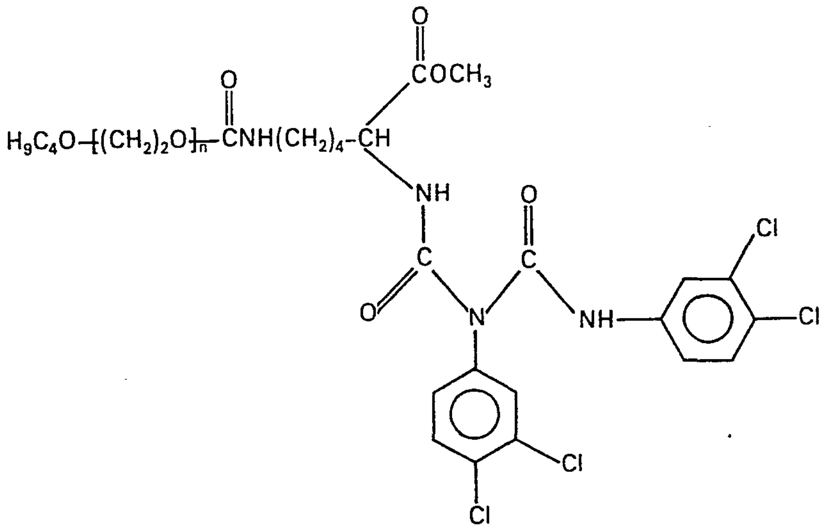

n = 21

Beispiel 35

9,9 g (0,01 mol) der nach Beispiel 3a erhaltenen Verbindung werden analog zu Beispiel 11 mit 3,5 g (0,01 mol) N,N′-Bis-(3,4-dichlorphenyl)-harnstoff umgesetzt.

Nachrührzeit: 5 Stunden bei 90°C.

Man erhält 12,1 g eines gelben, seifenartigen Feststoffes. Dieser erfindungsgemäss modifizierte neue Wirkstoff ist gut in Wasser und Methanol löslich und besitzt die idealisierte Formel

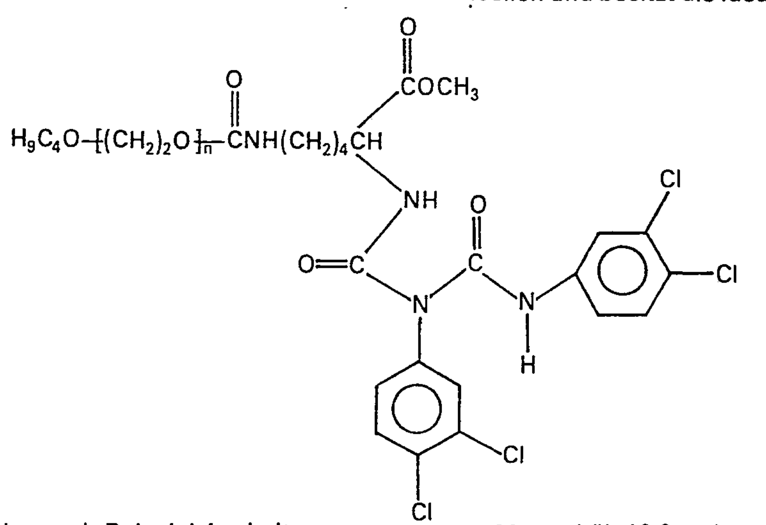

n = 16

Beispiel 36

7,3 g (0,01 mol) der nach Beispiel 4 erhaltenen Verbindung werden analog zu Beispiel 12 mit 3,5 g (0,01 mol) N,N′-Bis[3,4-dichlorphenyl]-harnstoff umgesetzt.

Nachrührzeit: 6 Stunden bei 70°C.

Man erhält 10,2 g einer dunkelgelben, viskosen Flüssigkeit. Dieser erfindungsgemäss modifizierte neue Wirkstoff ist gut in Wasser und/oder Methanol bzw. Äthanol löslich und besitzt die idealisierte Formel

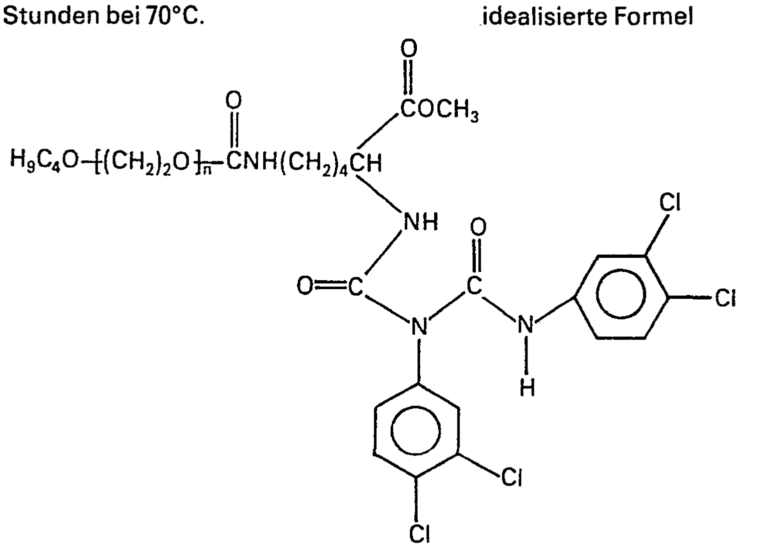

n = 10

Beispiel 37

7,3 g (0,01 mol) der nach Beispiel 4 erhaltenen Verbindung werden analog zu Beispiel 12 mit 3,2 g (0,01 mol) N-p-Chlorphenyl-N'-o,o'-Dichlorphenylharnstoff umgesetzt.

Nachrührzeit: 14 Stunden bei 70°C.

Man erhält 9,8 g einer hellbraunen, viskosen Flüssigkeit. Dieser erfindungsgemäss modifizierte neue Wirkstoff ist gut in Wasser und Methanol löslich und besitzt die idealisierte Formel

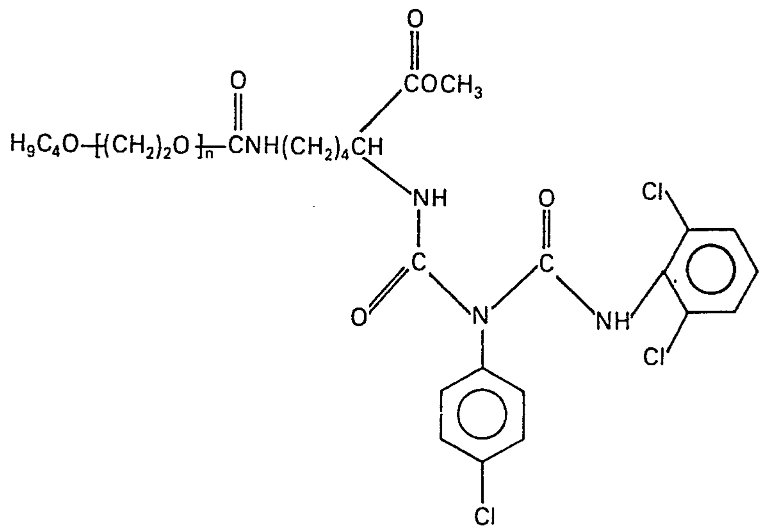

n = 10

Beispiel 38

7,3 g (0,01 mol) der nach Beispiel 4 erhaltenen Verbindung werden analog zu Beispiel 12 mit 1,5 g (0,01 mol) Phenylthioharnstoff umgesetzt.

Nachrührzeit: 2 Stunden bei 75°C.

Man erhält 7,8 g einer rötlich gelben, viskosen Flüssigkeit. Dieser erfindungsgemäss modifizierte neue Wirkstoff ist gut in Wasser und/oder niederen Alkoholen löslich und besitzt die idealisierte Formel

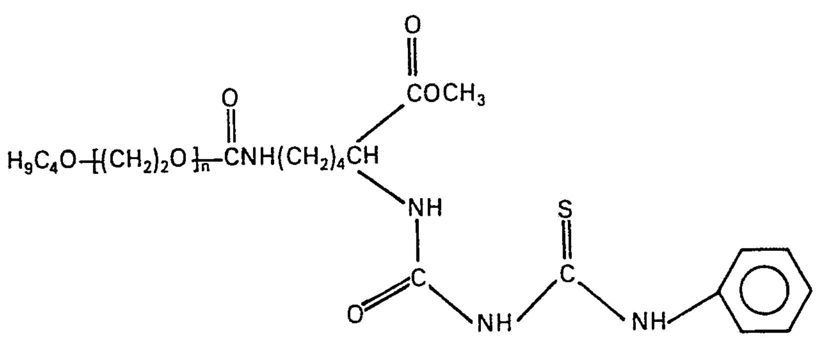

n = 10

Beispiel 39

22,1 g (0,01 mol) der nach Beispiel 1a erhaltenen Verbindung werden analog zu Beispiel 9 mit 2,0 g (0,01 mol) α-Naphthylthioharnstoff umgesetzt.

Nachrührzeit: 3 Stunden bei 110°C.

Man erhält 23,4 g eines gelbbraunen Feststoffes. Dieser erfindungsgemäss modifizierte neue Wirkstoff ist gut in Wasser und Methanol löslich und besitzt die idealisierte Formel

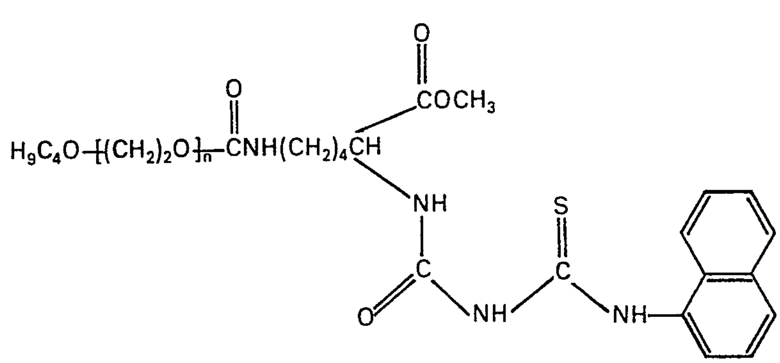

n = 43

Beispiel 40

22,1 g (0,01 mol) der nach Beispiel 1a erhaltenen Verbindung werden analog zu Beispiel 9 mit 2,9 g (0,01 mol) N,N-Dimethyl-N′-[p-(p-Chlorphenoxy)phenyl]-harnstoff umgesetzt.

Nachrührzeit: 6 Stunden bei 110°C.

Man erhält 23,8 g eines dunkelgelben Feststoffes. Dieser erfindungsgemäss modifizierte neue Wirkstoff ist gut in Wasser und Methanol löslich und besitzt die idealisierte Formel

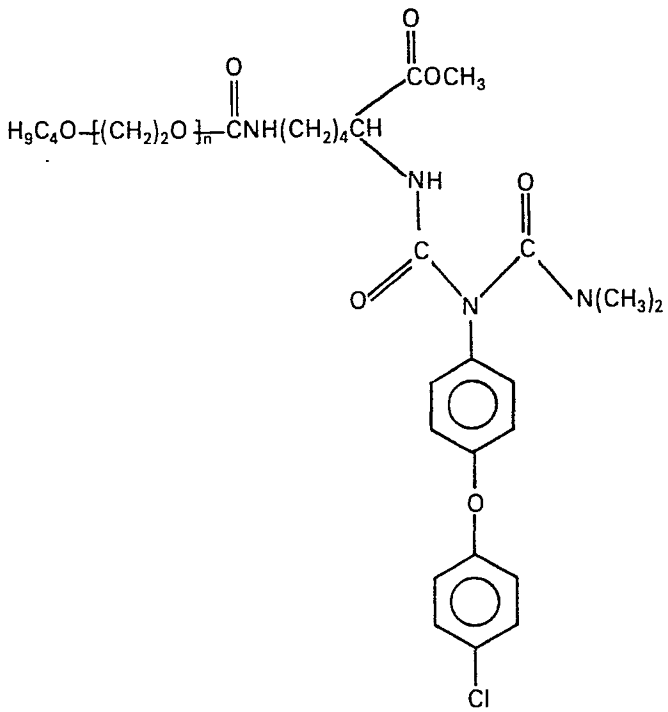

n = 43

Beispiel 41

22,1 g (0,01 mol) der nach Beispiel 1a erhaltenen Verbindung werden analog zu Beispiel 9 mit 2,3 g (0,01 mol) N-(3-Trifluormethylphenyl)-N′,N′-dimethylharnstoff umgesetzt.

Nachrührzeit: 8 Stunden bei 110°C.

Man erhält 23,9 g eines dunkelgelben Feststoffes. Dieser erfindungsgemäss modifizierte neue Wirkstoff ist gut in Wasser und/oder niederen Alkoholen löslich und besitzt die idealisierte Formel

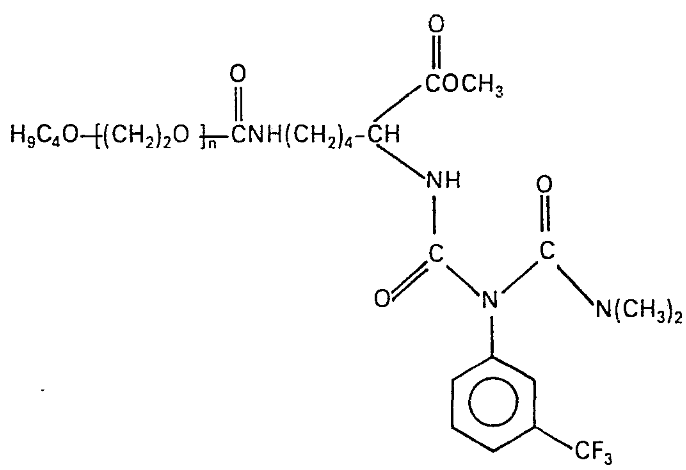

n = 43

Beispiel 42

22,1 g (0,01 mol) der nach Beispiel 1a erhaltenen Verbindung werden analog zu Beispiel 9 mit 2,1 g (0,01 mol) N-(4-Chlorphenyl)-N′-methoxy-N′-methylharnstoff umgesetzt.

Nachrührzeit: 5 Stunden bei 115°C.

Man erhält 23,7 g eines blassgelben Feststoffes. Dieser erfindungsgemäss modifizierte neue Wirkstoff ist gut in Wasser und/oder Methanol bzw. Äthanol löslich und besitzt die idealisierte Formel

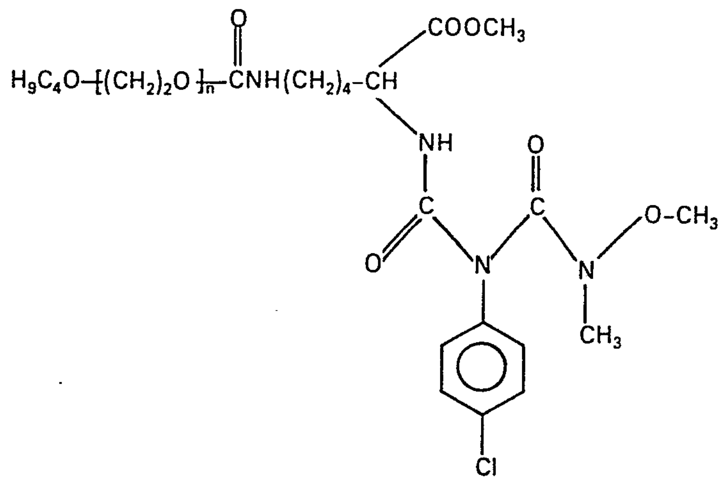

n = 43

Beispiel 43

22,1 g (0,01 mol) der nach Beispiel 1a erhaltenen Verbindung werden analog zu Beispiel 9 mit 2,1 g (0,01 mol) N-(2-Benzothiazolyl)-N'-methylharnstoff umgesetzt.

Nachrührzeit: 6 Stunden bei 110°C.

Man erhält 24,0 g eines goldgelben Feststoffes. Dieser erfindungsgemäss modifizierte neue Wirkstoff ist gut in Wasser und/oder Methanol löslich und besitzt die idealisierte Formel

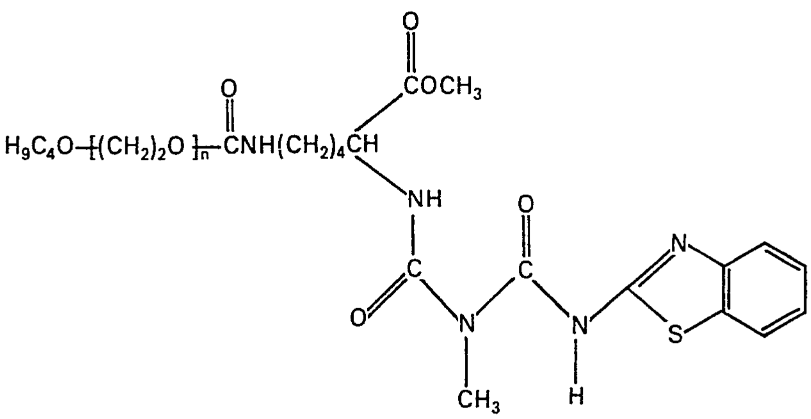

n = 43

Beispiel 44

14,6 g (0,02 mol) der nach Beispiel 4 erhaltenen Verbindung werden analog zu Beispiel 12 mit 2,8 g (0,02 mol) Phenylharnstoff umgesetzt.

Nachrührzeit: 14 Stunden bei 70°C.

Man erhält 15,2 g eines braungelben Feststoffes. Dieser erfindungsgemäss modifizierte neue Wirkstoff ist gut in Wasser und/oder niederen Alkoholen löslich und besitzt die idealisierte Formel

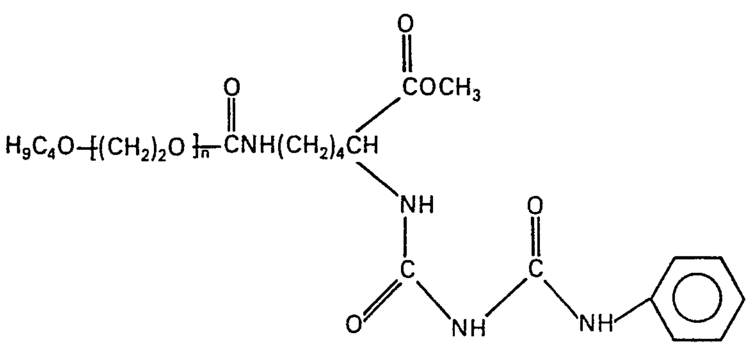

n = 10

Beispiel 45

12,1 g (0,01 mol) der nach Beispiel 2a erhaltenen Verbindung werden analog zu Beispiel 10 mit 2,9 g (0,01 mol) N-(4-Brom-3-chlorphenyl)-N'-methoxy-N'-methylharnstoff umgesetzt.

Nachrührzeit: 16 Stunden bei 95°C.

Man erhält 14,7 g eines dunkelbraunen Feststoffes. Dieser erfindungsgemäss modifizierte neue Wirkstoff ist gut in Wasser und niederen Alkoholen löslich und besitzt die idealisierte Formel

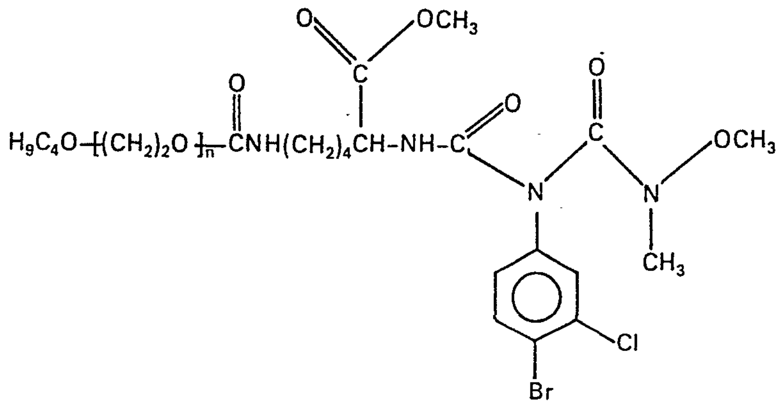

n = 21

Beispiel 46

12,1 g (0,01 mol) der nach Beispiel 2a erhaltenen Verbindung werden analog zu Beispiel 10 mit 1,9 g (0,01 mol) N-3-Tolyl-N',N'-dimethylharnstoff umgesetzt.

Nachrührzeit: 12 Stunden bei 105°C.

Man erhält 13,8 g eines gelben Feststoffes. Dieser erfindungsgemäss modifizierte neue Wirkstoff ist gut in Wasser und niederen Alkoholen löslich und besitzt die idealisierte Formel

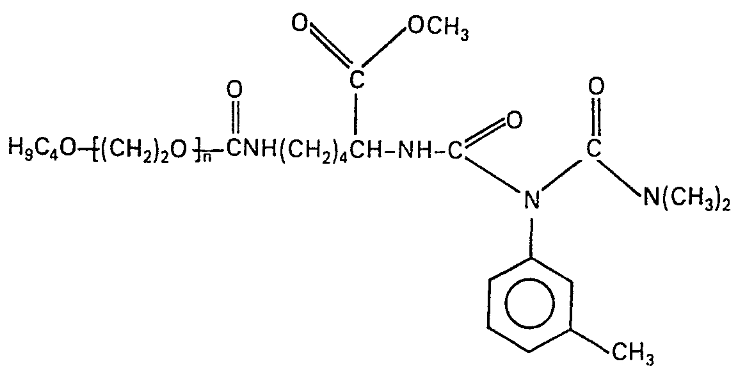

n = 21

Beispiel 47

22,1 g (0,01 mol) der nach Beispiel 1a erhaltenen Verbindung werden analog zu Beispiel 9 mit 2,2 g (0,01 mol) N-(2-Benzothiazolyl)-N-Methyl-N'-Methyl-harnstoff umgesetzt.

Nachrührzeit: 34 Stunden bei 80°C.

Man erhält 23,7 g eines goldgelben Feststoffes Dieser erfindungsgemäss modifizierte neue Wirkstoff ist gut in Wasser und/oder niederen Alkoholen löslich und besitzt die idealisierte Formel

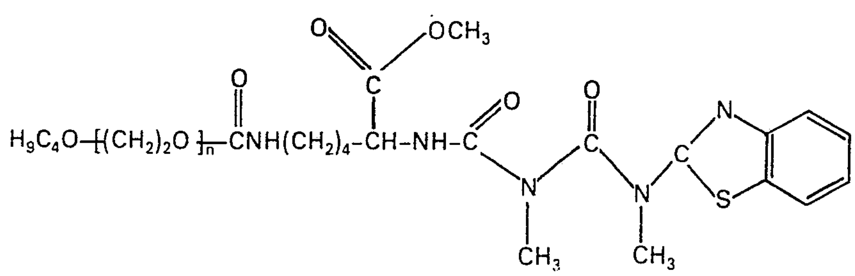

n = 43

Beispiel 48

12,1 g (0,01 mol) der nach Beispiel 2a erhaltenen Verbindung werden analog zu Beispiel 10 mit 2,2 g (0,01 mol) N-(2-Benzothiazolyl)-N,N'-Dimethylharnstoff umgesetzt.

Nachrührzeit: 38 Stunden bei 85°C.

Man erhält 14,0 g eines hellgelben Feststoffes. Dieser erfindungsgemäss modifizierte neue Wirkstoff ist gut in Wasser und niederen Alkoholen löslich und besitzt die idealisierte Formel

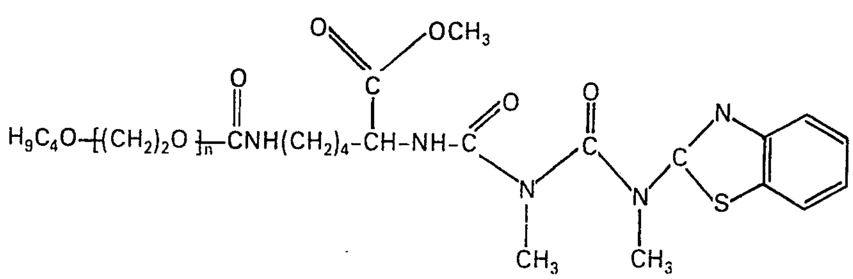

n = 21

Beispiel 49

9,9 g (0,01 mol) der nach Beispiel 3a erhaltenen Verbindung werden analog zu Beispiel 11 mit 2,2 g (0,01 mol) N-(2-Benzothiazolyl)-N,N'-Dimethylharnstoff umgesetzt.

Nachrührzeit: 32 Stunden bei 75°C.

Man erhält 11,7 g eines fahlgelben, hochviskosen Produktes. Dieser erfindungsgemäss modifizierte neue Wirkstoff ist gut in Wasser und niederen Alkoholen löslich und besitzt die idealisierte Formel

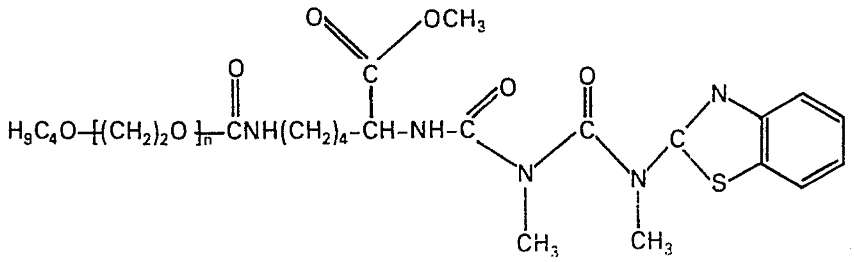

n = 16

Beispiel 50

22,1 g (0,01 mol) der nach Beispiel 1a erhaltenen Verbindung werden analog zu Beispiel 9 mit 2,0 g (0,01 mol) 3-Methyl-4-Amino-6-Phenyl-1,2,4-triazin-5(4H)-on umgesetzt.

Nachrührzeit: 8 Stunden bei 105°C.

Man erhält 23,7 g eines graugelben Feststoffes. Dieser erfindungsgemäss modifizierte neue Wirkstoff ist gut in Wasser und/oder niederen Alkoholen löslich und besitzt die idealisierte Formel

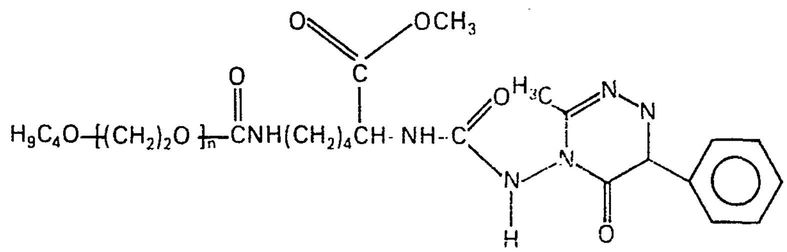

n = 43

Beispiel 51

9,9 g (0,01 mol) der nach Beispiel 3a erhaltenen Verbindung werden analog zu Beispiel 11 mit 2,0 g (0,01 mol) 3-Methyl-4-amino-6-phenyl-1,2,4-triazin-5(4H)-on umgesetzt.

Nachrührzeit: 12 Stunden bei 95°C.

Man erhält 11,6 g eines graugoldfarbigen, hochviskosen Produktes. Dieser erfindungsgemäss modifizierte neue Wirkstoff ist gut in Wasser und/oder niederen Alkoholen löslich und besitzt die idealisierte Formel

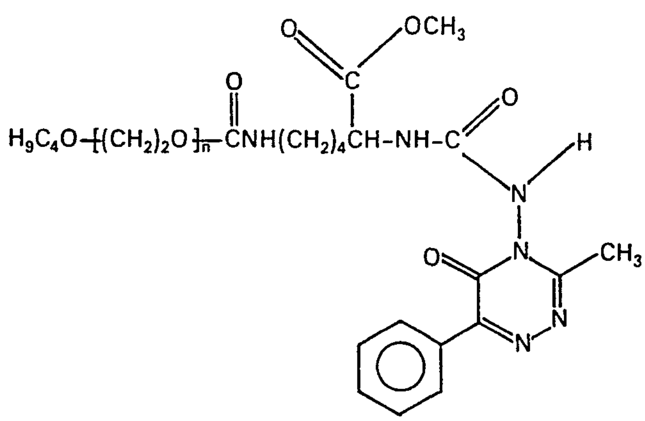

n = 16

Beispiel 52

22,1 g (0,01 mol) der nach Beispiel 1a erhaltenen Verbindung werden analog zu Beispiel 9 mit 2,6 g (0,01 mol) 1-Amino-3-(1-Methylpropyl)-5-Brom-6-methyl-1,3-diazin-(5H)-2,4-dion umgesetzt.

Nachrührzeit: 16 Stunden bei 100°C.

Man erhält 24,4 g eines dunkelgelben Feststoffes. Dieser erfindungsgemäss modifizierte neue Wirkstoff ist gut in Wasser und/oder niederen Alkoholen löslich und besitzt die idealisierte Formel

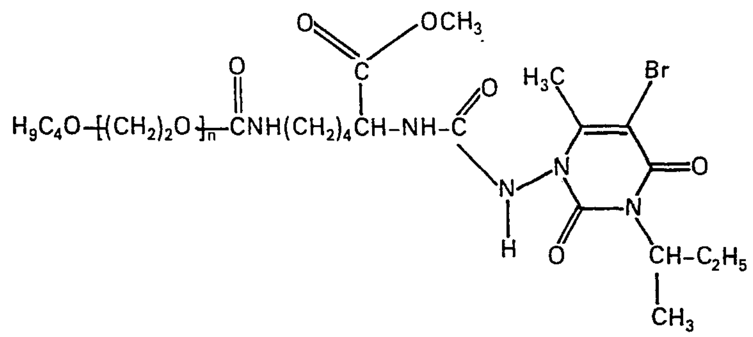

n = 43

Beispiel 53

9,9 g (0,01 mol) der nach Beispiel 3a erhaltenen Verbindung werden analog zu Beispiel 11 mit 2,6 g (0,01 mol) 1-Amino-3-(1-Methyl-propyl)-5-Brom-6-methyl-1,3-diazin-(5H)-2,4-dion umgesetzt.

Nachrührzeit: 14 Stunden bei 110°C.

Man erhält 12,1 g eines dunkelgelben, hochviskosen Produktes. Dieser erfindungsgemäss modifizierte neue Wirkstoff ist gut in Wasser und niederen Alkoholen löslich und besitzt die idealisierte Formel

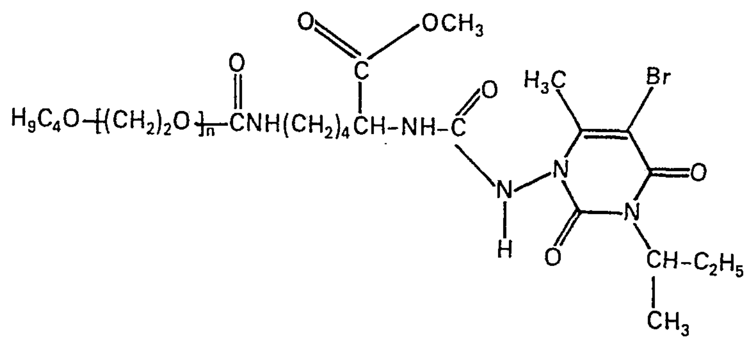

n = 16.

Beispiel 54

22,1 g (0,01 mol) der nach Beispiel 1a erhaltenen Verbindung werden analog zu Beispiel 9 mit 3,1 g (0,01 mol) N-o,o'-Difluorbenzoyl-N'-p-chlorphenylharnstoff umgesetzt.

Nachrührzeit: 6 Stunden bei 110°C.

Man erhält 24,6 g eines dunkelbraunen Feststoffes. Dieser erfindungsgemäss modifizierte neue Wirkstoff ist gut in Wasser und/oder niederen Alkoholen löslich und besitzt die idealisierte Formel

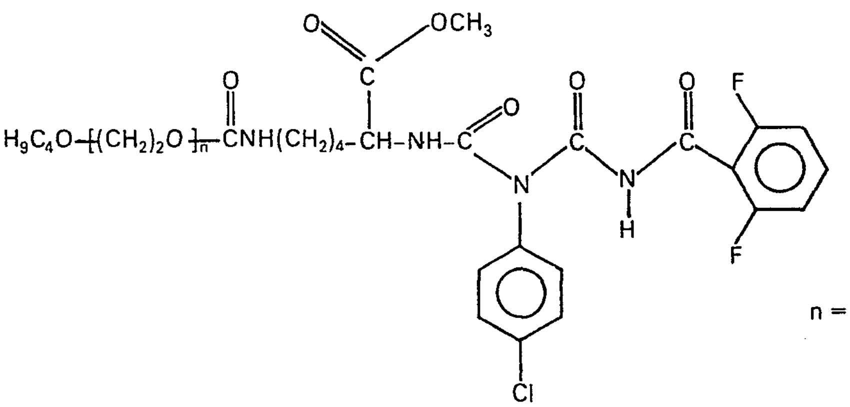

n = 43

Beispiel 55

22,1 g (0,01 mol) der nach Beispiel 1a erhaltenen Verbindung werden analog zu Beispiel 9 mit 3,7 g (0,01 mol) 3-(3,4-Dichlorphenyl)-6-(2,6-difluorphenyl)-1-oxa-3,5-diazin-(3H, 5H)-2,4-dion umgesetzt.

Nachrührzeit: 8 Stunden bei 100°C.

Man erhält 25,1 g eines grünlichgelben Feststoffes. Dieser erfindungsgemäss modifizierte neue Wirkstoff ist gut in Wasser und/oder niederen Alkoholen löslich und besitzt die idealisierte Formel

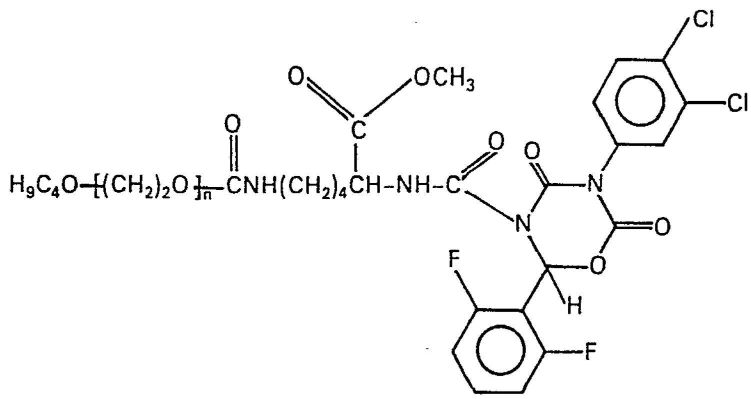

n = 43

Beispiel 56

12,1 g (0,01 mol) der nach Beispiel 2a erhaltenen Verbindung werden analog zu Beispiel 10 mit 3,7 g (0,01 mol) 3-(3,4-Dichlorphenyl)-6-(2,6-Difluorphenyl)-1-oxa-3,5-diazin-(3H, 4H)-2,4-dion umgesetzt.

Nachrührzeit: 10 Stunden bei 95°C.

Man erhält 15,2 g eines grünbraunen Feststoffes. Dieser erfindungsgemäss modifizierte neue Wirkstoff ist gut in Wasser und/oder niederen Alkoholen löslich und besitzt die idealisierte Formel

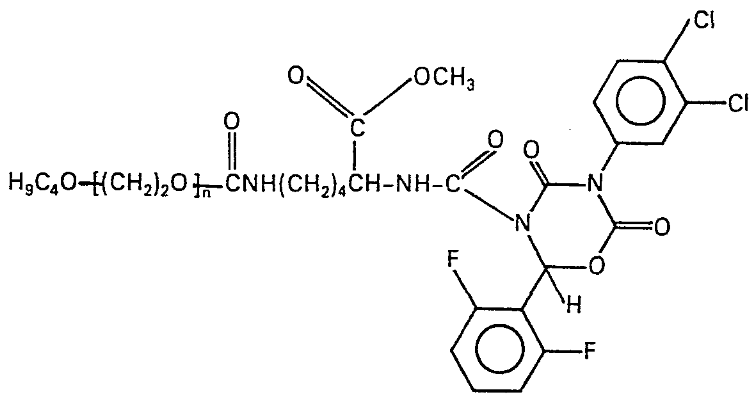

n = 21

Beispiel 57

22,1 g (0,01 mol) der nach Beispiel 1a erhaltenen Verbindung werden analog zu Beispiel 9 mit 3,4 g N-(2-Chlorbenzoyl)-N'-(4-Trifluormethoxyphenyl)-harnstoff umgesetzt.

Nachrührzeit: 10 Stunden bei 105°C.

Man erhält 25,1 g eines goldgelben Feststoffes. Dieser erfindungsgemäss modifizierte neue Wirkstoff ist gut in Wasser und/oder niederen Alkoholen löslich und besitzt die idealisierte Formel

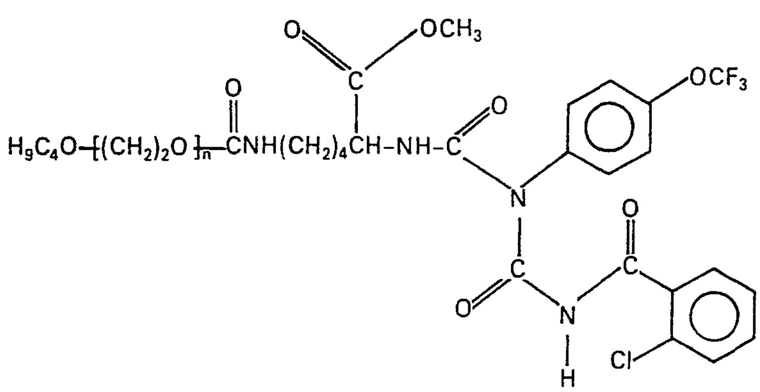

n = 43

Beispiel 58

22,1 g (0,01 mol) der nach Beispiel 1a erhaltenen Verbindung werden analog zu Beispiel 9 mit 2,1 g (0,01 mol) 4-Benzothienyl-N-methylcarbamat umgesetzt.

Nachrührzeit: 8 Stunden bei 90°C.

Man erhält 23,6 g eines gelben Feststoffes. Dieser erfindungsgemäss modifizierte neue Wirkstoff ist gut in Wasser und niederen Alkoholen löslich und besitzt die idealisierte Formel

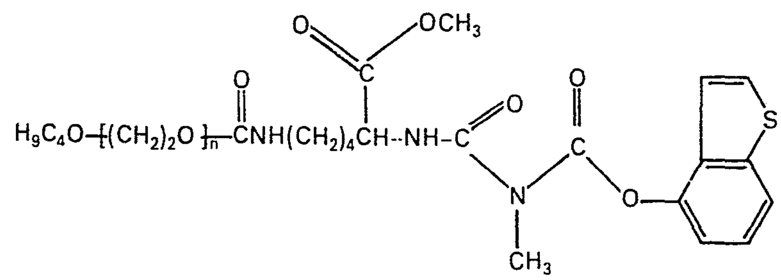

n = 43

Beispiel 59

12,1 g (0,01 mol) der nach Beispiel 2a erhaltenen Verbindung werden analog zu Beispiel 10 mit 2,0 g (0,01 mol) N-Methyl-1-naphthylcarbamat umgesetzt.

Nachrührzeit: 9 Stunden bei 90°C.

Man erhält 13,8 g eines goldgelben Feststoffes. Dieser erfindungsgemäss modifizierte neue Wirkstoff ist gut in Wasser und niederen Alkoholen löslich und besitzt die idealisierte Formel

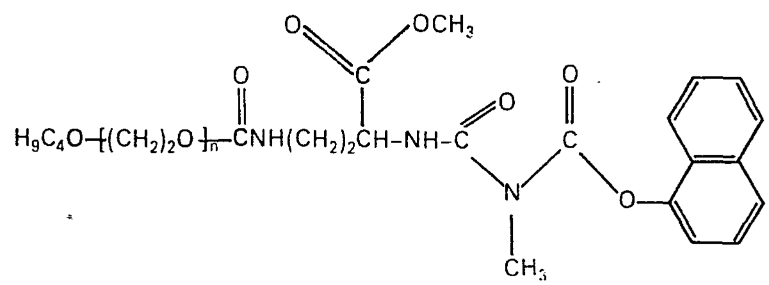

n = 21

Beispiel 60

9,9 g (0,01 mol) der nach Beispiel 3a erhaltenen Verbindung werden analog zu Beispiel 11 mit 1,8 g (0,01 mol) 3,4-Dimethylphenyl-N-methylcarbamat umgesetzt.

Nachrührzeit: 7 Stunden bei 95°C.

Man erhält 10,2 g eines blassgelben, hochviskosen Produktes. Dieser erfindungsgemäss modifizierte neue Wirkstoff ist gut in Wasser und niederen Alkoholen löslich und besitzt die idealisierte Formel

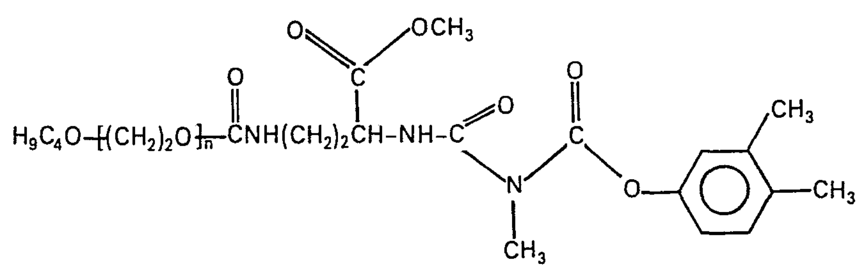

n = 16

Beispiel 61

12,2 g (0,01 mol) der nach Beispiel 1a erhaltenen Verbindung werden analog zu Beispiel 9 mit 3,1 g (0,01 mol) N-(2,6-Difluorbenzoyl)-p-chlorphenylcarbamat umgesetzt.

Nachrührzeit: 16 Stunden bei 110°C.

Man erhält 24,7 g eines gelblich braunen Feststoffes. Dieser erfindungsgemäss modifizierte neue Wirkstoff ist gut in Wasser und niederen Alkoholen löslich und besitzt die idealisierte Formel

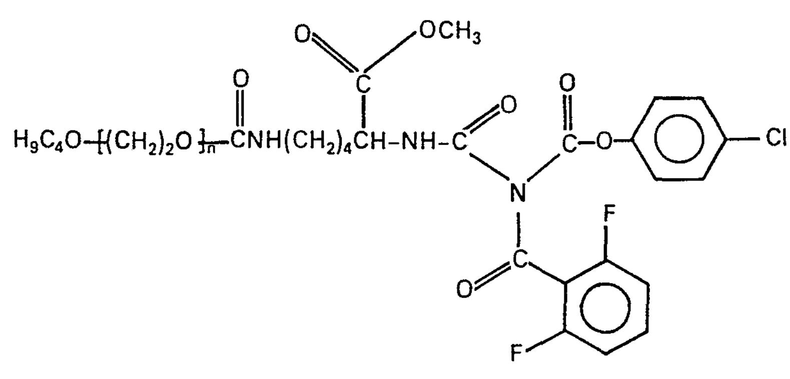

n = 43

Beispiel 62

9,9 g (0,01 mol) der nach Beispiel 3a erhaltenen Verbindung werden analog zu Beispiel 11 mit 3,5 g (0,01 mol) N-(2,6-Difluorbenzoyl)-o,o'-Dichlorphenylcarbamat umgesetzt.

Nachrührzeit: 20 Stunden bei 95°C.

Man erhält 11,5 g eines dunkelbraunen, hochviskosen Produktes. Dieser erfindungsgemäss modifizierte neue Wirkstoff ist gut in Wasser und niederen Alkoholen löslich und besitzt die idealisierte Formel

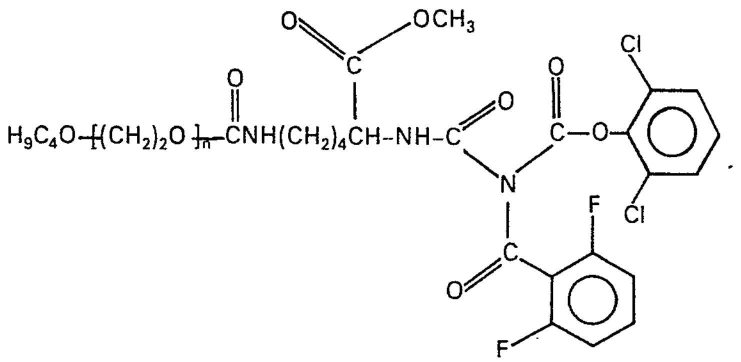

n = 16

Beispiel 63

22,1 g (0,01 mol) der nach Beispiel 1a erhaltenen Verbindung werden analog zu Beispiel 9 mit 3,2 g (0,01 mol) N-(2,6-Difluorbenzoyl)-7-Nitrophenylcarbamat umgesetzt.

Nachrührzeit: 12 Stunden bei 100°C.

Man erhält 25,1 g eines gelben Feststoffes. Dieser erfindungsgemäss modifizierte neue Wirkstoff ist gut in Wasser und niederen Alkoholen löslich und besitzt die idealisierte Formel

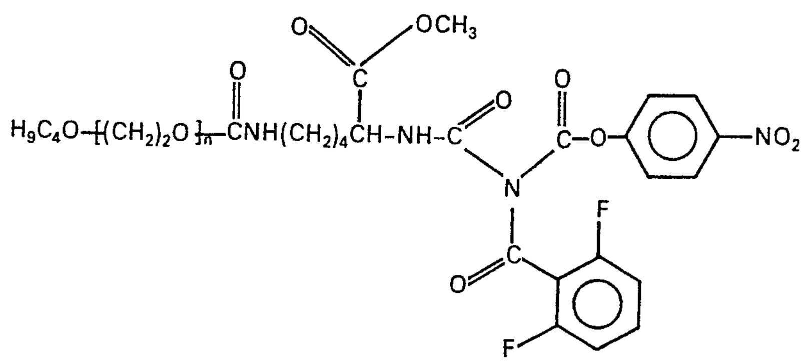

n = 43

Beispiel 64

9,9 g (0,01 mol) der nach Beispiel 3a erhaltenen Verbindung werden analog zu Beispiel 11 mit 3,6 g (0,01 mol) N-(2,6-Difluorbenzoyl)-2-chlor-4-nitrophenylcarbamat umgesetzt.

Nachrührzeit: 16 Stunden bei 80°C.

Man erhält 12,7 g eines dunkelbraunen, viskosen Produktes. Dieser erfindungsgemäss modifizierte neue Wirkstoff ist gut in Wasser und Methanol sowie Äthanol löslich und besitzt die idealisierte Formel

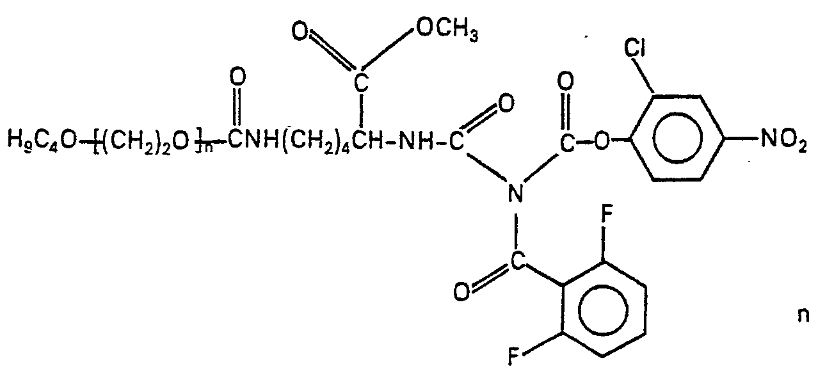

n = 16

Beispiel 65

22,1 g (0,01 mol) der nach Beispiel 1a erhaltenen Verbindung werden analog zu Beispiel 9 mit 3,3 g (0,01 mol) N-(2,6-Difluorbenzoyl)-p-chlorphenylthiocarbamat umgesetzt.

Nachrührzeit: 8 Stunden bei 90°C.

Man erhält 25,3 g eines hellbraunen Feststoffes. Dieser erfindungsgemäss modifizierte neue Wirkstoff ist gut in Wasser und/oder niederen Alkoholen löslich und besitzt die idealisierte Formel

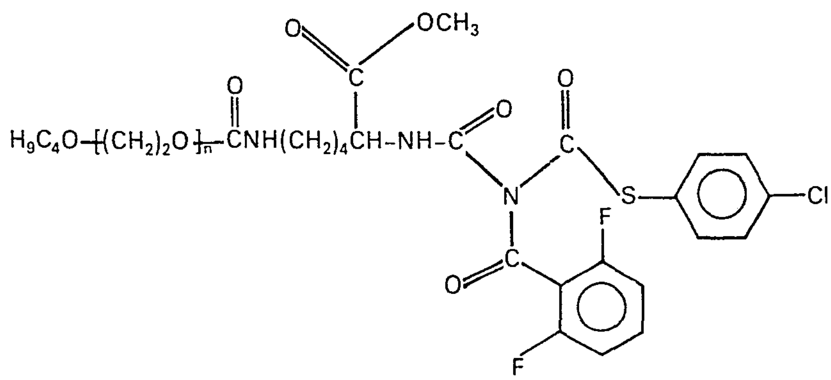

n = 43

Beispiel 66

22,1 g (0,01 mol) der nach Beispiel 1a erhaltenen Verbindung werden analog zu Beispiel 9 mit 2,1 g (0,01 mol) 2-Isopropyloxyphenyl-N-methylcarbamat umgesetzt.

Nachrührzeit: 6 Stunden bei 110°C.

Man erhält 24,0 g eines goldgelben Feststoffes. Dieser erfindungsgemäss modifizierte neue Wirkstoff ist gut in Wasser und/oder niederen Alkoholen löslich und besitzt die idealisierte Formel

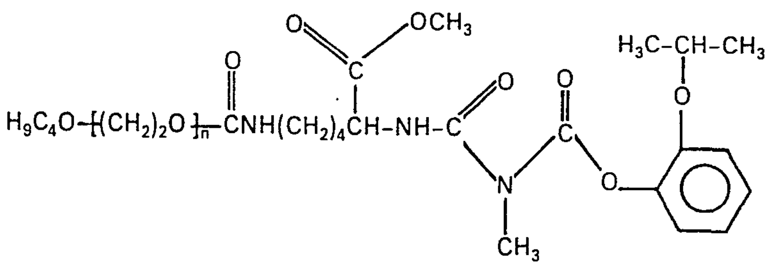

n = 43

Beispiel 67

12,1 g (0,01 mol) der nach Beispiel 2a erhaltenen Verbindung werden analog zu Beispiel 10 mit 2,1 g (0,01 mol) 2-Isopropyloxyphenyl-N-methylcarbamat umgesetzt.

Nachrührzeit: 7 Stunden bei 95°C.

Man erhält 13,7 g eines dunkelgelben Feststoffes von wachsartiger Konsistenz. Dieser erfindungsgemäss modifizierte neue Wirkstoff ist gut in Wasser und niederen Alkoholen löslich und besitzt die idealisierte Formel

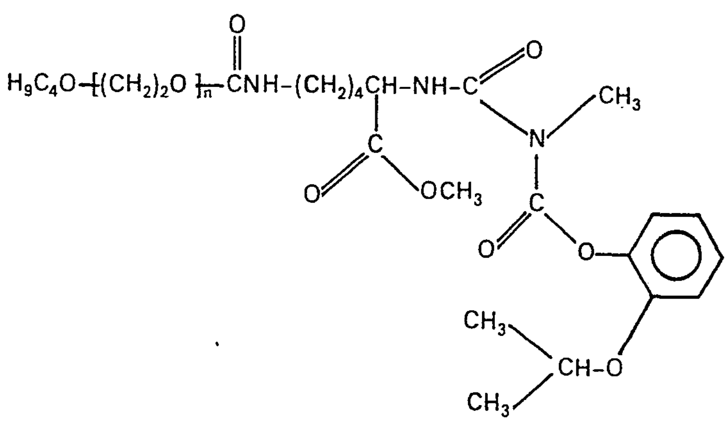

n = 21

Beispiel 68

9,9 g (0,01 mol) der nach Beispiel 3a erhaltenen Verbindung werden analog zu Beispiel 11 mit 2,1 g (0,01 mol) 2-Isopropyloxyphenyl-N-methylcarbamat umgesetzt.

Nachrührzeit: 8 Stunden bei 80°C.

Man erhält 11,6 g eines graugelben viskosen Produktes. Dieser erfindungsgemäss modifizierte neue Wirkstoff ist gut in Wasser und niederen Alkoholen löslich und besitzt die idealisierte Formel

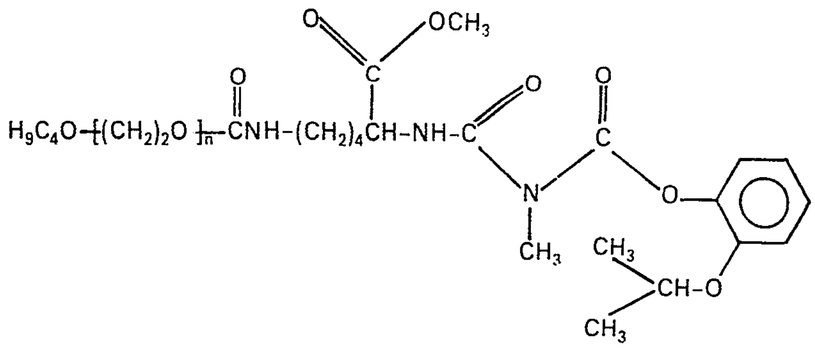

n = 16

Beispiel 69

22,1 g (0,01 mol) der nach Beispiel 1a erhaltenen Verbindung werden analog zu Beispiel 3 mit 2,2 g (0,01 mol) 2,3-Dihydro-2,2-dimethyl-7-benzofuranyl-N-methylcarbamat umgesetzt.

Nachrührzeit: 6 Stunden bei 110°C.

Man erhält 23,7 g eines fahlgelben Feststoffes. Dieser erfindungsgemäss modifizierte neue Wirkstoff ist gut in Wasser und/oder niederen Alkoholen löslich und besitzt die idealisierte Formel

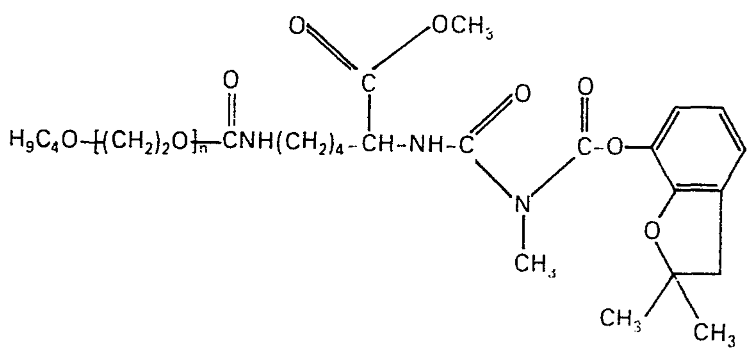

n = 43

Beispiel 70

12,1 g (0,01 mol) der nach Beispiel 2a erhaltenen Verbindung werden analog zu Beispiel 10 mit 2,2 g (0,01 mol) 2,3-Dihydro-2,2-dimethyl-7-benzofuranyl-N-methylcarbamat umgesetzt.

Nachrührzeit: 8 Stunden bei 90°C.

Man erhält 13,5 g eines gelben Feststoffes. Dieser erfindungsgemäss modifizierte neue Wirkstoff ist gut in Wasser und/oder niederen Alkoholen löslich und besitzt die idealisierte Formel

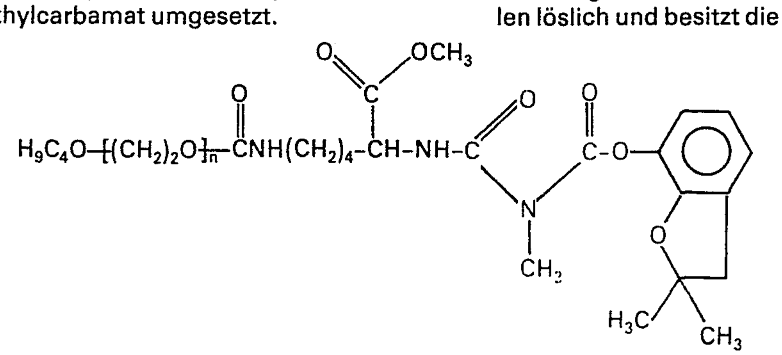

n = 21

Beispiel 71

12,1 g (0,01 mol) der nach Beispiel 2a erhaltenen Verbindung werden analog zu Beispiel 10 mit 2,25 g (0,01 mol) 3,5-Dimethyl-4-methylmercapto-phenyl-N-methylcarbamat umgesetzt.

Nachrührzeit: 11 Stunden bei 105°C.

Man erhält 13,9 g eines bräunlichen Feststoffes. Dieser erfindungsgemäss modifizierte neue Wirkstoff ist gut in Wasser und/oder niederen Alkoholen löslich und besitzt die idealisierte Formel

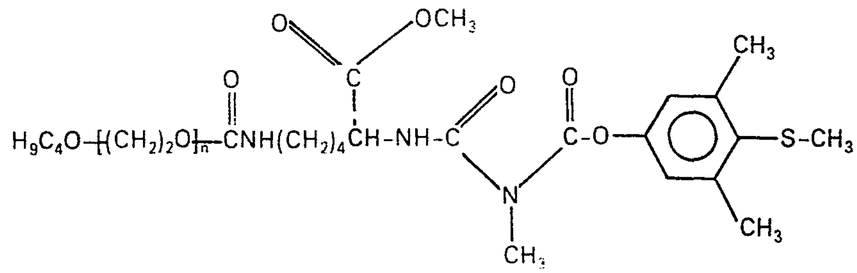

n = 21

Beispiel 72

9,9 g (0,01 mol) der nach Beispiel 3a erhaltenen Verbindung werden analog zu Beispiel 11 mit 2,25 g (0,01 mol) 3,5-Dimethyl-4-methylmercapto-phenyl-N-methylcarbamat umgesetzt.

Nachrührzeit: 16 Stunden bei 80°C.

Man erhält 11,7 g eines dunkelgelben, viskosen Produktes. Dieser erfindungsgemäss modifizierte neue Wirkstoff ist gut in Wasser und/oder niederen Alkoholen löslich und besitzt die idealisierte Formel

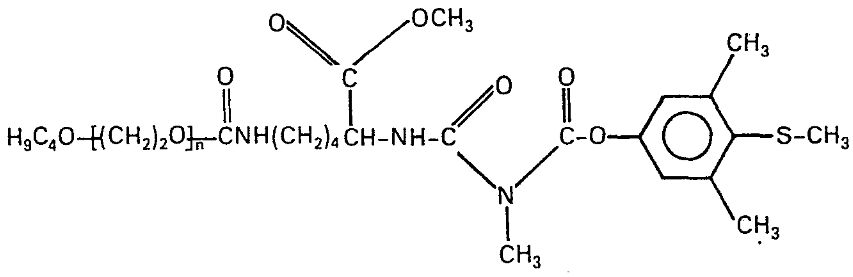

n = 16

Beispiel 73

22,1 g (0,01 mol) der nach Beispiel 1a erhaltenen Verbindung werden analog zu Beispiel 9 mit 2,1 g (0,01 mol) 3-Amino-2,5-dichlorbenzoesäure umgesetzt.

Nachrührzeit: 1 Stunde bei 80°C.

Man erhält 23,8 g eines farblosen Feststoffes. Dieser erfindungsgemäss modifizierte neue Wirkstoff ist gut in Wasser löslich und besitzt die idealisierte Formel

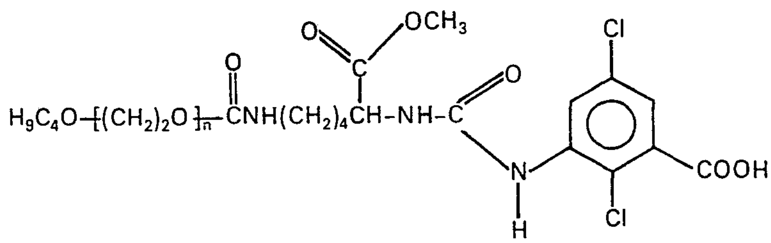

n = 43

Beispiel 74

22,1 g (0,01 mol) der nach Beispiel 1a erhaltenen Verbindung werden analog zu Beispiel 10 mit 2,9 g (0,01 mol) 3-(d-Tetralyl)-4-hydroxycumarin umgesetzt.

Nachrührzeit: 4 Stunden bei 80°C.

Man erhält 24,6 g eines dunkelgelben Feststoffes. Dieser erfindungsgemäss modifizierte neue Wirkstoff ist gut in Wasser und niederen Alkoholen löslich und besitzt die idealisierte Formel

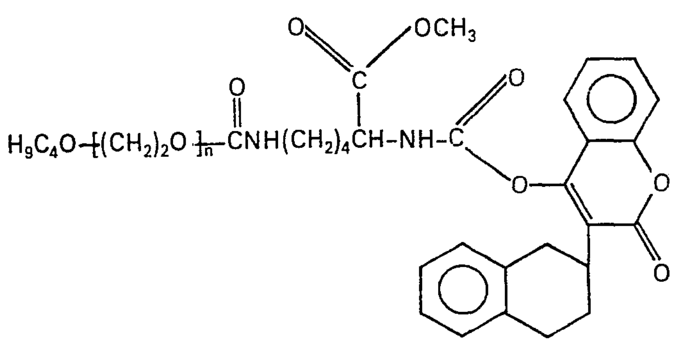

n = 43

Beispiel 75

9,9 g (0,01 mol) der nach Beispiel 3a erhaltenen Verbindung werden analog zu Beispiel 11 mit 2,9 g (0,01 mol) 3-(d-Tetralyl)-4-Hydroxycumarin umgesetzt.

Nachrührzeit: 2 Stunden bei 110°C.

Man erhält 13,4 g eines dunkelgelb-braunen, viskosen Produktes. Dieser erfindungsgemäss modifizierte neue Wirkstoff ist gut in Wasser und niederen Alkoholen löslich und besitzt die idealisierte Formel

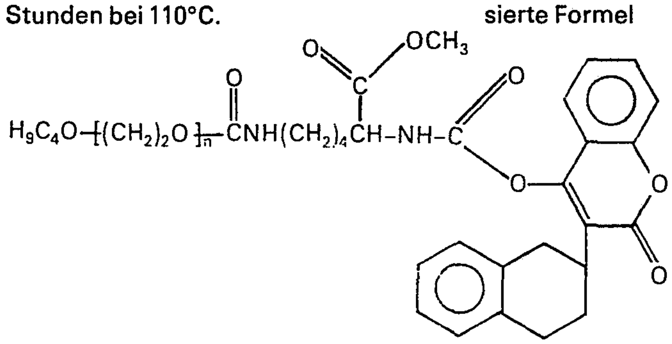

n = 16

Beispiel 76

12,1 g (0,01 mol) der nach Beispiel 2a erhaltenen Verbindung werden analog zu Beispiel 10 mit 3,1 g (0,01 mol) 3-(1'-Phenyl-2'-acetyläthyl)-4-hydroxycumarin umgesetzt.

Nachrührzeit: 3 Stunden bei 105°C.

Man erhält 14,7 g eines braunrose Feststoffes von wachsartiger Konsistenz. Dieser erfindungsgemäss modifizierte neue Wirkstoff ist gut in Wasser und niederen Alkoholen löslich und besitzt die idealisierte Formel

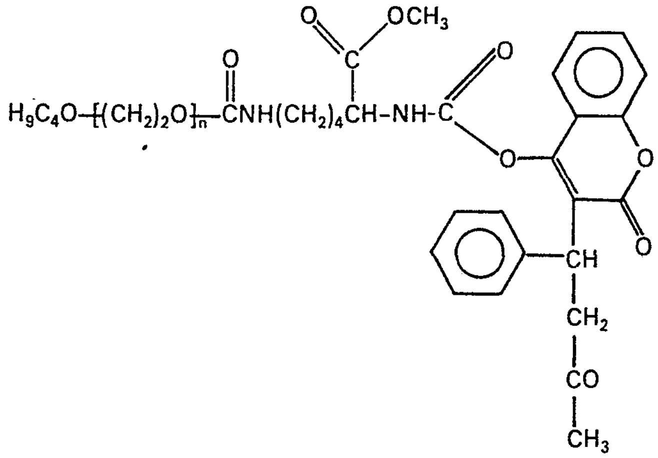

n = 21

Beispiel 77

22,1 g (0,01 mol) der nach Beispiel 1a erhaltenen Verbindung werden analog zu Beispiel 9 mit 1,2 g (0,01 mol) 4-Aminoperhydro-1,2-oxazin-3-on umgesetzt.

Nachrührzeit: 30 Minuten bei 90°C.

Man erhält 22,9 g eines gelben Feststoffes. Dieser erfindungsgemässe modifizierte neue Wirkstoff ist gut in Wasser und/oder niederen Alkoholen löslich und besitzt die idealisierte Formel

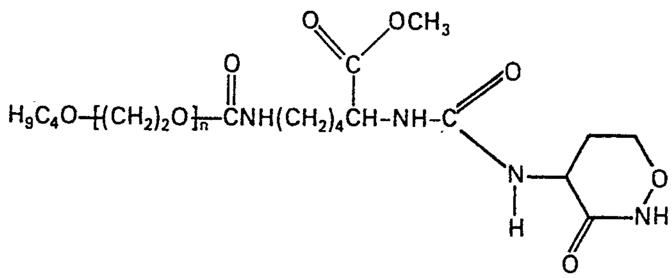

n = 43

Beispiel 78

12,1 g (0,01 mol) der nach Beispiel 2a erhaltenen Verbindung werden analog zu Beispiel 10 mit 1,2 g (0,01 mol) Allylthioharnstoff umgesetzt.

Nachrührzeit: 36 Stunden bei 70°C.

Man erhält 22,7 g eines goldgelben Feststoffes. Dieser erfindungsgemäss modifizierte neue Wirkstoff ist gut in Wasser und niederen Alkoholen löslich und besitzt die idealisierte Formel

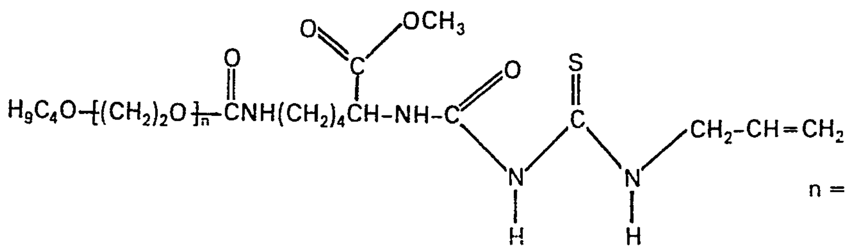

n = 21

Beispiel 79

9,9 g (0,01 mol) der nach Beispiel 3a erhaltenen Verbindung werden analog zu Beispiel 11 mit 1,2 g (0,01 mol) Nicotinsäureamid umgesetzt.

Nachrührzeit: 20 Stunden bei 105°C.

Man erhält 10,4 g eines dunkelgelbroten, viskosen Produktes. Dieser erfindungsgemäss modifizierte neue Wirkstoff ist gut in Wasser und Methanol sowie Äthanol löslich und besitzt die idealisierte Formel

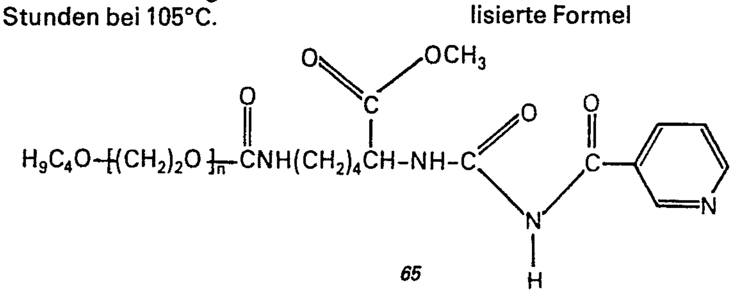

n = 16

Beispiel 80

22,1 g (0,01 mol) der nach Beispiel 1a erhaltenen Verbindung werden analog zu Beispiel 9 mit 1,3 g (0,01 mol) Isonicotinsäurehydrazid umgesetzt.

Nachrührzeit: 2 Stunden bei 95°C.

Man erhält 22,8 g eines hellgelblichweissen Feststoffes. Dieser erfindungsgemäss modifizierte neue Wirkstoff ist gut in Wasser und/oder niederen Alkoholen löslich und besitzt die idealisierte Formel

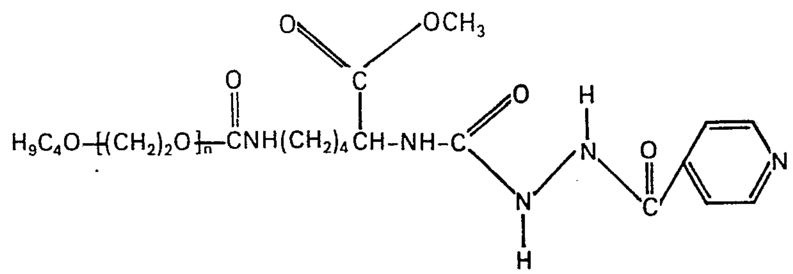

n = 43

Beispiel 81

12,1 g (0,01 mol) der nach Beispiel 2a erhaltenen Verbindung werden analog zu Beispiel 10 mit 1,3 g (0,01 mol) Isonicotinsäurehydrazid umgesetzt.

Nachrührzeit: 2 Stunden bei 95°C.

Man erhält 12,9 g eines gelbweissen Feststoffes. Dieser erfindungsgemäss modifizierte neue Wirkstoff ist gut in Wasser und/oder niederen Alkoholen löslich und besitzt die idealisierte Formel

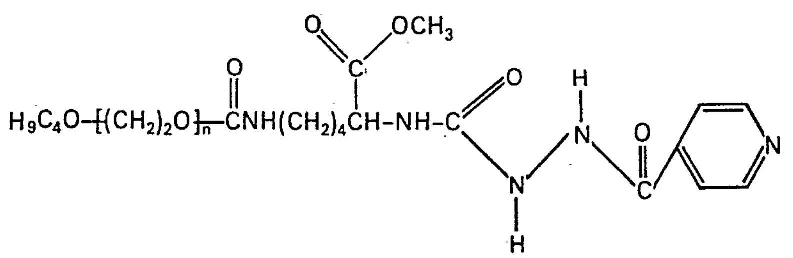

n = 21

Beispiel 82

9,9 g (0,01 mol) der nach Beispiel 3a erhaltenen Verbindung werden analog zu Beispiel 11 mit 1,3 g (0,01 mol) Isonicotinsäurehydrazid umgesetzt.

Nachrührzeit: 2 Stunden bei 95°C.

Man erhält 10,9 g eines goldgelben, viskosen Produktes. Dieser erfindungsgemäss modifizierte neue Wirkstoff ist gut in Wasser und Methanol sowie Äthanol löslich und besitzt die idealisierte Formel

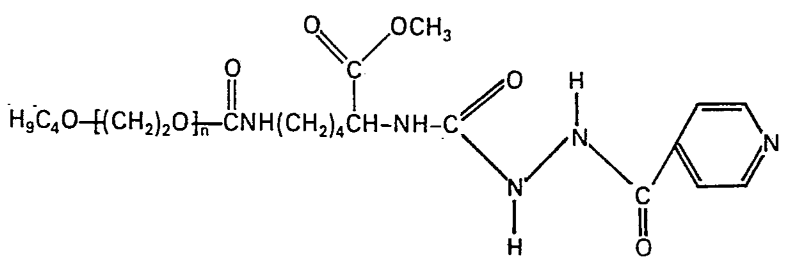

n = 16

Beispiel 83

22,1 g (0,01 mol) der nach Beispiel 1a erhaltenen Verbindung werden analog zu Beispiel 9 mit 1,8 g (0,01 mol) 1,3-Dimethylpurin-2,6-(1H, 3H)-dion umgesetzt.

Nachrührzeit: 1 Stunde bei 80°C.

Man erhält 23,6 g eines dunkelgelben Feststoffes. Dieser erfindungsgemäss modifizierte neue Wirkstoff ist gut in Wasser und/oder niederen Alkoholen löslich und besitzt die idealisierte Formel

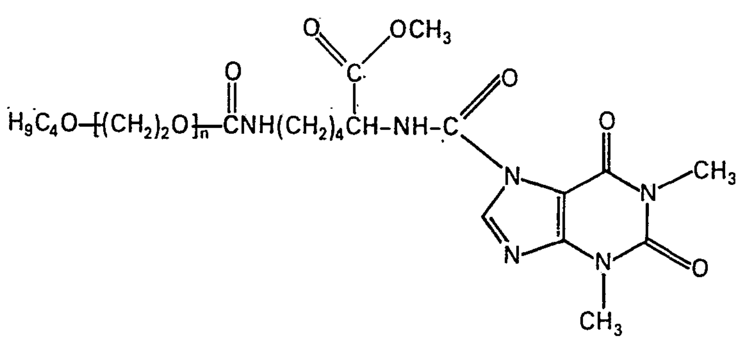

n = 43

Beispiel 84

12,1 g (0,01 mol) der nach Beispiel 2a erhaltenen Verbindung werden analog zu Beispiel 10 mit 2,4 g (0,01 mol) 4-Amino-1-ribofuranosyl-1,3,5-triazin-2(1H)-on umgesetzt.

Nachrührzeit: 2 Stunden bei 55°C.

Man erhält 14,2 g eines fast farblosen Feststoffes. Dieser erfindungsgemäss modifizierte neue Wirkstoff zeigt eine hervorragende Wasserlöslichkeit und besitzt die idealisierte Formel

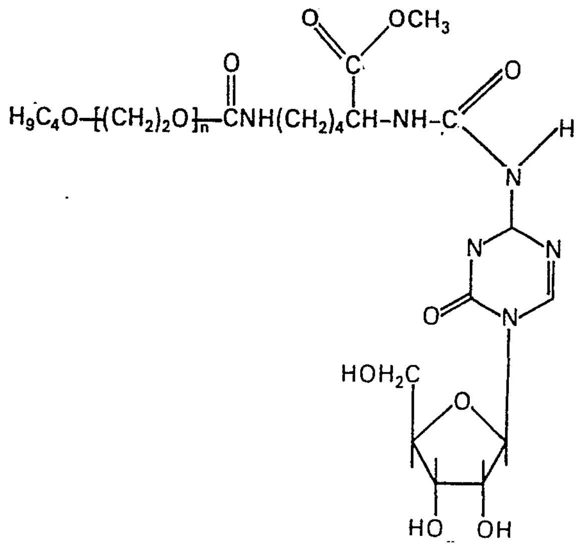

n = 21

Beispiel 85

22,1 g (0,01 mol) der nach Beispiel 1a erhaltenen Verbindung werden analog zu Beispiel 9 mit 2,6 g (0,01 mol) 2-Sulfanilamidothiazol umgesetzt.

Nachrührzeit: 30 Minuten bei 70°C.

Man erhält 24,3 g eines gelben Feststoffes. Dieser erfindungsgemäss modifizierte neue Wirkstoff ist gut in Wasser und/oder niederen Alkoholen löslich und besitzt die idealisierte Formel

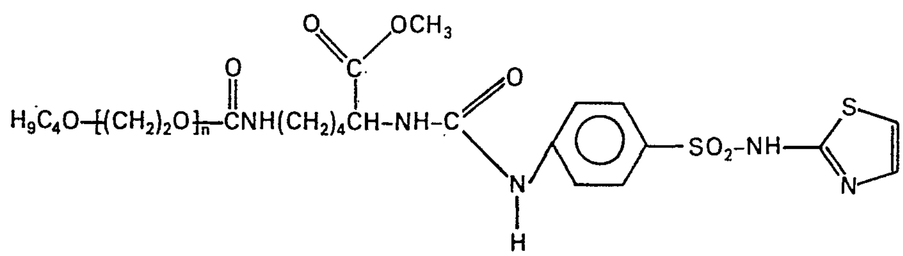

n = 43

Beispiel 86

22,1 g (0,01 mol) der nach Beispiel 1a erhaltenen Verbindung werden analog zu Beispiel 9 mit 2,6 g (0,01 mol) 3(5)-Ribofuranosyl-4-hydroxypyrazol-6(3)-carboxamid umgesetzt.

Nachrührzeit: 9 Stunden bei 50°C.

Man erhält 24,2 g eines dunkelgelbroten Feststoffes. Dieser erfindungsgemässe modifizierte neue Wirkstoff ist gut in Wasser und/oder niederen Alkoholen löslich und besitzt die idealisierte Formel

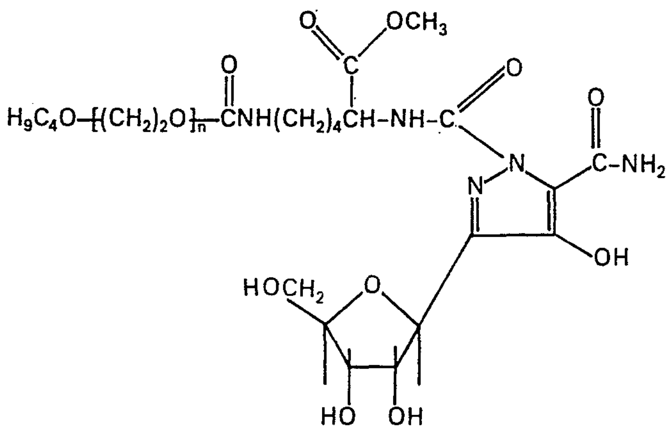

n = 43

Beispiel 87

44,2 g (0,02 mol) der nach Beispiel 1a erhaltenen Verbindung werden analog zu Beispiel 9 mit 2,2 g (0,01 mol) 2-Methyl-2-n-propyl-trimethylendicarbamat umgesetzt.

Nachrührzeit: 17 Stunden bei 110°C.

Man erhält 45,7 g eines braungelben Feststoffes. Dieser erfindungsgemäss modifizierte neue Wirkstoff ist ausgezeichnet in Wasser und/oder niederen Alkoholen löslich und besitzt die idealisierte Formel

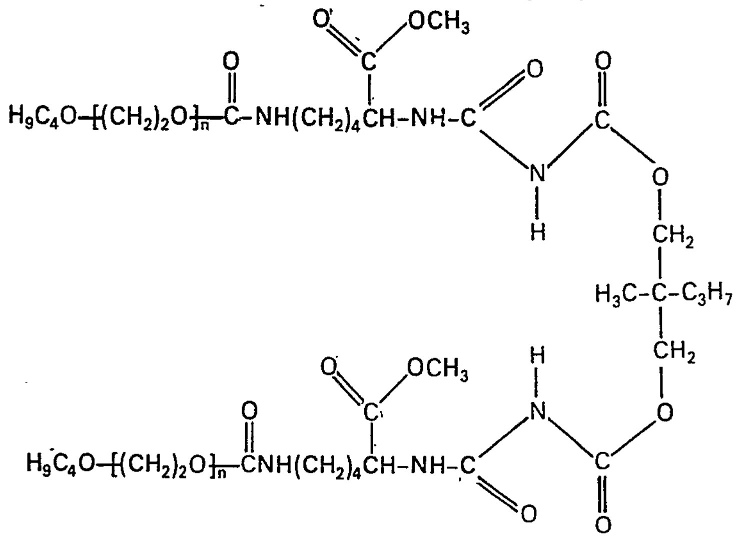

n = 43

Beispiel 88

19,8 g (0,02 mol) der nach Beispiel 3a erhaltenen Verbindung werden analog zu Beispiel 11 mit 2,2 g (0,01 mol) 2-Methyl-2-n-propyl-trimethylendicarbamat umgesetzt.

Nachrührzeit: 17 Stunden bei 110°C.

Man erhält 21,6 g eines braunroten, viskosen Produktes. Dieser erfindungsgemäss modifizierte neue Wirkstoff ist gut in Wasser und/oder niederen Alkoholen löslich und besitzt die idealisierte Formel

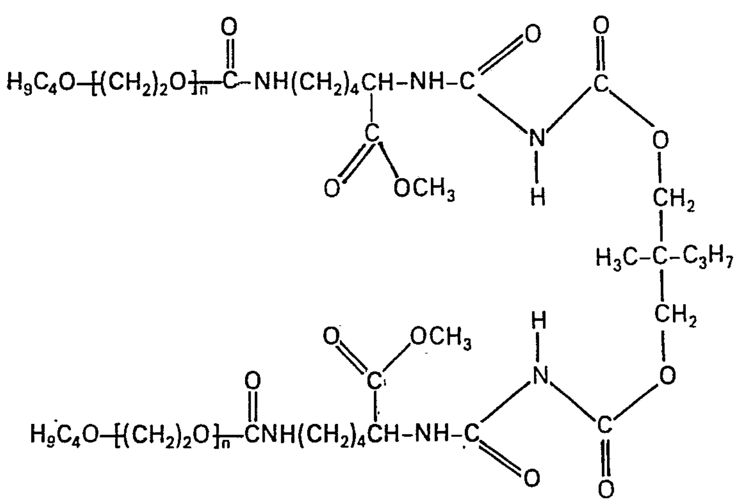

n = 16

Beispiel 89

22,1 g (0,01 mol) der nach Beispiel 1a erhaltenen Verbindung werden analog zu Beispiel 9 mit 2,2 g (0,01 mol) D(−)-threo-1-(p-Nitrophenyl)-2-dichloracetamido-1,3-propandiol umgesetzt.

Nachrührzeit: 6 Stunden bei 95°C.

Man erhält 24,0 g eines goldgelben Feststoffes. Dieser erfindungsgemäss modifizierte neue Wirkstoff ist gut in Wasser und/oder niederen Alkoholen löslich und besitzt die idealisierte Formel

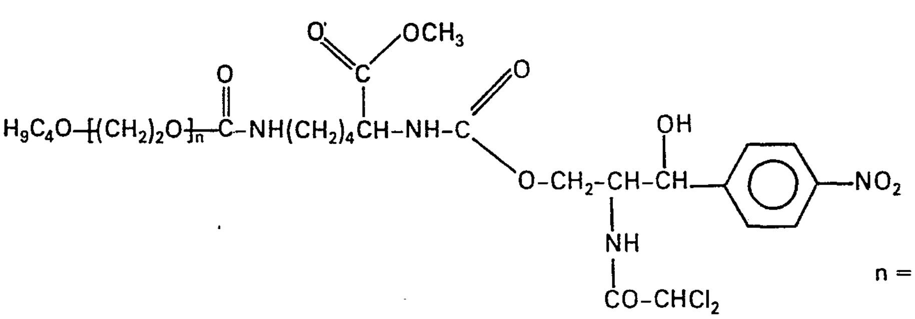

n = 43

Beispiel 90

9,9 g (0,01 mol) der nach Beispiel 3a erhaltenen Verbindung werden analog zu Beispiel 11 mit 2,2 g (0,01 mol) D(–)threo-1-(p-Nitrophenyl)-2-dichloracetamido-1,3-propandiol umgesetzt.

Nachrührzeit: 5 Stunden bei 85°C.

Man erhält 11,7 g eines rosegelben, viskosen Produktes. Dieser erfindungsgemäss modifizierte neue Wirkstoff ist gut in Wasser und/oder niederen Alkoholen löslich und besitzt die idealisierte Formel

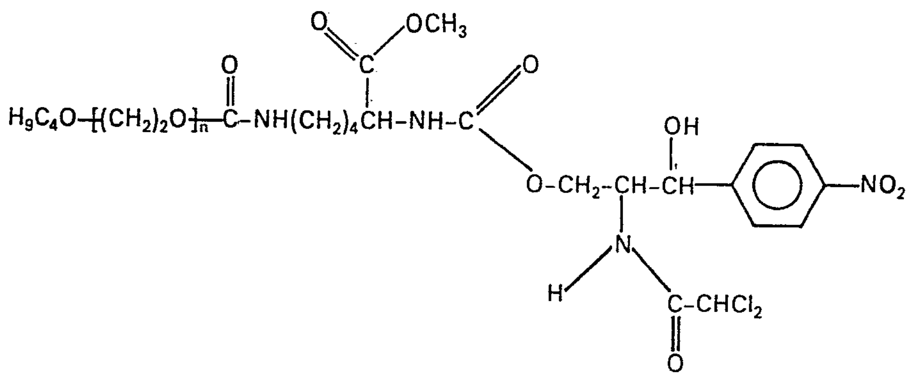

n = 16

Beispiel 91

12,1 g (0,01 mol) der nach Beispiel 2a erhaltenen Verbindung werden analog zu Beispiel 10 mit 2,2 g (0,01 mol) 5,6-Dihydro-2-(2,6-xylidino)-4H-1,3-thiazin umgesetzt.

Nachrührzeit: 1 Stunde bei 75°C.

Man erhält 14,0 g eines dunkelgelben Feststoffes. Dieser erfindungsgemäss modifizierte neue Wirkstoff ist gut in Wasser und niederen Alkoholen löslich und besitzt die idealisierte Formel

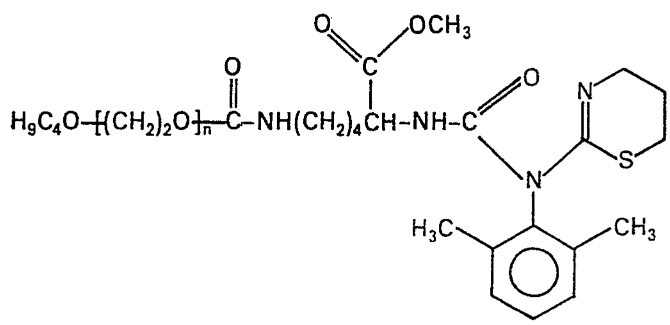

n = 21

Beispiel 92

22,1 g (0,01 mol) der nach Beispiel 1a erhaltenen Verbindung werden analog zu Beispiel 9 mit 3,9 g (0,01 mol) N-(4-Chlor-3-sulfamoylbenzolsulfonyl)-N-methyl-2-aminomethyl-2-methyltetrahydrofuran umgesetzt.

Nachrührzeit: 18 Stunden bei 95°C.

Man erhält 25 g eines braungelben Feststoffes. Dieser erfindungsgemäss modifizierte neue Wirkstoff ist gut in Wasser und niederen Alkoholen löslich und besitzt die idealisierte Formel

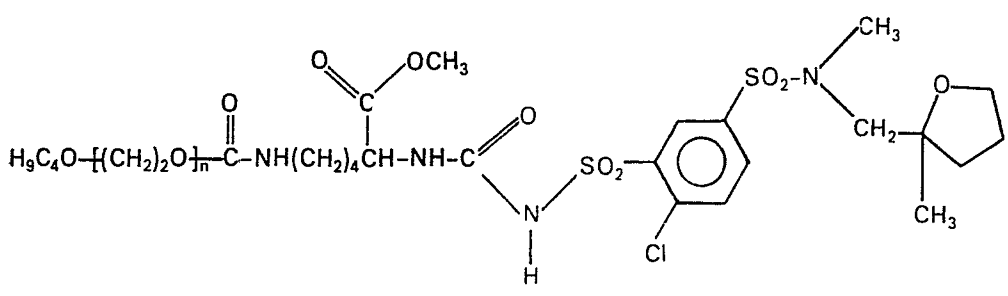

n = 43

Beispiel 93

12,1 g (0,01 mol) der nach Beispiel 2a erhaltenen Verbindung werden analog zu Beispiel 10 mit 2,4 g (0,01 mol) 2-Diäthylaminoäthyl-p-aminobenzoat umgesetzt.

Nachrührzeit: 30 Minuten bei 70°C.

Man erhält 14,2 g eines farblosen Feststoffes von wachsartiger Konsistenz. Dieser erfindungsgemäss modifizierte neue Wirkstoff ist gut in Wasser und/oder niederen Alkoholen löslich und besitzt die idealisierte Formel

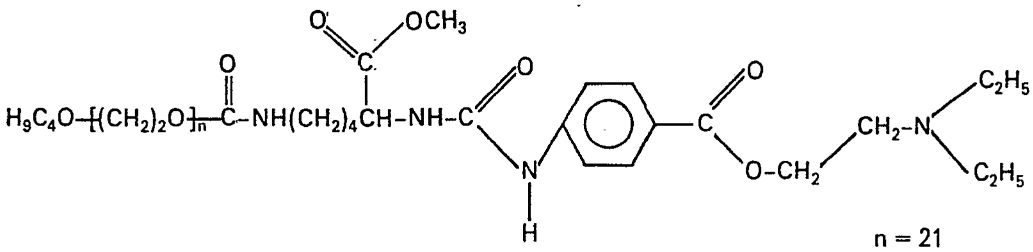

n = 21

Beispiel 94

22,1 g (0,01 mol) der nach Beispiel 1a erhaltenen Verbindung werden analog zu Beispiel 9 mit 3,6 g (0,01 mol) D(+)-6-(5-Amino-5-carboxyvaleramido)-3,3-dimethyl-7-oxo-4-thia-1-azabicyclo-[3,2,0]-heptan-2-carbonsäure umgesetzt.

Nachrührzeit: 2 Stunden bei 55°C.

Man erhält 25,3 g eines blassgelben Feststoffes. Dieser erfindungsgemäss modifizierte neue Wirkstoff ist gut in Wasser und/oder niederen Alkoholen löslich und besitzt die idealisierte Formel

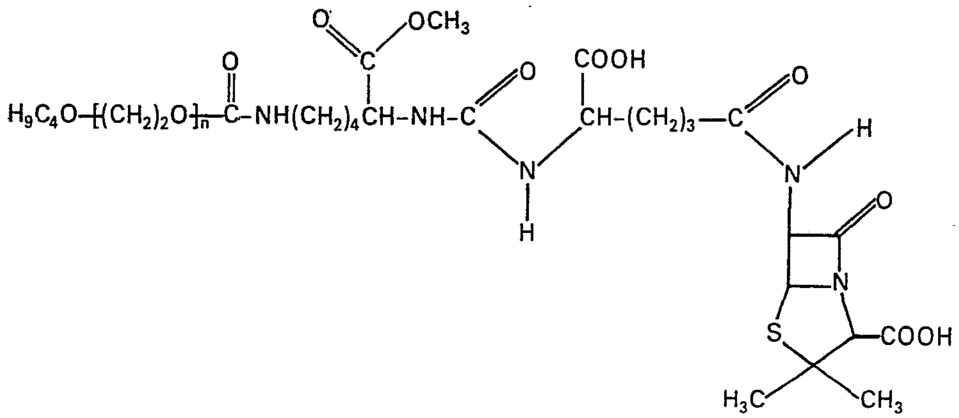

n = 43

Beispiel 95

9,9 g (0,01 mol) der nach Beispiel 3a erhaltenen Verbindung werden analog zu Beispiel 11 mit 3,2 g (0,01 mol) 1-Phenyl-5-sulfanilamidopyrazol umgesetzt.

Nachrührzeit: 45 Minuten bei 85°C.

Man erhält 12,8 g eines rosegelben, viskosen Produktes. Dieser erfindungsgemäss modifizierte neue Wirkstoff ist gut in Wasser und/oder Methanol sowie Äthanol löslich und besitzt die idealisierte Formel

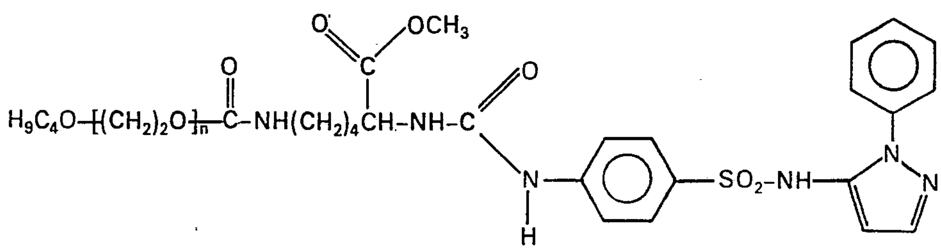

n = 16

Beispiel 96

22,1 g (0,01 mol) der nach Beispiel 1a erhaltenen Verbindung werden analog zu Beispiel 9 mit 3,8 g (0,01 mol) D-7-(2-Amino-2-phenylacetamido)-3-methyl-8-oxo-5-thia-1-oxobicyclo [4,2,0]oct-2-en-2-carbonsäure umgesetzt.

Nachrührzeit: 3 Stunden bei 55°C.

Man erhält 25,6 g eines blassgelben Feststoffes. Dieser erfindungsgemäss modifizierte neue Wirkstoff ist gut in Wasser und/oder niederen Alkoholen löslich und besitzt die idealisierte Formel

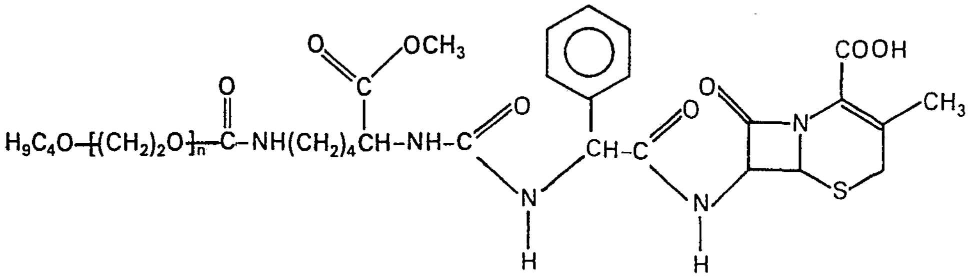

n = 43

Beispiel 97

12,1 g (0,01 mol) der nach Beispiel 2a erhaltenen Verbindung werden analog zu Beispiel 10 mit 3,5 g (0,01 mol) D(–)-6-(α-Aminophenylacetamido)-3,3-dimethyl-7-oxo-4-thia-1-azabicyclo-[3,2,0]-heptan-2-carbonsäure umgesetzt.

Nachrührzeit: 45 Minuten bei 60°C.

Man erhält 15,3 g eines blassgelben Feststoffes. Dieser erfindungsgemäss modifizierte neue Wirkstoff ist gut in Wasser und/oder niederen Alkoholen löslich und besitzt die idealisierte Formel

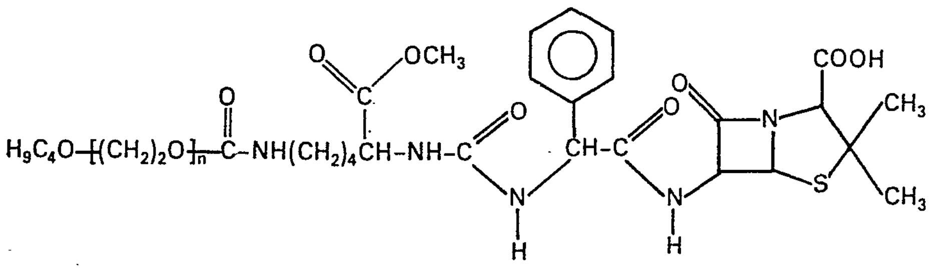

n = 21

Beispiel 98

9,9 g (0,01 mol) der nach Beispiel 3a erhaltenen Verbindung werden analog zu Beispiel 11 mit 3,3 g (0,01 mol) D(+)lysergsäure-β-hydroxyisopropylamid umgesetzt.

Nachrührzeit: 1 Stunde bei 70°C.

Man erhält 13,1 g eines gelbroten viskosen Produktes. Dieser erfindungsgemäss modifizierte neue Wirkstoff ist gut in Wasser und/oder Äthanol löslich und besitzt die idealisierte Formel

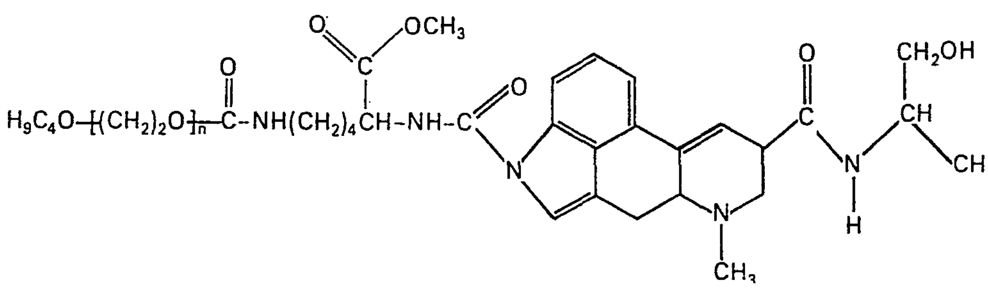

n = 16

Beispiel 99

22,1 g (0,01 mol) der nach Beispiel 1a erhaltenen Verbindung werden analog zu Beispiel 9 mit 5,3 g (0,01 mol) 9-Acetyl-7,8,9,10-tetrahydro-6,7,9,11-tetrahydroxy-4-methoxy-5,12-naphthacenchinon-7-(3-Amino-5-methyl-2,3-didesoxy-α-lyxo-pyranosid) umgesetzt.

Nachrührzeit: 2 Stunden bei 55°C.

Man erhält 27,0 g eines blassgelben Feststoffes. Dieser erfindungsgemäss modifizierte neue Wirkstoff ist gut in Wasser und/oder niederen Alkoholen löslich und besitzt die idealisierte Formel

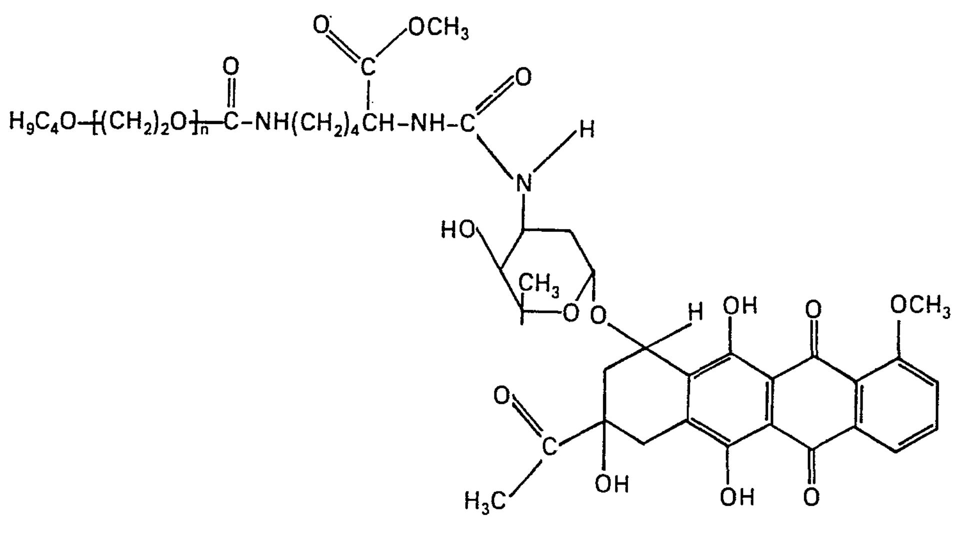

n = 43

Beispiel 100

22,1 g (0,01 mol) der nach Beispiel 1a erhaltenen Verbindung werden analog zu Beispiel 9 mit 1,7 g (0,01 mol) 6-Aminopurin-8-thiol umgesetzt.

Nachrührzeit: 15 Minuten bei 45°C.

Man erhält 23,4 g eines rotgelben Feststoffes. Dieser erfindungsgemäss modifizierte neue Wirkstoff ist gut in Wasser und/oder niederen Alkoholen löslich und besitzt die idealisierte Formel

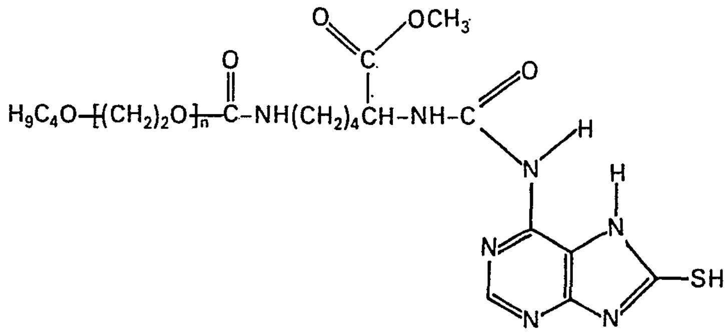

n = 43

Beispiel 101

22,1 g (0,01 mol) der nach Beispiel 1a erhaltenen Verbindung werden analog zu Beispiel 9 mit 2,2 g (0,01 mol) 6-Amino-3,3-dimethyl-7-oxo-4-thia-1-azabicyclo-[3,2,0]-heptan-carbonsäure umgesetzt.

Nachrührzeit: 30 Minuten bei 60°C.

Man erhält 24,0 g eines weisslichgelben Feststoffes. Dieser erfindungsgemäss modifizierte neue Wirkstoff ist gut in Wasser und/oder niederen Alkoholen sowie Essigester und Aceton löslich und besitzt die idealisierte Formel

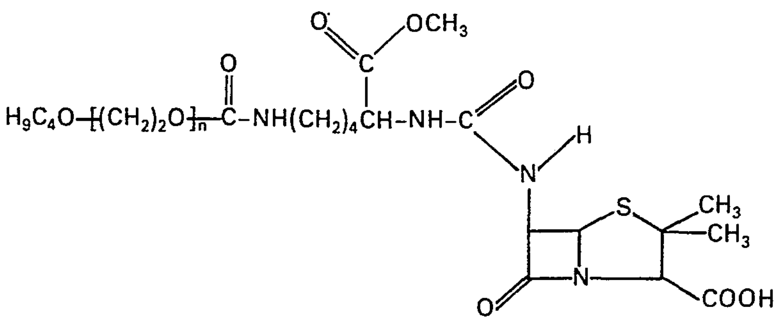

n = 43

Beispiel 102

9,9 g (0,01 mol) der nach Beispiel 3a erhaltenen Verbindung werden analog zu Beispiel 11 mit 2,4 g (0,01 mol) 2-Äthyl-2-brombutyryl-harnstoff umgesetzt.

Nachrührzeit: 36 Stunden bei 95°C.

Man erhält 12 g eines rotbraunen, viskosen Produktes. Dieser erfindungsgemäss modifizierte neue Wirkstoff ist gut in Wasser und Methanol sowie Äthanol löslich und besitzt die idealisierte Formel

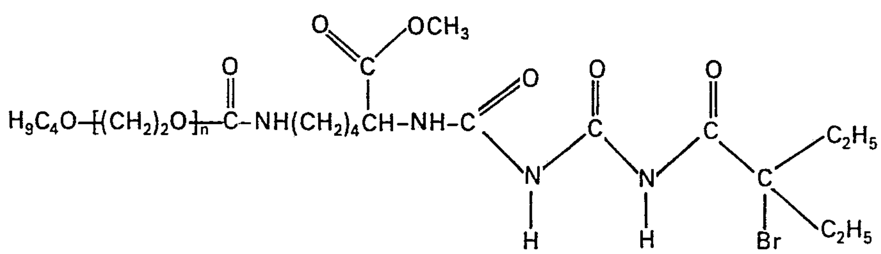

n = 16

Beispiel 103

12,1 g (0,01 mol) der nach Beispiel 2a erhaltenen Verbindung werden analog zu Beispiel 10 mit 2,3 g (0,01 mol) 4-Aminobenzolsulfothiocarbamid umgesetzt.

Nachrührzeit: 20 Minuten bei 85°C.

Man erhält 14,1 g eines rotgoldenen, wachsartigen Feststoffes. Dieser erfindungsgemäss modifizierte neue Wirkstoff ist gut in Wasser und/oder niederen Alkoholen löslich und besitzt die idealisierte Formel

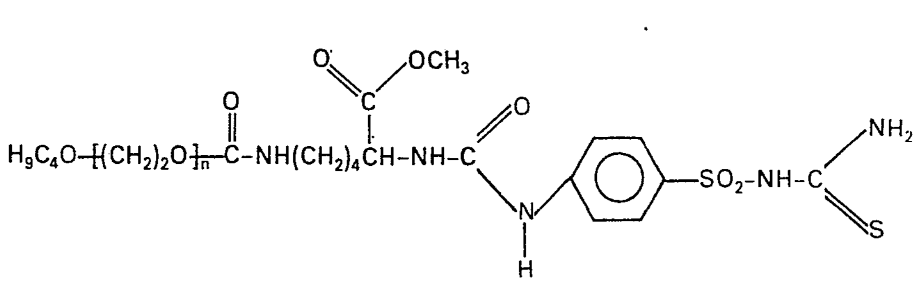

n = 21

Beispiel 104

22,1 g (0,01 mol) der nach Beispiel 1a erhaltenen Verbindung werden analog zu Beispiel 9 mit 3,5 g (0,01 mol) 2,6-Dimethyl-3,5-diacetyl-4-o-nitrophenyl-1,4-dihydropyridin umgesetzt.

Nachrührzeit: 1 Stunde bei 70°C.

Man erhält 25,3 g eines dunkelgelben Feststoffes. Dieser erfindungsgemäss modifizierte neue Wirkstoff ist gut in Wasser und/oder niederen Alkoholen löslich und besitzt die idealisierte Formel

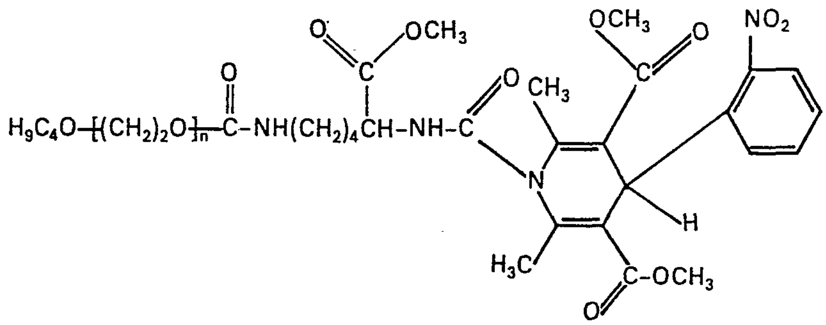

n = 43

Beispiel 105

12,1 g (0,01 mol) der nach Beispiel 2a erhaltenen Verbindung werden analog zu Beispiel 10 mit 3,5 g (0,01 mol) 2,6-Dimethyl-3,5-Diacetyl-4-o-nitrophenyl-1,4-dihydropyridin umgesetzt.

Nachrührzeit: 1 Stunde bei 70°C.

Man erhält 15,1 g eines dunkelgelben, wachsartigen Feststoffes. Dieser erfindungsgemäss modifizierte neue Wirkstoff ist gut in Wasser und/oder niederen Alkoholen löslich und besitzt die idealisierte Formel

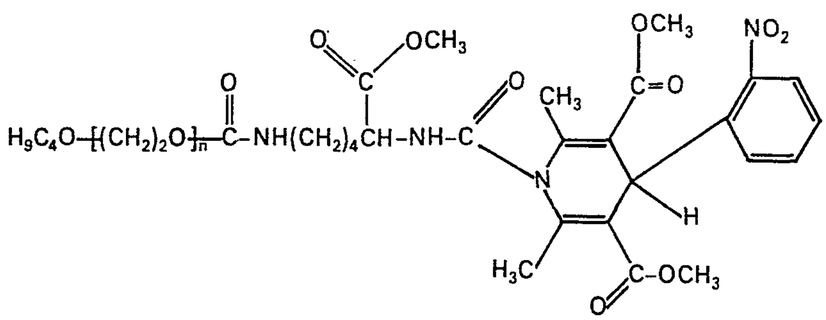

n = 21

Beispiel 106

9,9 g (0,01 mol) der nach Beispiel 3a erhaltenen Verbindung werden analog zu Beispiel 11 mit 3,5 g (0,01 mol) 2,6-Dimethyl-3,5-Diacetyl-4-o-nitrophenyl-1,4-dihydropyridin umgesetzt.

Nachrührzeit: 1 Stunde bei 70°C.

Man erhält 13,0 g eines rötlichgelben, viskosen Produktes. Dieser erfindungsgemäss modifizierte neue Wirkstoff ist gut in Wasser und/oder niederen Alkoholen löslich und besitzt die idealisierte Formel

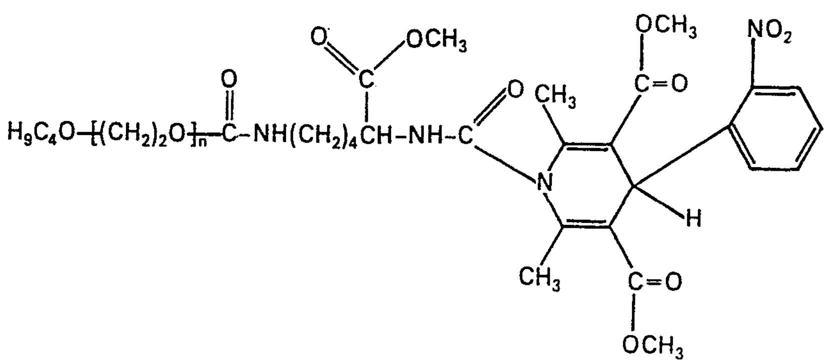

n = 16

Beispiel 107

22,1 g (0,01 mol) der nach Beispiel 1a erhaltenen Verbindung werden analog zu Beispiel 9 mit 2,8 g (0,01 mol) 2-Sulfanilamido-5-methoxypyrimidin umgesetzt.

Nachrührzeit: 30 Minuten bei 75°C.

Man erhält 24,7 g eines hellgelben Feststoffes. Dieser erfindungsgemäss modifizierte neue Wirkstoff ist gut in Wasser und/oder niederen Alkoholen löslich und besitzt die idealisierte Formel

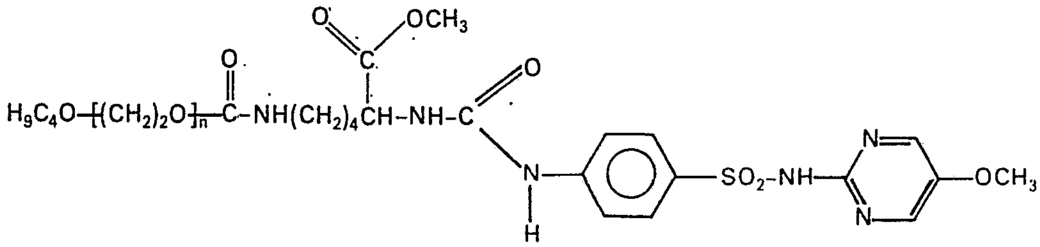

n = 43

Beispiel 108

108,4 g (0,05 mol) der nach Beispiel 1b erhaltenen Verbindung werden analog zu Beispiel 25 mit 10,7 g (0,05 mol) 4-Amino-6-tert.-butyl-3-(methylthio)-1,2,4-triazin-5(4H)-on umgesetzt.

Nachrührzeit: 3 Stunden bei 105°C.

Man erhält 117,4 g eines gelben Feststoffes. Dieser erfindungsgemäss modifizierte neue Wirkstoff ist sehr gut in Wasser und/oder niederen Alkoholen löslich und besitzt die idealisierte Formel

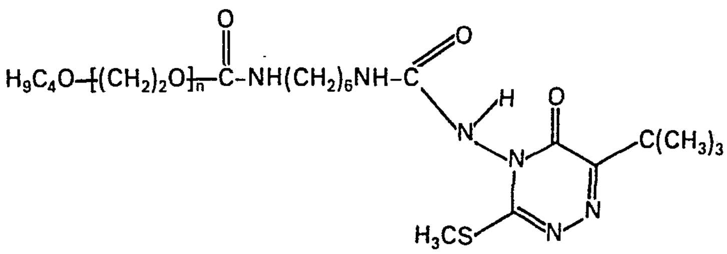

n = 43

Beispiel 109

58,4 g (0,05 mol) der nach Beispiel 2b erhaltenen Verbindung werden analog zu Beispiel 26 mit 10,7 g (0,05 mol) 4-Amino-6-tert.-butyl-3-(methylthio)-1,2,4-triazin-5-(4H)-on umgesetzt.

Nachrührzeit: 2 Stunden bei 100°C.

Man erhält 68,3 g eines dunkelgelben, wachsartigen Feststoffes. Dieser erfindungsgemäss modifizierte neue Wirkstoff ist gut in Wasser und/oder niederen Alkoholen löslich und besitzt die idealisierte Formel

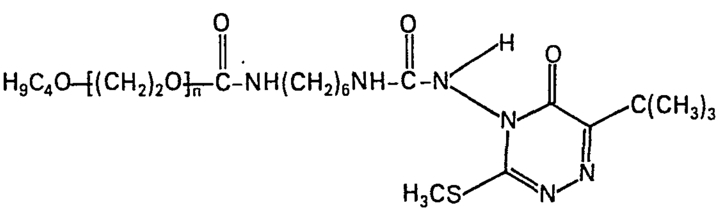

n = 21

Beispiel 110

47,6 g (0,05 mol) der nach Beispiel 3b erhaltenen Verbindung werden analog zu Beispiel 27 mit 10,7 g (0,05 mol) 4-Amino-6-tert.-butyl-3-(methylthio)-1,2,4-triazin-5(4H)-on umgesetzt.

Nachrührzeit: 115 Minuten bei 115°C.

Man erhält 58,1 g eines dunkelgelbroten, viskosen Produktes. Dieser erfindungsgemäss modifizierte neue Wirkstoff ist sehr gut in Wasser und/oder niederen Alkoholen löslich und besitzt die idealisierte Formel

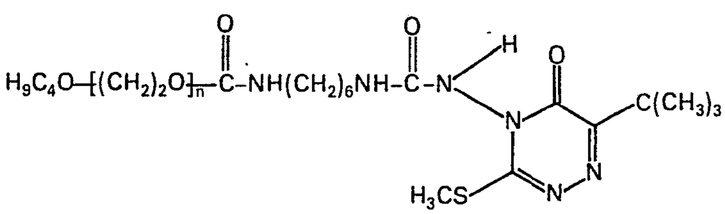

n = 16

Beispiel 111

21,7 g (0,01 mol) der nach Beispiel 1b erhaltenen Verbindung werden analog zu Beispiel 47 mit 2,2 g (0,01 mol) N-(2-Benzothiazolyl)-N,N′-dimethyl-harnstoff umgesetzt.

Nachrührzeit: 27 Stunden bei 95°C.

Man erhält 23,5 g eines dunkelgraugelben, wachsartigen Feststoffes. Dieser erfindungsgemäss modifizierte neue Wirkstoff ist gut in Wasser und/oder niederen Alkoholen löslich und besitzt die idealisierte Formel

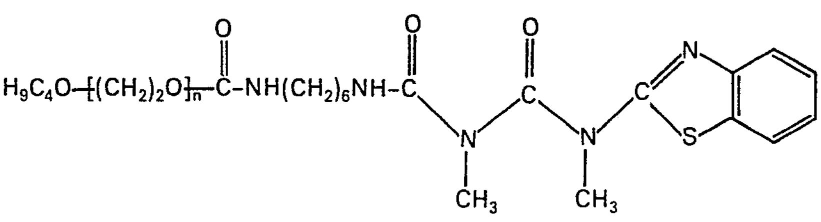

n = 43

Beispiel 112

11,7 g (0,01 mol) der nach Beispiel 2b erhaltenen Verbindung werden analog zu Beispiel 48 mit 2,2 g (0,61 mol) N-(2-Benzothiazolyl)-N,N'-dimethylharnstoff umgesetzt.

Nachrührzeit: 31 Stunden bei 85°C.

Man erhält 13,4 g eines dunkelgelbbraunen, wachsartigen Feststoffes. Dieser erfindungsgemäss modifizierte neue Wirkstoff ist gut in Wasser und/oder niederen Alkoholen löslich und besitzt die idealisierte Formel

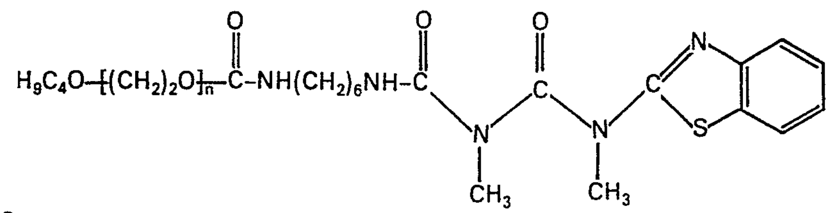

n = 21

Beispiel 113

9,5 g (0,01 mol) der nach Beispiel 3b erhaltenen Verbindung werden analog zu Beispiel 49 mit 2,2 g (0,01 mol) N-(2-Benzothiazolyl)-N,N'-dimethylharnstoff umgesetzt.

Nachrührzeit: 32 Stunden bei 75°C.

Man erhält 11,4 g eines braungelben, viskosen Produktes. Dieser erfindungsgemäss modifizierte neue Wirkstoff ist gut in Wasser und Methanol sowie Äthanol löslich und besitzt die idealisierte Formel

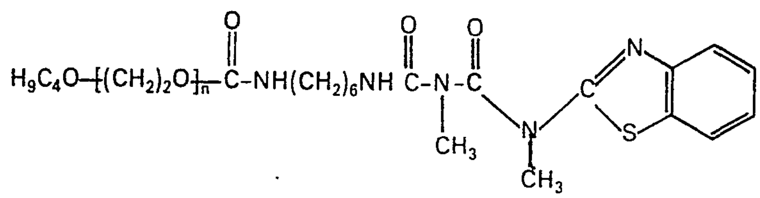

n = 16

Beispiel 114

11,7 g (0,01 mol) der nach Beispiel 2b beschriebenen Verbindung werden analog zu Beispiel 52 mit 2,6 g (0,01 mol) 1-Amino-3-(1-methyl-propyl)-5-Brom-6-methyl-1,3-diazin-(5H)-2,4-dion umgesetzt.

Nachrührzeit: 17 Stunden bei 100°C.

Man erhält 13,9 g eines dunkelgelbgrauen Feststoffes. Dieser erfindungsgemäss modifizierte neue Wirkstoff ist gut in Wasser und/oder niederen Alkoholen löslich und besitzt die idealisierte Formel

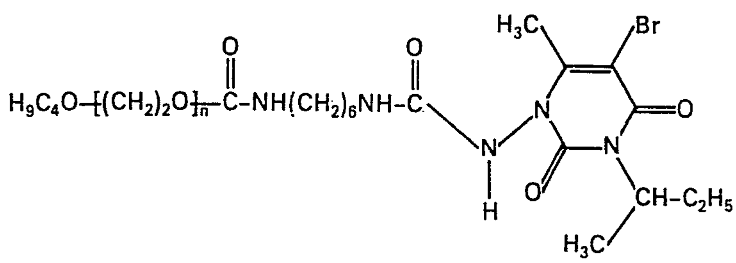

n = 21

Beispiel 115

11,7 g (0,01 mol) der nach Beispiel 2b erhaltenen Verbindung werden analog zu Beispiel 54 mit 3,1 g (0,01 mol) N-(o,o'-Difluorbenzoyl)-N'-(p-Chlorphenyl)-harnstoff umgesetzt.

Nachrührzeit: 18 Stunden bei 110°C.

Man erhält 14,5 g eines dunkelbraunen, wachsartigen Feststoffes. Dieser erfindungsgemäss modifizierte Wirkstoff ist gut in Wasser und/oder niederen Alkoholen löslich und besitzt die idealisierte Formel

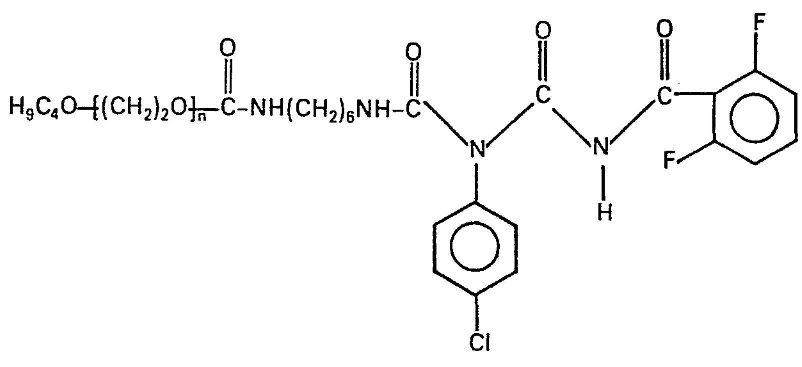

n = 21

Beispiel 116

9,5 g (0,01 mol) der nach Beispiel 3b erhaltenen Verbindung werden analog zu Beispiel 55 mit 3,7 g (0,01 mol) 3-(3,4-Dichlorphenyl)-6-(2,6-Difluorphenyl)-1-oxa-3,5-diazin-(3H,5H)-2,4-dion umgesetzt.

Nachrührzeit: 12 Stunden bei 95°C.

Man erhält 12,7 g eines graugrünbraunen, viskosen Produktes. Dieser erfindungsgemäss modifizierte neue Wirkstoff ist gut in Wasser und Methanol sowie Äthanol und Essigester löslich und besitzt die idealisierte Formel

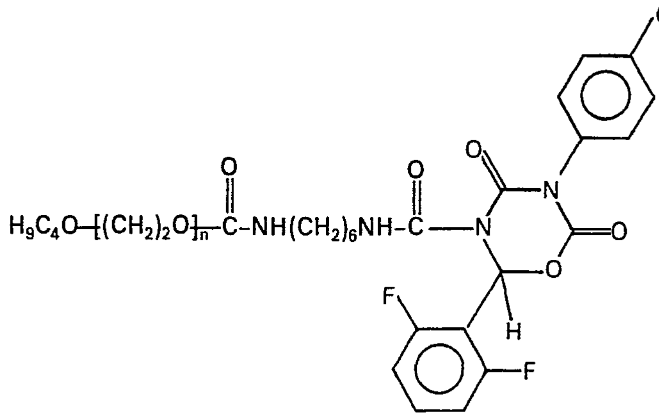

$n = 16$

Beispiel 117

21,7 g (0,01 mol) der nach Beispiel 1b erhaltenen Verbindung werden analog zu Beispiel 66 mit 2,1 g (0,01 mol) 2-Isopropyloxyphenyl-N-methylcarbamat umgesetzt.

Nachrührzeit: 7 Stunden bei 105°C.

Man erhält 23,6 g eines goldgelben Feststoffes. Dieser erfindungsgemäss modifizierte neue Wirkstoff ist gut in Wasser und/oder niederen Alkoholen löslich und besitzt die idealisierte Formel

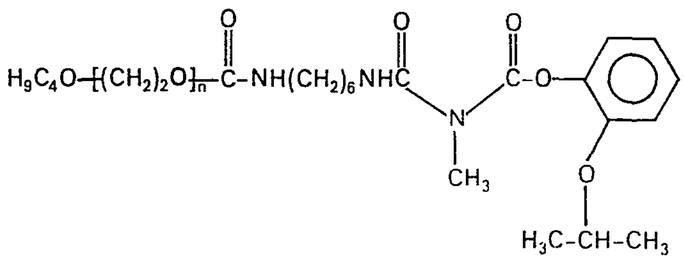

$n = 43$

Beispiel 118

11,7 g (0,01 mol) der nach Beispiel 2b erhaltenen Verbindung werden analog zu Beispiel 67 mit 2,1 g (0,01 mol) 2-Isopropyloxyphenyl-N-methylcarbamat umgesetzt.

Nachrührzeit: 6 Stunden bei 100°C.

Man erhält 13,3 g eines dunkelgelbgrauen Feststoffes von wachsartiger Konsistenz. Dieser erfindungsgemäss modifizierte neue Wirkstoff ist gut in Wasser und/oder niederen Alkoholen löslich und besitzt die idealisierte Formel

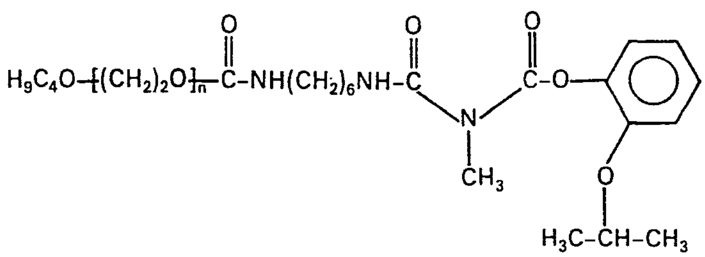

$n = 21$

Beispiel 119

9,5 g (0,01 mol) der nach Beispiel 3b erhaltenen Verbindung werden analog zu Beispiel 68 mit 2,1 g (0,01 mol) 2-Isopropyloxyphenyl-N-methylcarbamat umgesetzt.

Nachrührzeit: 9 Stunden bei 75°C.

Man erhält 11,2 g eines graugelben, viskosen Produktes. Dieser erfindungsgemäss modifizierte neue Wirkstoff ist gut in Wasser und niederen Alkoholen löslich und besitzt die idealisierte Formel

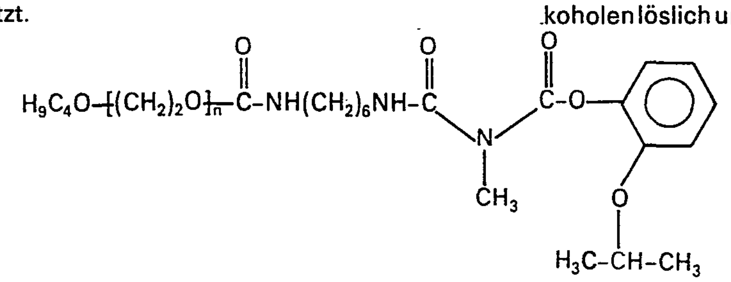

$n = 16$

Beispiel 120

21,7 g (0,01 mol) der nach Beispiel 1b erhaltenen Verbindung werden analog zu Beispiel 71 mit 2,25 g (0,01 mol) 3,5-Dimethyl-4-methyl-mercapto-phenyl-N-methyl-carbamat umgesetzt.

Nachrührzeit: 12 Stunden bei 95°C.

Man erhält 23,7 g eines braungrauen Feststoffes. Dieser erfindungsgemäss modifizierte neue Wirkstoff ist gut in Wasser und/oder niederen Alkoholen löslich und besitzt die idealisierte Formel

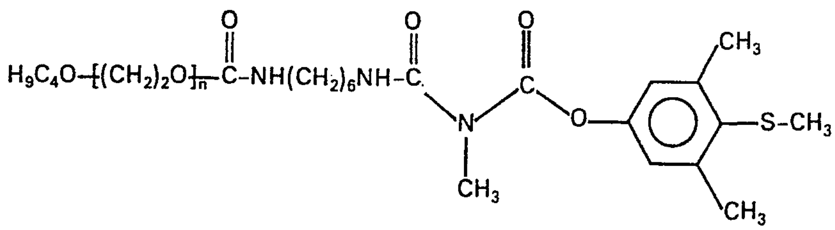

n = 43

Beispiel 121

11,7 g (0,01 mol) der nach Beispiel 2b erhaltenen Verbindung werden analog zu Beispiel 74 mit 2,9 g (0,01 mol) 3-(d-Tetralyl)-4-hydroxycumarin umgesetzt.

Nachrührzeit: 3 Stunden bei 90°C.

Man erhält 14,1 g eines dunkelrosagelben Feststoffes. Dieser erfindungsgemäss modifizierte Wirkstoff ist gut in Wasser und/oder niederen Alkoholen löslich und besitzt die idealisierte Formel

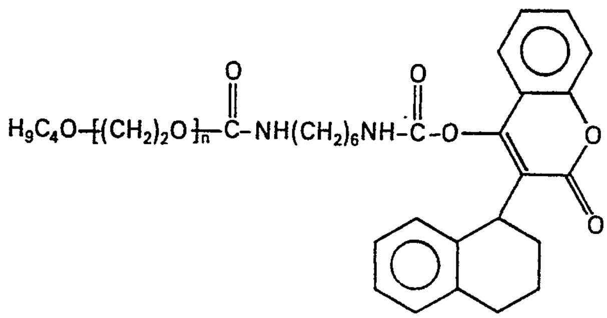

n = 21

Beispiel 122

21,7 g (0,01 mol) der nach Beispiel 3b erhaltenen Verbindung werden analog zu Beispiel 76 mit 3,1 g (0,01 mol) 3-(1′-Phenyl-2′-acetyläthyl)-4-hydroxycoumarin umgesetzt.

Nachrührzeit: 3 Stunden bei 105°C.

Man erhält 24,3 g eines rotbraunen Feststoffes. Dieser erfindungsgemäss modifizierte neue Wirkstoff ist sehr gut in Wasser und/oder niederen Alkoholen löslich und besitzt die idealisierte Formel

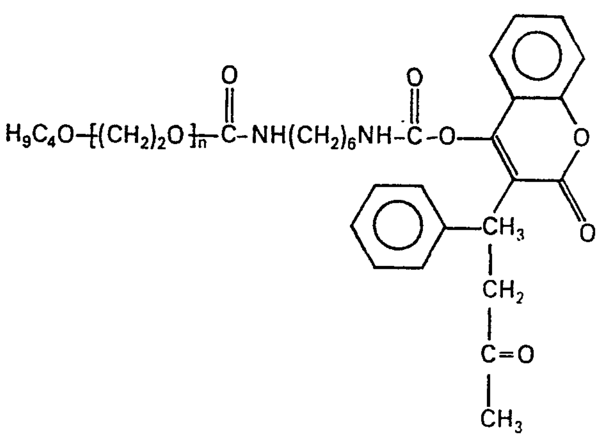

n = 43

Beispiel 123

21,7 g (0,01 mol) der nach Beispiel 1b erhaltenen Verbindung werden analog zu Beispiel 80 mit 1,3 g (0,01 mol) Isonicotinsäurehydrazid umgesetzt.

Nachrührzeit: 3 Stunden bei 90°C.

Man erhält 22,7 g eines grauweissen Feststoffes. Dieser erfindungsgemäss modifizierte neue Wirkstoff ist gut in Wasser und/oder niederen Alkoholen löslich und besitzt die idealisierte Formel

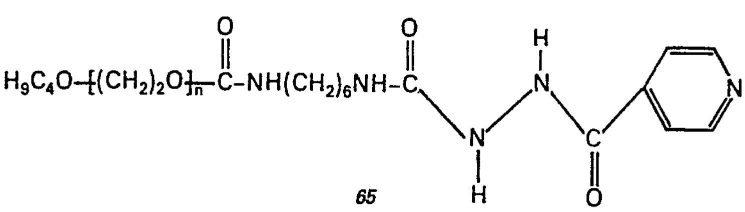

n = 43

Beispiel 124

11,7 g (0,01 mol) der nach Beispiel 2b erhaltenen Verbindung werden analog zu Beispiel 85 mit 2,6 g (0,01 mol) 2-Sulfanilamidothiazol umgesetzt.

Nachrührzeit: 30 Minuten bei 70°C.

Man erhält 13,0 g eines graugelben Feststoffes. Dieser erfindungsgemäss modifizierte neue Wirkstoff ist gut in Wasser und/oder niederen Alkoholen löslich und besitzt die idealisierte Formel

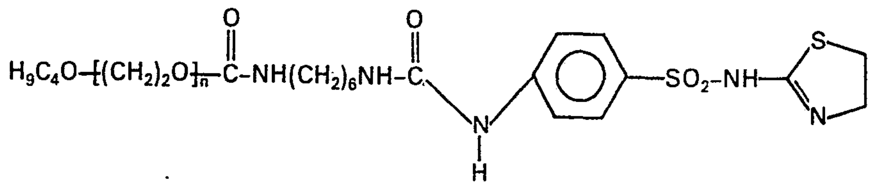

n = 21

Beispiel 125

11,7 g (0,01 mol) der nach Beispiel 2b erhaltenen Verbindung werden analog zu Beispiel 89 mit 2,2 g (0,01 mol) D(–)-threo-1-(p-Nitrophenyl)-2-dichloracetamido-1,3-propandiol umgesetzt.

Nachrührzeit: 7 Stunden bei 85°C.

Man erhält 13,5 g eines rotgoldfarbenen Feststoffes von wachsartiger Konsistenz. Dieser erfindungsgemäss modifizierte neue Wirkstoff ist gut in Wasser und/oder niederen Alkoholen löslich und besitzt die idealisierte Formel

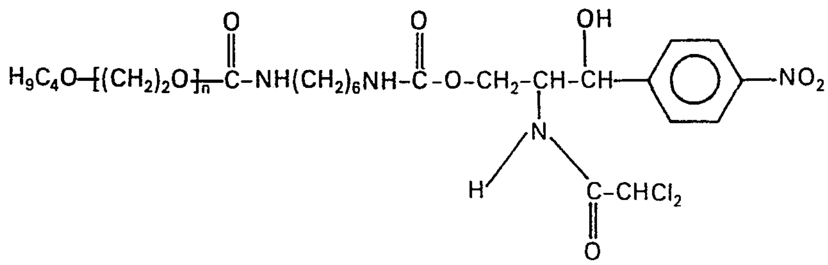

n = 21

Beispiel 126

11,7 g (0,01 mol) der nach Beispiel 2b erhaltenen Verbindung werden analog zu Beispiel 92 mit 3,9 g (0,01 mol) N-(4-Chlor-3-sulfamoylbenzolsulfonyl)-N-methyl-2-aminomethyl-2-methyltetrahydrofuran umgesetzt.

Nachrührzeit: 16 Stunden bei 100°C.

Man erhält 15,5 g eines braungelben Feststoffes von wachsartiger Konsistenz. Dieser erfindungsgemäss modifizierte neue Wirkstoff ist gut in Wasser und/oder niederen Alkoholen löslich und besitzt die idealisierte Formel

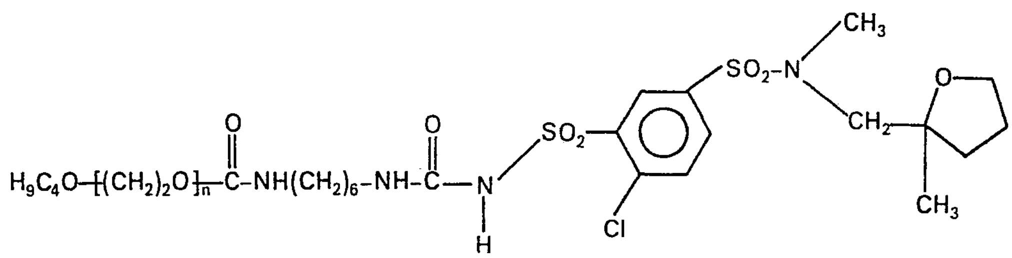

Beispiel 127

11,7 g (0,01 mol) der nach Beispiel 2 erhaltenen Verbindung werden analog zu Beispiel 94 mit 3,6 g (0,01 mol (D(+)-6-(5-Amino-5-carboxyvaleramido)-3,3-dimethyl-7-oxo-4-thia-1-azabicyclo-[3,2,0]-heptan-2-carbonsäure umgesetzt.

Nachrührzeit: 90 Minuten bei 60°C.

Man erhält 14,8 g eines graugelben Feststoffes. Dieser erfindungsgemäss modifizierte neue Wirkstoff ist gut in Wasser und/oder niederen Alkoholen löslich und besitzt die idealisierte Formel

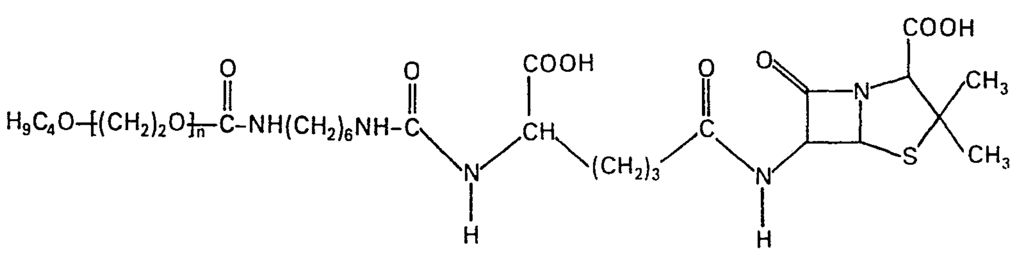

n = 21

Beispiel 128

21,7 g (0,01 mol) der nach Beispiel 1b erhaltenen Verbindung werden analog zu Beispiel 95 mit 3,2 g (0,01 mol) 1-Phenyl-5-sulfonilamidopyrazol umgesetzt.

Nachrührzeit: 30 Minuten bei 80°C.

Man erhält 24,5 g eines graurosafarbenen Produktes. Dieser erfindungsgemäss modifizierte neue Wirkstoff ist gut in Wasser und/oder niederen Alkoholen löslich und besitzt die idealisierte Formel

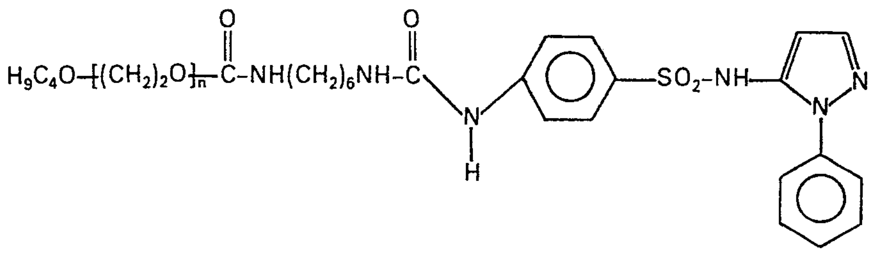

n = 43

Beispiel 129

9,5 g (0,01 mol) der nach Beispiel 3b erhaltenen Verbindung werden analog zu Beispiel 96 mit 3,8 g (0,01 mol) D-(–)-7-(2-Amino-2-phenylacetamido)-3-methyl-8-oxo-5-thia-1-azabicyclo-[4,2,0]-oct-2-en-2-carbonsäure umgesetzt.

Nachrührzeit: 4 Stunden bei 50°C.

Man erhält 12,9 g eines blassgelben, viskosen Produktes. Dieser erfindungsgemäss modifizierte neue Wirkstoff ist gut in Wasser und/oder niederen Alkoholen löslich und besitzt die idealisierte Formel

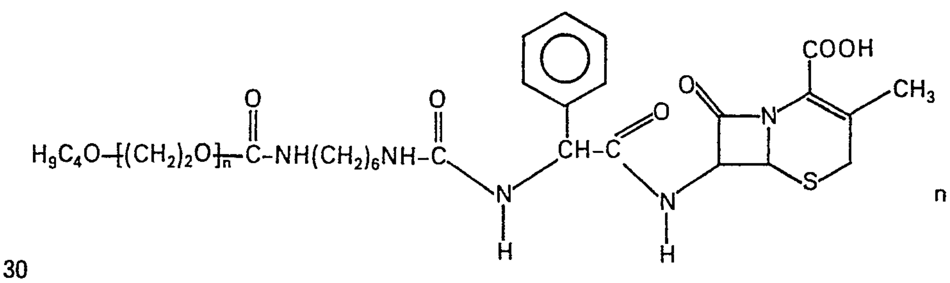

n = 16

Beispiel 130

21,7 g (0,01 mol) der nach Beispiel 1b erhaltenen Verbindung werden analog zu Beispiel 97 mit 3,5 g (0,01 mol) D(–)-6-(α-Aminophenylacetamido)-3,3-dimethyl-7-oxo-4-thia-1-azabicyclo-[3,2,0]-heptan-2-carbonsäure umgesetzt.

Nachrührzeit: 35 Minuten bei 60°C.

Man erhält 24,7 g eines blassgelben Feststoffes. Dieser erfindungsgemäss modifizierte neue Wirkstoff ist gut in Wasser und/oder niederen Alkoholen löslich und besitzt die idealisierte Formel

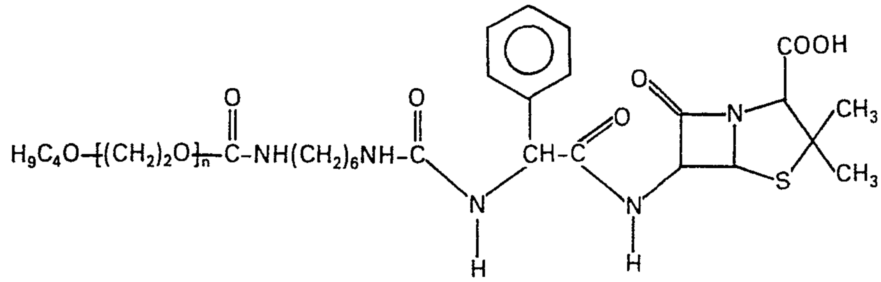

n = 43

Beispiel 131

11,7 g (0,01 mol) der nach Beispiel 2b erhaltenen Verbindung werden analog zu Beispiel 99 mit 5,3 g (0,01 mol) 9-Acetyl-7,8,9,10-tetrahydro-6,7,9,11-tetrahydroxy-4-methoxy-5,12-naphthacenchinon-7-(3-amino-5-methyl-2,3-didesoxy-α-lyxopyranosid) umgesetzt.

Nachrührzeit: 105 Minuten bei 55°C.

Man erhält 16,6 g eines blassgraugelben Feststoffes. Dieser erfindungsgemäss modifizierte neue Wirkstoff ist gut in Wasser und/oder niederen Alkoholen löslich und besitzt die idealisierte Formel

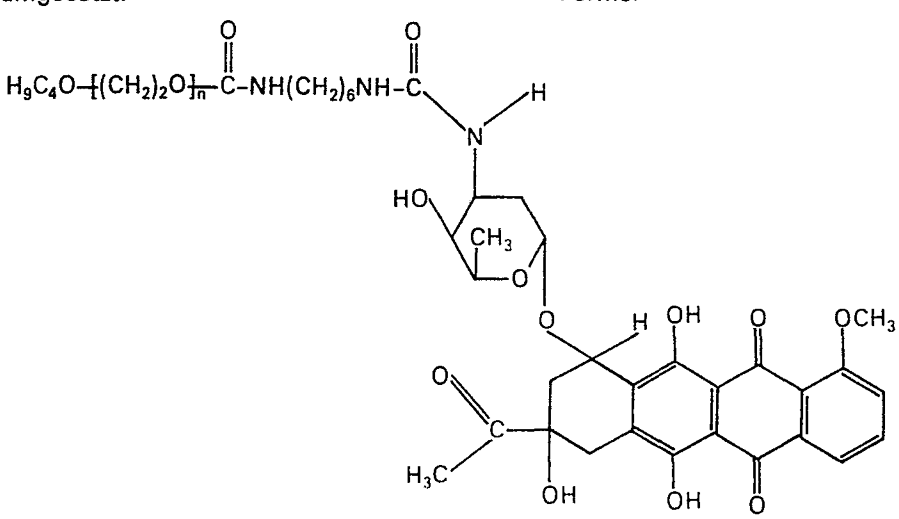

n = 21

Beispiel 132

11,7 g (0,01 mol) der nach Beispiel 2b erhaltenen Verbindung werden analog zu Beispiel 100 mit 1,7 g (0,01 mol) 6-Aminopurin-8-thiol umgesetzt.

Nachrührzeit: 15 Minuten bei 45°C.

Man erhält 13,0 g eines rotgelben, wachsartigen Feststoffes. Dieser erfindungsgemäss modifizierte neue Wirkstoff ist gut in Wasser und/oder niederen Alkoholen löslich und besitzt die idealisierte Formel

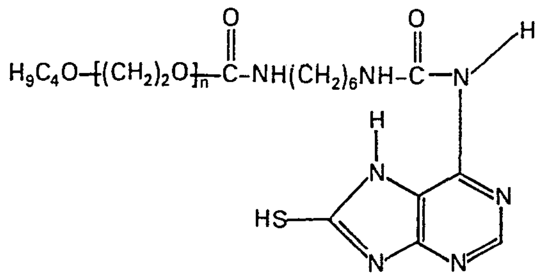

n = 21

Beispiel 133

11,7 g (0,01 mol) der nach Beispiel 2b erhaltenen Verbindung werden analog zu Beispiel 101 mit 2,2 g (0,01 mol) 6-Amino-3,3-dimethyl-7-oxo-4-thia-1-azabicyclo-[3,2,0]heptan-carbonsäure umgesetzt.

Nachrührzeit: 20 Minuten bei 60°C.

Man erhält 13,6 g eines weisslichgelben, wachsartigen Feststoffes. Dieser erfindungsgemäss modifizierte neue Wirkstoff ist gut in Wasser und/oder niederen Alkoholen löslich und besitzt die idealisierte Formel

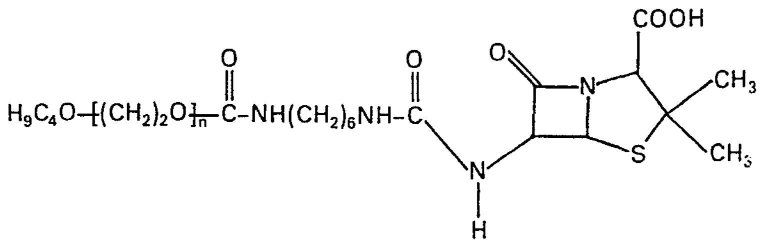

n = 21

Beispiel 134

21,7 g (0,01 mol) der nach Beispiel 1b erhaltenen Verbindung werden analog zu Beispiel 103 mit 2,3 g (0,01 mol) 4-Aminobenzolsulfothiocarbamid umgesetzt.

Nachrührzeit: 15 Minuten bei 85°C.

Man erhält 23,6 g eines rötlichgelben Feststoffes. Dieser erfindungsgemäss modifizierte neue Wirkstoff ist gut in Wasser und/oder niederen Alkoholen löslich und besitzt die idealisierte Formel

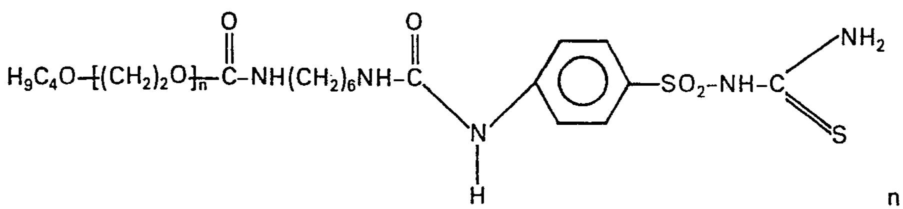

n = 43

Beispiel 135

21,7 g (0,01 mol) der nach Beispiel 1b erhaltenen Verbindung werden analog zu Beispiel 104 mit 3,5 g (0,01 mol) 2,6-Dimethyl-3,5-Diacetyl-4-o-nitrophenyl-1,4-dihydropyridin umgesetzt.

Nachrührzeit: 45 Minuten bei 75°C.

Man erhält 24,7 g eines graugelben Feststoffes. Dieser erfindungsgemäss modifizierte neue Wirkstoff ist gut in Wasser und/oder niederen Alkoholen löslich und besitzt die idealisierte Formel

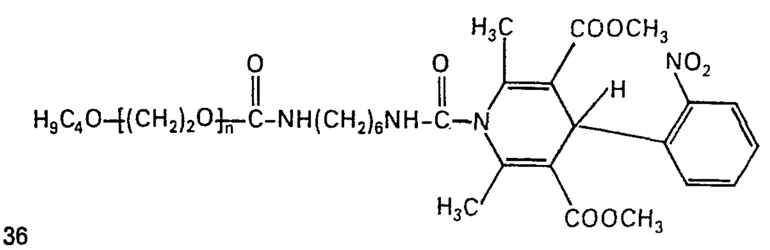

n = 43

Beispiel 136

11,7 g (0,01 mol) der nach Beispiel 2b erhaltenen Verbindung werden analog zu Beispiel 105 mit 3,5 g (0,01 mol) 2,6-Dimethyl-3,5-diacetyl-4-o-nitrophenyl-1,4-dihydropyridin umgesetzt.

Nachrührzeit: 55 Minuten bei 75°C.

Man erhält 14,8 g eines dunkelgelben, wachsartigen Feststoffes. Dieser erfindungsgemäss modifizierte neue Wirkstoff ist gut in Wasser und niederen Alkoholen löslich und besitzt die idealisierte Formel

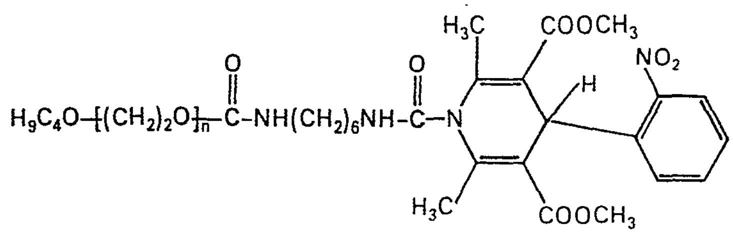

n = 21

Beispiel 137

9,5 g (0,01 mol) der nach Beispiel 3b erhaltenen Verbindung werden analog zu Beispiel 106 mit 3,5 g (0,01 mol) 2,6-Dimethyl-3,5-diacetyl-4-o-nitrophenyl-1,4-dihydropyridin umgesetzt.

Nachrührzeit: 80 Minuten bei 65°C.

Man erhält 12,6 g eines rötlichgraugelben, viskosen Produktes. Dieser erfindungsgemäss modifizierte neue Wirkstoff ist gut in Wasser und Methanol sowie Äthanol löslich und besitzt die idealisierte Formel

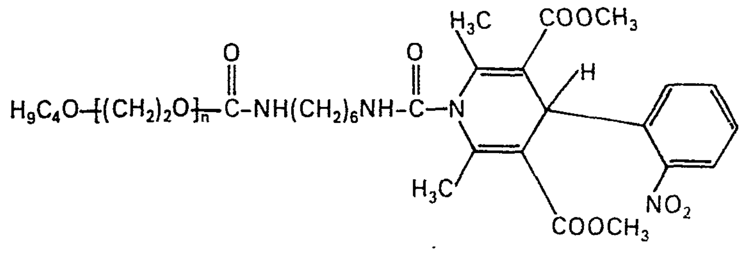

n = 16

Beispiel 138

11,7 g (0,01 mol) der nach Beispiel 2b erhaltenen Verbindung werden analog zu Beispiel 107 mit 2,8 g (0,01 mol) 2-Sulfanilamido-5-methoxypyrimidin umgesetzt.

Nachrührzeit: 20 Minuten bei 65°C.

Man erhält 13,1 g eines graugelben Feststoffes, von wachsartiger Konsistenz. Dieser erfindungsgemäss modifizierte neue Wirkstoff ist gut in Wasser und/oder niederen Alkoholen löslich und besitzt die idealisierte Formel

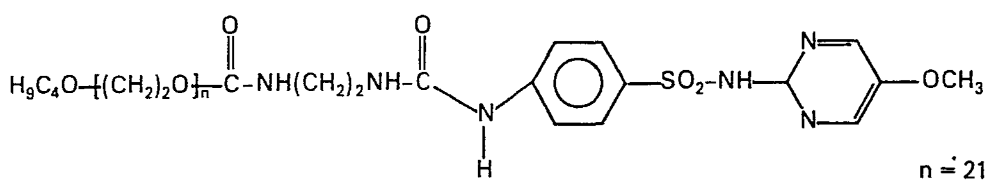

n = 21

Beispiel 139

21,4 g (0,01 mol) des nach Beispiel 1c erhaltenen Produktes werden in 100 ml absolutem Toluol gelöst. Zu dieser Lösung lässt man innerhalb einer halben Stunde eine Lösung von 2,14 g (0,01 mol) 4-Amino-6-tert.-butyl-3-(methylthio)-1,2,4-triazin-5(4H)-on in 50 ml Toluol bei 105°C zutropfen. Entstehendes Methanol wird während des Zutropfens und nachher bis zum Ende der Reaktion über einen Claisenaufsatz abdestilliert. Anschliessend wird die Temperatur auf 120°C im Bad erhöht und das Lösungsmittel abdestilliert.

Nachrührzeit: 8 Stunden bei 120°C im Bad.

Anschliessend lässt man auf 80°C abkühlen und evakuiert auf 25 mbar, wodurch letzte Spuren am Methanol und Lösungsmittel entfernt werden. Man kühlt auf Raumtemperatur ab, belüftet mit Stickstoff und erhält 23,3 g eines braunroten Feststoffes. Dieser erfindungsgemäss modifizierte neue Wirkstoff ist gut in Wasser und/oder niederen Alkoholen löslich und besitzt die idealisierte Formel

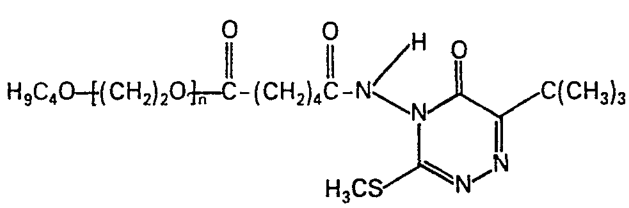

n = 43

Beispiel 140

9,2 g (0,01 mol) des nach Beispiel 3c erhaltenen Produktes werden in 50 ml absolutem Toluol gelöst. Zu dieser Lösung lässt man innerhalb 1 Stunde eine Lösung von 3,4 g (0,01 mol) 1,3-Di-p-Chlorphenyl-1H, 2H, 3H, 4H, 5H, 6H)-1,3,5-triazin-2,4-dion in 50 ml absolutem Aceton unter starkem Rühren bei 80°C zutropfen, wobei der grösste Teil des Acetons während des Zutropfens über einen Claisenaufsatz abdestilliert. Man erhöht die Temperatur auf 120°C im Bad und destilliert über eine Vigraux-Kolonne bei der Reaktion entstehendes Methanol ab.

Nachrührzeit: 24 Stunden bei 120°C im Bad.

Anschliessend wird das Lösungsmittel abdestillert und danach der Rückstand im Vakuum (25 mbar) von Resten an Methanol und Lösungsmittel

befreit.

Man erhält 12,2 g eines dunkelbraunen, viskosen Produktes. Dieser erfindungsgemäss modifizierte neue Wirkstoff ist gut in Wasser und Methanol sowie Äthanol löslich und besitzt die idealisierte Formel

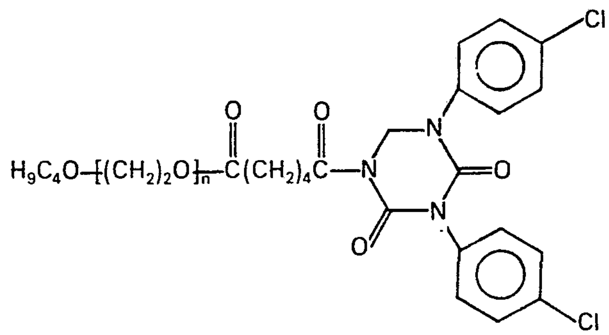

n = 16

Beispiel 141

21,4 g (0,01 mol) des nach Beispiel 1c erhaltenen Produktes werden analog zu Beispiel 139 mit 2,14 g (0,01 mol) 4-Amino-6-tert.-butyl-3-(methylthio)-1,2,4-triazin-5(4H)-on umgesetzt. Zusätzlich gibt man jedoch nach Ende des Zutropfens 0,1 g Natriummetall zu. Die weitere Bearbeitung erfolgt wie in Beispiel 139 angegeben.

Nachrührzeit: 2 Stunden bei 120°C im Bad.

Nach dem Abkühlen wird der Rückstand mit 50 ml Methanol und mit 0,5 ml konzentrierter Salzsäure versetzt. Man saugt vom Unlöslichen ab und entfernt anschliessend das Methanol aus dem Filtrat durch Einengen im Vakuum (200 mbar).

Man erhält 23,2 g eines braungelben Feststoffes. Dieser erfindungsgemäss modifizierte neue Wirkstoff ist gut in Wasser und/oder niederen Alkoholen löslich und besitzt die idealisierte Formel

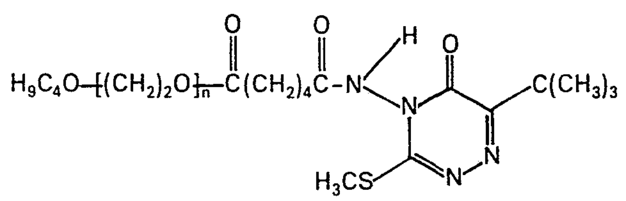

n = 43

Beispiel 142

9,2 g (0,01 mol) des nach Beispiel 3c erhaltenen Produktes werden analog zu Beispiel 140 mit 3,4 g (0,01 mol) 1,3-Di-p-Chlorphenyl-(1H, 2H, 3H, 4H, 5H, 6H)-1,3,5-triazin-2,4-dion umgesetzt. Zusätzlich gibt man jedoch nach Ende des Abdestillierens des Acetons vor Beginn der Temperaturerhöhung auf 130°C 0,1 g Natriummetall zu. Die weitere Bearbeitung erfolgt wie bei Beispiel 140 angegeben.

Nachrührzeit: 40 Stunden bei 120°C im Bad.

Nach dem Abkühlen wird der Rückstand mit 50 ml Methanol und 0,5 ml konzentrierter Salzsäure versetzt. Man saugt ab vom Unlöslichen und entfernt aus dem Filtrat das Methanol durch Einengen im Vakuum.

Man erhält 12,1 g eines rotbraunen, viskosen Produktes. Dieser erfindungsgemäss modifizierte neue Wirkstoff ist gut in Wasser und/oder Methanol sowie Äthanol löslich und besitzt die idealisierte Formel

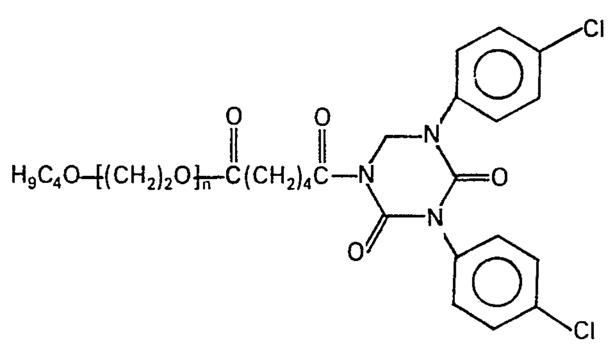

n = 16

Beispiel 143

21,4 g (0,01 mol) der nach Beispiel 1c erhaltenen Verbindung werden analog zu Beispiel 141 mit 2,0 g (0,01 mol) 3-Methyl-4-amino-6-phenyl-1,2,4-triazin-5(4H)-on umgesetzt.

Nachrührzeit: 2 Stunden bei 120°C.

Man erhält 22,8 g eines dunkelgelben Feststoffes. Dieser erfindungsgemäss modifizierte neue Wirkstoff ist gut in Wasser und/oder niederen Alkoholen löslich und besitzt die idealisierte Formel

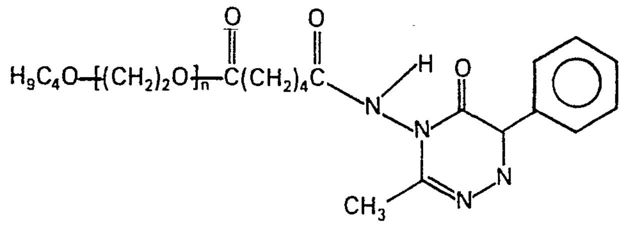

n = 43

Beispiel 144

9,2 g (0,01 mol) der nach Beispiel 3c erhaltenen Verbindung werden analog zu Beispiel 142 mit 3,1 g (0,01 mol) N-(o,o′-Difluorbenzoyl)-N′-(p-Chlorphenyl)-harnstoff umgesetzt.

Nachrührzeit: 36 Stunden bei 120°C.

Man erhält 11,6 g eines dunkelbraunen, viskosen Produktes. Dieser erfindungsgemäss modifizierte neue Wirkstoff ist gut in Wasser und/oder niederen Alkoholen löslich und besitzt die idealisierte Formel

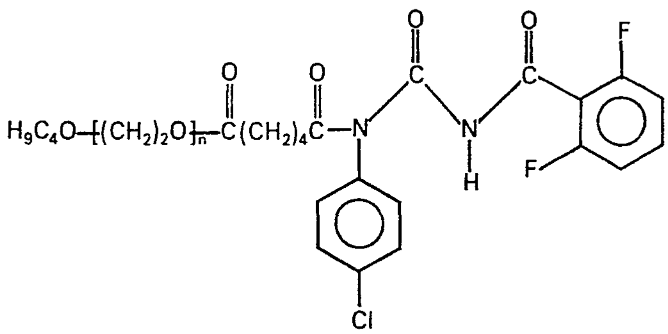

n = 16

Beispiel 145

21,4 g (0,01 mol) der nach Beispiel 1c erhaltenen Verbindung werden analog zu Beispiel 141 mit 3,7 g (0,01 mol) 3-(3,4-Dichlorphenyl)-6-(2,6-Difluorphenyl)-1-oxa-3,5-diazin-(3H,5H)-2,4-dion umgesetzt.

Nachrührzeit: 22 Stunden bei 120°C.

Man erhält 24,6 g eines dunklen, grünlichbraunen Feststoffes. Dieser erfindungsgemäss modifizierte neue Wirkstoff ist gut in Wasser und/oder niederen Alkoholen löslich und besitzt die idealisierte Formel

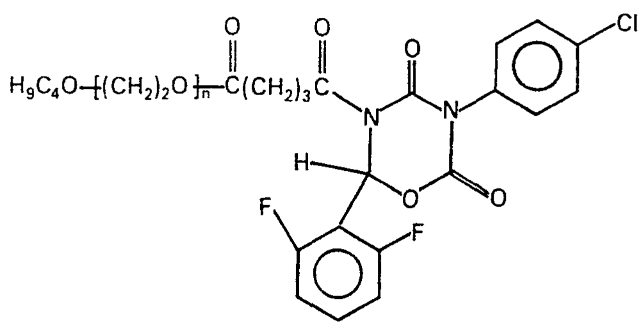

n = 43

Beispiel 146

21,4 g (0,01 mol) der nach Beispiel 1c erhaltenen Verbindung werden analog zu Beispiel 141 mit 2,1 g (0,01 mol) 2-Isopropyloxyphenyl-N-methylcarbamat umgesetzt.

Nachrührzeit: 48 Stunden bei 120°C.

Man erhält 23,1 g eines dunkelbraunen Feststoffes. Dieser erfindungsgemäss modifizierte neue Wirkstoff ist gut in Wasser und/oder niederen Alkoholen löslich und besitzt die idealisierte Formel

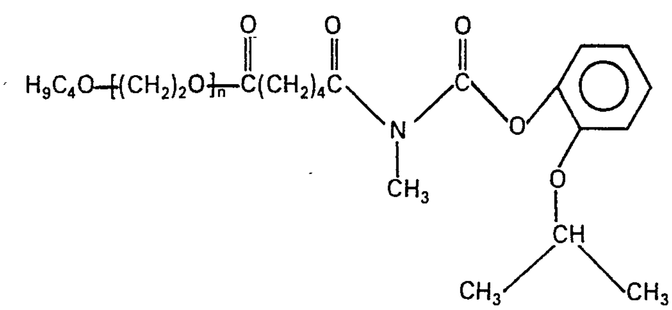

n = 43

Beispiel 147

9,2 g (0,01 mol) der nach Beispiel 3c erhaltenen Verbindung werden analog zu Beispiel 142 mit 2,6 g (0,01 mol) 2-Sulfanilamidothiazol umgesetzt.

Nachrührzeit: 2 Stunden bei 120°C.

Man erhält 11,3 g eines goldgelben, viskosen Produktes. Dieser erfindungsgemäss modifizierte neue Wirkstoff ist gut in Wasser und Methanol sowie Äthanol löslich und besitzt die idealisierte Formel

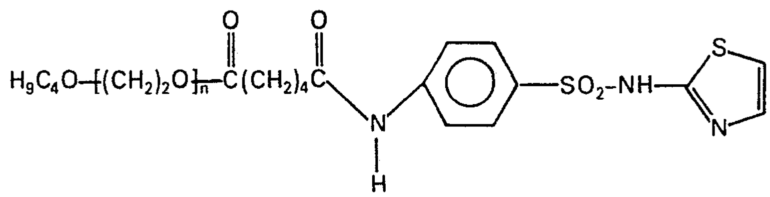

n = 16

Beispiel 148

21,4 g (0,01 mol) der nach Beispiel 1c erhaltenen Verbindung werden analog zu Beispiel 139 mit 2,2 g (0,01 mol) D(–)-threo-1-(p-Nitrophenyl)-2-dichloracetamido-1,3-propandiol umgesetzt.

Nachrührzeit: 17 Stunden bei 105°C.

Man erhält 23,0 g eines rotgoldfarbenen, wachsartigen Feststoffes. Dieser erfindungsgemäss modifizierte neue Wirkstoff ist gut in Wasser und/oder niederen Alkoholen löslich und besitzt die idealisierte Formel

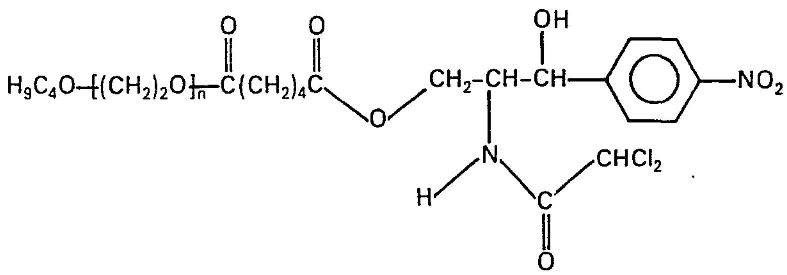

n = 43

Beispiel 149

9,4 g (0,01 mol) der nach Beispiel 3c erhaltenen Verbindung werden analog zu Beispiel 142 mit 2,3 g (0,01 mol) 4-Aminobenzolsulfothiocarbamid umgesetzt.

Nachrührzeit: 45 Minuten bei 120°C.

Man erhält 11,1 g eines rotgelben, viskosen Produktes. Dieser erfindungsgemäss modifizierte neue Wirkstoff ist gut in Wasser und Methanol sowie Äthanol löslich und besitzt die idealisierte Formel

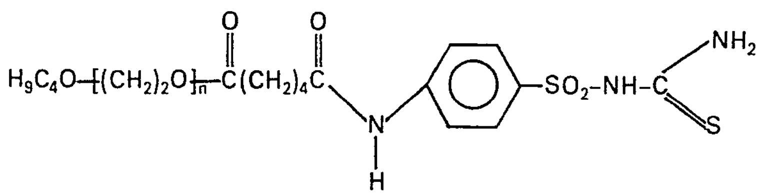

n = 16

Beispiel 150

21,4 g (0,01 mol) der nach Beispiel 1c erhaltenen Verbindung werden analog zu Beispiel 141 mit 2,8 g (0,01 mol) 2-Sulfanilamido-5-methoxypyrimidin umgesetzt.

Nachrührzeit: 155 Minuten bei 120°C.

Man erhält 23,5 g eines dunkelgelben Feststoffes. Dieser erfindungsgemäss modifizierte neue Wirkstoff ist gut in Wasser und/oder niederen Alkoholen löslich und besitzt die idealisierte Formel

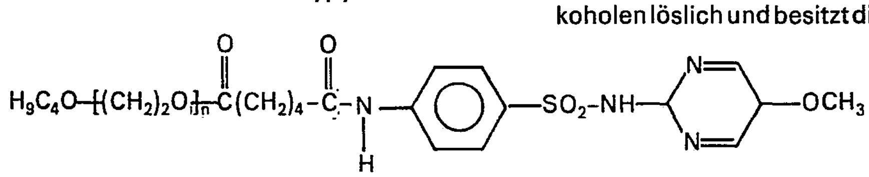

n = 43

Beispiel 151

864 g (0,4 mol) eines auf n-Butanol gestarteten Äthylenoxidpolyäthers vom mittleren Molgewicht 2160 (mittlere Anzahl an Äthylenoxideinheiten n = 42) mit endständiger p-Isocyanatophenylurethangruppe (NCO-Gehalt: 1,94 Gew.-%) werden bei 40°C vorgelegt. Unter schnellem Rühren werden innerhalb von 2 Stunden 85,2 g (0,4 mol) 4-Amino-6-tert.-butyl-3-(methylthio)-1,2,4-triazin-5-(4H)-on, welches in 300 ml abs. Toluol gelöst ist, zugetropft. Nach Ende des Zutropfens wird 10 Min. bei 40°C nachgerührt. Danach ist IR-spektroskopisch kein NCO in der Reaktionsmischung mehr nachweisbar. Anschliessend wird im Vakuum das Lösungsmittel abdestilliert.

Man erhält 946 g eines hellgelben Feststoffes. Dieser erfindungsgemäss modifizierte neue Wirkstoff ist sehr gut in Wasser und/oder niederen Alkoholen löslich und besitzt die idealisierte Formel

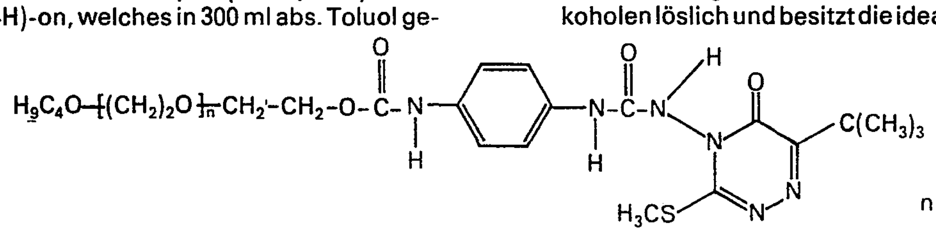

n = 42

**Patentansprüche**

1. Biologisch aktive organische Verbindungen, dadurch gekennzeichnet, dass man biologisch aktive Wirkstoffe mit mindestens einem Zerewitinoff-aktiven Wasserstoffatom mit einem OH-, NH- oder $NH_2$-monofunktionellen hydrophilen Polyäther mit einem Wasseraufnahmevermögen von mindestens 15%, bezogen auf das Gewicht des Polyäthers und einer organischen Verbindung mit $m \geq 2$ gegenüber Zerewitinoff-aktiven Wasserstoffatomen reaktiven Gruppen umsetzt, wobei entweder

a) zunächst der Polyäther mit der organischen Verbindung im Äquivalentverhältnis (m-1):m umgesetzt wird und die so erhaltene Verbindung im Molverhältnis 1:1 mit dem Wirkstoff umgesetzt wird oder

b) der Wirkstoff mit der organischen Verbindung im Molverhältnis 1:1 umgesetzt wird und die so erhaltene Verbindung dann mit dem Polyäther im Äquivalentverhältnis m:(m-1) umgesetzt wird oder

c) Wirkstoff, Polyäther und organische Verbindung im Äquivalentverhältnis 1:m:(m-1) umgesetzt werden.

2. Verfahren zur Herstellung von Verbindungen gemäss Anspruch 1, dadurch gekennzeichnet, dass man biologisch aktive Wirkstoffe mit mindestens einem Zerewitinoff-aktiven Wasserstoffatom mit einem OH- NH-oder $NH_2$-monofunktionellen hydrophilen Polyäther mit einem Wasseraufnahmevermögen von mindestens 15%, bezogen auf das Gewicht des Polyäthers und einer organischen Verbindung mit $m \geq 2$ gegenüber Zerwitinoff-aktiven Wasserstoffatomen reaktiven Gruppen umsetzt, wobei entweder

a) zunächst der Polyäther mit der organischen Verbindung im Äquivalentverhältnis (m-1):m umgesetzt wird und die so erhaltene Verbindung im Molverhältnis 1:1 mit dem Wirkstoff umgesetzt wird oder

b) der Wirkstoff mit der organischen Verbindung im Molverhältnis 1:1 umgesetzt wird und die so erhaltene Verbindung dann mit dem Polyäther im Äquivalentverhältnis m:(m-1) umgesetzt wird oder

c) Wirkstoff, Polyäther und organische Verbindung im Äquivalentverhältnis 1:m:(m-1) umgesetzt werden.

3. Verwendung der Verbindungen gemäss Anspruch 1 als biologisch aktive Wirkstoffe mit in Wasser und niederen aliphatischen Alkoholen verbesserter Löslichkeit.

**Claims**

1. Organic compounds which have a biological action, characterised in that they are able to be prepared by reacting active compounds which have a biological action and which have at least one hydrogen atom which is active in Zerewitinoff reactions with a hydrophilic polyether containing one OH, NH or $NH_2$ group and having a water absorption capacity of at least 15%, relative to the weight of the polyether, and with an organic compound with $m \geq 2$ groups which are reactive towards hydrogen atoms which are active in Zerewitinoff reactions, wherein either

a) the polyether is first reacted with the organic compound in an equivalent ratio of (m-1):m and the resulting compound is reacted with the active compound in a molar ratio of 1:1 or

b) the active compound is reacted with the organic compound in a molar ratio of 1:1 and the resulting compound is then reacted with the polyether in an equivalent ratio of m:(m-1) or

c) the active compound, the polyether and the organic compound are reacted in an equivalent ratio of 1:m:(m-1).

2. Process for preparing compounds according to Claim 1, characterised in that active compounds which have a biological action and which have at least one hydrogen atom which is active in Zerewitinoff reactions are reacted with a hydrophilic polyether containing one OH,NH or $NH_2$ group and having a water absorption capacity of at least 15%, relative to the weight of the polyether, and with an organic compound with $m \geq 2$ groups which are reactive towards hydrogen atoms which are active in Zerewitinoff reactions, wherein either

a) the polyether is first reacted with the organic compound in an equivalent ratio of (m-1):m and the resulting compound is reacted with the active compound in a molar ratio of 1:1 or

b) the active compound is reacted with the organic compound in a molar ratio of 1:1 and the resulting compound is then reacted with the polyether in an equivalent ratio of m:(m-1) or

c) the active compound, the polyether and the organic compound are reacted in an equivalent ratio of 1:m:(m-1).

3. Use of the compounds according to Claim 1 as active compounds which have a biological action and which have improved solubility in water and in lower aliphatic alcohols.

**Revendications**

1. Composés organiques biologiquement actifs, caractérisés en ce qu'ils peuvent être préparés par le fait qu'on fait réagir des substances actives douées d'activité biologique portant au moins un atome d'hydrogène actif selon Zerwitinoff avec un polyéther hydrophile à une seule fonction OH, NH ou $NH_2$, de pouvoir d'absorption de l'eau d'au moins 15%, par rapport au poids du polyéther, et un composé organique portant $m \geq 2$ groupes réactifs envers des atomes d'hydrogène actifs selon Zerewitinoff, en sorte que ou bien

a) tout d'abord le polyéther est amené à réagir avec le composé organique dans le rapport d'équivalents (m-1):m et le composé ainsi obtenu est amené à réagir avec la substance active dans le rapport molaire de 1:1, ou bien

b) la substance active est amenée à réagir avec le composé organique dans le rapport molaire de 1:1 et le composé ainsi obtenu est amené à réagir ensuite avec le polyéther dans le rapport d'équivalents de m:(m-1), ou bien

c) la substance active, le polyéther et le composé organique sont amenés à réagir dans le rapport d'équivalents de 1:m:(m-1).

2. Procédé de production de composés suivant la revendication 1, caractérisé en ce qu'on fait réagir des substances actives douées d'activité biologique portant au moins un atome d'hydrogène actif selon Zerewitinoff avec un polyéther hydrophile à une seule fonction OH, NH ou $NH_2$ et ayant un pouvoir d'absorption de l'eau d'au moins 15%, par rapport au poids du polyéther, et un composé organique portant $m \geqq 2$ groupes réactifs envers des atomes d'hydrogène actifs selon Zerewitinoff, en sorte que ou bien

a) le polyéther est tout d'abord amené à réagir avec le composé organique dans le rapport d'équivalents de (m-1):m et le composé ainsi obtenu est amené à réagir avec la substance active dans le rapport molaire de 1:1, ou bien

b) la substance active est amenée à réagir avec le composé organique dans le rapport molaire de 1:1 et le composé ainsi obtenu est ensuite amené à réagir avec le polyéther dans le rapport d'équivalents de m:(m-1), ou bien

c) la substance active, le polyéther et le composé organique sont amenés à réagir dans le rapport d'équivalents de 1:m:(m-1).

3. Utilisation des composés suivant la revendication 1, comme substances actives douées d'activité biologique dont la solubilité dans l'eau et dans les alcools aliphatiques inférieurs est améliorée.